# EUROPEAN PATENT SPECIFICATION

(11) **EP 2 418 212 B1**
(45) Date of publication and mention of the grant of the patent: **15.06.2016**
(21) Application number: 10182052.0
(22) Date of filing: 28.11.2000
(51) Int. Cl.: C07H 21/04, C07K 14/00, C07K 16/00, G01N 33/53, A61K 39/395, C07K 14/705

(54) **Isolation of five novel genes coding for new fc receptors-type melanoma involved in the pathogenesis of lymphoma/melanoma**
Isolierung von fünf neuen Genen für das neue Fc-Rezeptor-Melanom, das die Pathogenese von Lymphomen bzw. Melanomen beeinflusst
Isolation de cinq nouveaux gènes codant pour des nouveaux récepteurs Fc de type mélanome intervenant dans la pathogénèse du lymphome malin et du mélanome

(30) Priority: 29.11.1999 US 168151 P
(43) Date of publication of application: 15.02.2012
(62) Divisional of application: 00983778.2
(73) Proprietor: The Trustees of Columbia University in the City of New York, New York, New York 10027 (US)
(72) Inventor: Dalla-Favera, Riccardo, New York, NY 10027-6818 (US)
(74) Representative: Vossius & Partner Patentanwälte Rechtsanwälte mbB

(56) References cited:
- EP-A- 0 330 191
- WO-A1-00/17222
- MILLER I J ET AL: "IRTAS: A NEW FAMILY OF FC-RELATED CELL-SURFACE RECEPTORS EXPRESSED IN B CELLS AND IMPLICATED IN LYMPHOMAGENESIS", BLOOD, AMERICAN SOCIETY OF HEMATOLOGY, US, vol. 96, no. 11, 16 November 2000 (2000-11-16), page 499A, XP002944801, ISSN: 0006-4971

### SEQUENCE LISTING

<110> Dalla-Favera, Riccardo
<120> ISOLATION OF FIVE NOVEL GENES ENCODING FOR NEW Fc RECEPTORS-TYPE MELANOMA INVOLVED IN THE PATHOGENESIS OF LYMPHOMA/MYELOMA
<130> 0575/58044-a-PCT-EPO
<140> 00 98 3778.2 (PCT/US00/32403)
   <141> 2000-11-28
<160> 44
<170> PatentIn version 3.1
<210> 1
   <211> 515
   <212> PRT
   <213> Homo Sapiens
<400> 1
   Met Leu Leu Trp Ala Ser Leu Leu Ala Phe Ala Pro Val Cys Gly Gln
   1               5                   10                  15
   Ser Ala Ala Ala His Lys Pro Val Ile Ser Val His Pro Pro Trp Thr
               20                  25                  30
   Thr Phe Phe Lys Gly Glu Arg Val Thr Leu Thr Cys Asn Gly Phe Gln
           35                  40                  45
   Phe Tyr Ala Thr Glu Lys Thr Thr Trp Tyr His Arg His Tyr Trp Gly
       50                  55                  60
   Glu Lys Leu Thr Leu Thr Pro Gly Asn Thr Leu Glu Val Arg Glu Ser
   65                  70                  75                  80
   Gly Leu Tyr Arg Cys Gln Ala Arg Gly Ser Pro Arg Ser Asn Pro Val
                   85                  90                  95
   Arg Leu Leu Phe Ser Ser Asp Ser Leu Ile Leu Gln Ala Pro Tyr Ser
               100                 105                 110
   Val Phe Glu Gly Asp Thr Leu Val Leu Arg Cys His Arg Arg Arg Lys
           115                 120                 125
   Glu Lys Leu Thr Ala Val Lys Tyr Thr Trp Asn Gly Asn Ile Leu Ser
       130                 135                 140
   Ile Ser Asn Lys Ser Trp Asp Leu Leu Ile Pro Gln Ala Ser Ser Asn
   145                 150                 155                 160
   Asn Asn Gly Asn Tyr Arg Cys Ile Gly Tyr Gly Asp Glu Asn Asp Val
                   165                 170                 175
   Phe Arg Ser Asn Phe Lys Ile Ile Lys Ile Gln Glu Leu Phe Pro His
               180                 185                 190
   Pro Glu Leu Lys Ala Thr Asp Ser Gln Pro Thr Glu Gly Asn Ser Val
           195                 200                 205
   Asn Leu Ser Cys Glu Thr Gln Leu Pro Pro Glu Arg Ser Asp Thr Pro
       210                 215                 220
   Leu His Phe Asn Phe Phe Arg Asp Gly Glu Val Ile Leu Ser Asp Trp
   225                 230                 235                 240
   Ser Thr Tyr Pro Glu Leu Gln Leu Pro Thr Val Trp Arg Glu Asn Ser
                   245                 250                 255
   Gly Ser Tyr Trp Cys Gly Ala Glu Thr Val Arg Gly Asn Ile His Lys
               260                 265                 270
   His Ser Pro Ser Leu Gln Ile His Val Gln Arg Ile Pro Val Ser Gly
           275                 280                 285
   Val Leu Leu Glu Thr Gln Pro Ser Gly Gly Gln Ala Val Glu Gly Glu
       290                 295                 300
   Met Leu Val Leu Val Cys Ser Val Ala Glu Gly Thr Gly Asp Thr Thr
   305                 310                 315                 320
   Phe Ser Trp His Arg Glu Asp Met Gln Glu Ser Leu Gly Arg Lys Thr
                   325                 330                 335
   Gln Arg Ser Leu Arg Ala Glu Leu Glu Leu Pro Ala Ile Arg Gln Ser
               340                 345                 350
   His Ala Gly Gly Tyr Tyr Cys Thr Ala Asp Asn Ser Tyr Gly Pro Val
   355 360 365
   Gln Ser Met Val Leu Asn Val Thr Val Arg Glu Thr Pro Gly Asn Arg
   370 375 380
   Asp Gly Leu Val Ala Ala Gly Ala Thr Gly Gly Leu Leu Ser Ala Leu
   385 390 395 400
   Ile Leu Ala Val Ala Leu Leu Phe His Cys Trp Arg Arg Arg Lys Ser
   405 410 415
   Gly Val Gly Phe Leu Gly Asp Glu Thr Arg Leu Pro Pro Ala Pro Gly
   420 425 430
   Pro Gly Glu Ser Ser His Ser Ile Cys Pro Ala Gln Val Glu Leu Gln
   435 440 445
   Ser Leu Tyr Val Asp Val His Pro Lys Lys Gly Asp Leu Val Tyr Ser
   450 455 460
   Glu Ile Gln Thr Thr Gln Leu Gly Glu Glu Glu Glu Ala Asn Thr Ser
   465 470 475 480
   Arg Thr Leu Leu Glu Asp Lys Asp Val Ser Val Val Tyr Ser Glu Val
   485 490 495
   Lys Thr Gln His Pro Asp Asn Ser Ala Gly Lys Ile Ser Ser Lys Asp
   500 505 510
   Glu Glu Ser
   515
<210> 2
   <211> 2499
   <212> DNA
   <213> Homo Sapiens
<400> 2
   ctcaatcagc tttatgcaga gaagaagctt actgagctca ctgctggtgc tggtgtaggc 60
   aagtgctgct ttggcaatct gggctgacct ggcttgtctc ctcagaactc cttctccaac 120
   cctggagcag gcttccatgc tgctgtgggc gtccttgctg gcctttgctc cagtctgtgg 180
   acaatctgca gctgcacaca aacctgtgat ttccgtccat cctccatgga ccacattctt 240
   caaaggagag agagtgactc tgacttgcaa tggatttcag ttctatgcaa cagagaaaac 300
   aacatggtat catcggcact actggggaga aaagttgacc ctgaccccag gaaacaccct 360
   cgaggttcgg gaatctggac tgtacagatg ccaggcccgg ggctccccac gaagtaaccc 420
   tgtgcgcttg ctcttttctt cagactcctt aatcctgcag gcaccatatt ctgtgtttga 480
   aggtgacaca ttggttctga gatgccacag aagaaggaaa gagaaattga ctgctgtgaa 540
   atatacttgg aatggaaaca ttctttccat ttctaataaa agctgggatc ttcttatccc 600
   acaagcaagt tcaaataaca atggcaatta tcgatgcatt ggatatggag atgagaatga 660
   tgtatttaga tcaaatttca aaataattaa aattcaagaa ctatttccac atccagagct 720
   gaaagctaca gactctcagc ctacagaggg gaattctgta aacctgagct gtgaaacaca 780
   gcttcctcca gagcggtcag acaccccact tcacttcaac ttcttcagag atggcgaggt 840
   catcctgtca gactggagca cgtacccgga actccagctc ccaaccgtct ggagagaaaa 900
   ctcaggatcc tattggtgtg gtgctgaaac agtgaggggt aacatccaca agcacagtcc 960
   ctcgctacag atccatgtgc agcggatccc tgtgtctggg gtgctcctgg agacccagcc 1020
   ctcagggggc caggctgttg aaggggagat gctggtcctt gtctgctccg tggctgaagg 1080
   cacaggggat accacattct cctggcaccg agaggacatg caggagagtc tggggaggaa 1140
   aactcagcgt tccctgagag cagagctgga gctccctgcc atcagacaga gccatgcagg 1200
   gggatactac tgtacagcag acaacagcta cggccctgtc cagagcatgg tgctgaatgt 1260
   cactgtgaga gagaccccag gcaacagaga tggccttgtc gccgcgggag ccactggagg 1320
   gctgctcagt gctcttctcc tggctgtggc cctgctgttt cactgctggc gtcggaggaa 1380
   gtcaggagtt ggtttcttgg gagacgaaac caggctccct cccgctccag gcccaggaga 1440
   gtcctcccat tccatctgcc ctgcccaggt ggagcttcag tcgttgtatg ttgatgtaca 1500
   ccccaaaaag ggagatttgg tatactctga gatccagact actcagctgg gagaagaaga 1560
   ggaagctaat acctccagga cacttctaga ggataaggat gtctcagttg tctactctga 1620
   ggtaaagaca caacacccag ataactcagc tggaaagatc agctctaagg atgaagaaag 1680
   ttaagagaat gaaaagttac gggaacgtcc tactcatgtg atttctccct tgtccaaagt 1740
   cccaggccca gtgcagtcct tgcggcacct ggaatgatca actcattcca gctttctaat 1800
   tcttctcatg catatgcatt cactcccagg aatactcatt cgtctactct gatgttggga 1860
   tggaatggcc tctgaaagac ttcactaaaa tgaccaggat ccacagttaa gagaagaccc 1920
   tgtagtattt gctgtgggcc tgacctaatg cattccctag ggtctgcttt agagaagggg 1980
   gataaagaga gagaaggact gttatgaaaa acagaagcac aaattttggt gaattgggat 2040
   ttgcagagat gaaaaagact gggtgacctg gatctctgct taatacatct acaaccattg    2100
   tctcactgga gactcacttg catcagtttg tttaactgtg agtggctgca caggcactgt    2160
   gcaaacaatg aaaagcccct tcacttctgc ctgcacagct tacactgtca ggattcagtt    2220
   gcagattaaa gaacccatct ggaatggttt acagagagag gaatttaaaa gaggacatca    2280
   gaagagctgg agatgcaagc tctaggctgc gcttccaaaa gcaaatgata attatgttaa    2340
   tgtcattagt gacaaagatt tgcaacatta gagaaaagag acacaaatat aaaattaaaa    2400
   acttaagtac caactctcca aaactaaatt tgaacttaaa atattagtat aaactcataa    2460
   taaactctgc ctttaaataa aaaaaaaaaa aaaaaaaaa                            2499
<210> 3
   <211> 592
   <212> PRT
   <213> Homo Sapiens
<400> 3
   Met Leu Leu Trp Val Ile Leu Leu Val Leu Ala Pro Val Ser Gly Gln
   1               5                   10                  15
   Phe Ala Arg Thr Pro Arg Pro Ile Ile Phe Leu Gln Pro Pro Trp Thr
               20                  25                  30
   Thr Val Phe Gln Gly Glu Arg Val Thr Leu Thr Cys Lys Gly Phe Arg
           35                  40                  45
   Phe Tyr Ser Pro Gln Lys Thr Lys Trp Tyr His Arg Tyr Leu Gly Lys
       50                  55                  60
   Glu Ile Leu Arg Glu Thr Pro Asp Asn Ile Leu Glu Val Gln Glu Ser
   65                  70                  75                  80
   Gly Glu Tyr Arg Cys Gln Ala Gln Gly Ser Pro Leu Ser Ser Pro Val
                   85                  90                  95
   His Leu Asp Phe Ser Ser Ala Ser Leu Ile Leu Gln Ala Pro Leu Ser
               100                 105                 110
   Val Phe Glu Gly Asp Ser Val Val Leu Arg Cys Arg Ala Lys Ala Glu
           115                 120                 125
   Val Thr Leu Asn Asn Thr Ile Tyr Lys Asn Asp Asn Val Leu Ala Phe
   130 135 140
   Leu Asn Lys Arg Thr Asp Phe His Ile Pro His Ala Cys Leu Lys Asp
   145 150 155 160
   Asn Gly Ala Tyr Arg Cys Thr Gly Tyr Lys Glu Ser Cys Cys Pro Val
   165 170 175
   Ser Ser Asn Thr Val Lys Ile Gln Val Gln Glu Pro Phe Thr Arg Pro
   180 185 190
   Val Leu Arg Ala Ser Ser Phe Gln Pro Ile Ser Gly Asn Pro Val Thr
   195 200 205
   Leu Thr Cys Glu Thr Gln Leu Ser Leu Glu Arg Ser Asp Val Pro Leu
   210 215 220
   Arg Phe Arg Phe Phe Arg Asp Asp Gln Thr Leu Gly Leu Gly Trp Ser
   225 230 235 240
   Leu Ser Pro Asn Phe Gln Ile Thr Ala Met Trp Ser Lys Asp Ser Gly
   245 250 255
   Phe Tyr Trp Cys Lys Ala Ala Thr Met Pro His Ser Val Ile Ser Asp
   260 265 270
   Ser Pro Arg Ser Trp Ile Gln Val Gln Ile Pro Ala Ser His Pro Val
   275 280 285
   Leu Thr Leu Ser Pro Glu Lys Ala Leu Asn Phe Glu Gly Thr Lys Val
   290 295 300
   Thr Leu His Cys Glu Thr Gln Glu Asp Ser Leu Arg Thr Leu Tyr Arg
   305 310 315 320
   Phe Tyr His Glu Gly Val Pro Leu Arg His Lys Ser Val Arg Cys Glu
   325 330 335
   Arg Gly Ala Ser Ile Ser Phe Ser Leu Thr Thr Glu Asn Ser Gly Asn
   340 345 350
   Tyr Tyr Cys Thr Ala Asp Asn Gly Leu Gly Ala Lys Pro Ser Lys Ala
   355 360 365
   Val Ser Leu Ser Val Thr Val Pro Val Ser His Pro Val Leu Asn Leu
       370                 375                 380
   Ser Ser Pro Glu Asp Leu Ile Phe Glu Gly Ala Lys Val Thr Leu His
   385                 390                 395                 400
   Cys Glu Ala Gln Arg Gly Ser Leu Pro Ile Leu Tyr Gln Phe His His
                   405                 410                 415
   Glu Asp Ala Ala Leu Glu Arg Arg Ser Ala Asn Ser Ala Gly Gly Val
               420                 425                 430
   Ala Ile Ser Phe Ser Leu Thr Ala Glu His Ser Gly Asn Tyr Tyr Cys
           435                 440                 445
   Thr Ala Asp Asn Gly Phe Gly Pro Gln Arg Ser Lys Ala Val Ser Leu
       450                 455                 460
   Ser Ile Thr Val Pro Val Ser His Pro Val Leu Thr Leu Ser Ser Ala
   465                 470                 475                 480
   Glu Ala Leu Thr Phe Glu Gly Ala Thr Val Thr Leu His Cys Glu Val
                   485                 490                 495
   Gln Arg Gly Ser Pro Gln Ile Leu Tyr Gln Phe Tyr His Glu Asp Met
               500                 505                 510
   Pro Leu Val Ser Ser Ser Thr Pro Ser Val Gly Arg Val Ser Phe Ser
           515                 520                 525
   Phe Ser Leu Thr Glu Gly His Ser Gly Asn Tyr Tyr Cys Thr Ala Asp
       530                 535                 540
   Asn Gly Phe Gly Pro Gln Arg Ser Glu Val Val Ser Leu Phe Val Thr
   545                 550                 555                 560
   Gly Lys Cys Trp Val Leu Ala Ser Lys Pro Pro Leu Ala Glu Phe Ser
                   565                 570                 575
   Leu Thr His Ser Phe Lys Asn Leu Phe Ala Leu Ser Ser Phe Leu Pro
               580                 585                 590
<210> 4
   <211> 5308
   <212> DNA
   <213> Homo Sapiens
<900> 4
<210> 5
   <211> 734
   <212> PRT
   <213> Homo Sapiens
<400> 5
   Met Leu Leu Trp Leu Leu Leu Leu Ile Leu Thr Pro Gly Arg Glu Gln
   1               5                   10                  15
   Ser Gly Val Ala Pro Lys Ala Val Leu Leu Leu Asn Pro Pro Trp Ser
               20                  25                  30
   Thr Ala Phe Lys Gly Glu Lys Val Ala Leu Ile Cys Ser Ser Ile Ser
           35                  40                  45
   His Ser Leu Ala Gln Gly Asp Thr Tyr Trp Tyr His Asp Glu Lys Leu
       50                  55                  60
   Leu Lys Ile Lys His Asp Lys Ile Gln Ile Thr Glu Pro Gly Asn Tyr
   65                  70                  75                  80
   Gln Cys Lys Thr Arg Gly Ser Ser Leu Ser Asp Ala Val His Val Glu
                   85                  90                  95
   Phe Ser Pro Asp Trp Leu Ile Leu Gln Ala Leu His Pro Val Phe Glu
               100                 105                 110
   Gly Asp Asn Val Ile Leu Arg Cys Gln Gly Lys Asp Asn Lys Asn Thr
           115                 120                 125
   His Gln Lys Val Tyr Tyr Lys Asp Gly Lys Gln Leu Pro Asn Ser Tyr
       130                 135                 140
   Asn Leu Glu Lys Ile Thr Val Asn Ser Val Ser Arg Asp Asn Ser Lys
   145                 150                 155                 160
   Tyr His Cys Thr Ala Tyr Arg Lys Phe Tyr Ile Leu Asp Ile Glu Val
                   165                 170                 175
   Thr Ser Lys Pro Leu Asn Ile Gln Val Gln Glu Leu Phe Leu His Pro
   180 185 190
   Val Leu Arg Ala Ser Ser Ser Thr Pro Ile Glu Gly Ser Pro Met Thr
   195 200 205
   Leu Thr Cys Glu Thr Gln Leu Ser Pro Gln Arg Pro Asp Val Gln Leu
   210 215 220
   Gln Phe Ser Leu Phe Arg Asp Ser Gln Thr Leu Gly Leu Gly Trp Ser
   225 230 235 240
   Arg Ser Pro Arg Leu Gln Ile Pro Ala Met Trp Thr Glu Asp Ser Gly
   245 250 255
   Ser Tyr Trp Cys Glu Val Glu Thr Val Thr His Ser Ile Lys Lys Arg
   260 265 270
   Ser Leu Arg Ser Gln Ile Arg Val Gln Arg Val Pro Val Ser Asn Val
   275 280 285
   Asn Leu Glu Ile Arg Pro Thr Gly Gly Gln Leu Ile Glu Gly Glu Asn
   290 295 300
   Met Val Leu Ile Cys Ser Val Ala Gln Gly Ser Gly Thr Val Thr Phe
   305 310 315 320
   Ser Trp His Lys Glu Gly Arg Val Arg Ser Leu Gly Arg Lys Thr Gln
   325 330 335
   Arg Ser Leu Leu Ala Glu Leu His Val Leu Thr Val Lys Glu Ser Asp
   340 345 350
   Ala Gly Arg Tyr Tyr Cys Ala Ala Asp Asn Val His Ser Pro Ile Leu
   355 360 365
   Ser Thr Trp Ile Arg Val Thr Val Arg Ile Pro Val Ser His Pro Val
   370 375 380
   Leu Thr Phe Arg Ala Pro Arg Ala His Thr Val Val Gly Asp Leu Leu
   385 390 395 400
   Glu Leu His Cys Glu Ser Leu Arg Gly Ser Pro Pro Ile Leu Tyr Arg
   405 410 415
   Phe Tyr His Glu Asp Val Thr Leu Gly Asn Ser Ser Ala Pro Ser Gly
   420 425 430
   Gly Gly Ala Ser Phe Asn Leu Ser Leu Thr Ala Glu His Ser Gly Asn
   435 440 445
   Tyr Ser Cys Asp Ala Asp Asn Gly Leu Gly Ala Gln His Ser His Gly
   450 455 460
   Val Ser Leu Arg Val Thr Val Pro Val Ser Arg Pro Val Leu Thr Leu
   465 470 475 480
   Arg Ala Pro Gly Ala Gln Ala Val Val Gly Asp Leu Leu Glu Leu His
   485 490 495
   Cys Glu Ser Leu Arg Gly Ser Phe Pro Ile Leu Tyr Trp Phe Tyr His
   500 505 510
   Glu Asp Asp Thr Leu Gly Asn Ile Ser Ala His Ser Gly Gly Gly Ala
   515 520 525
   Ser Phe Asn Leu Ser Leu Thr Thr Glu His Ser Gly Asn Tyr Ser Cys
   530 535 540
   Glu Ala Asp Asn Gly Leu Gly Ala Gln His Ser Lys Val Val Thr Leu
   545 550 555 560
   Asn Val Thr Gly Thr Ser Arg Asn Arg Thr Gly Leu Thr Ala Ala Gly
   565 570 575
   Ile Thr Gly Leu Val Leu Ser Ile Leu Val Leu Ala Ala Ala Ala Ala
   580 585 590
   Leu Leu His Tyr Ala Arg Ala Arg Arg Lys Pro Gly Gly Leu Ser Ala
   595 600 605
   Thr Gly Thr Ser Ser His Ser Pro Ser Glu Cys Gln Glu Pro Ser Ser
   610 615 620
   Ser Arg Pro Ser Arg Ile Asp Pro Gln Glu Pro Thr His Ser Lys Pro
   625 630 635 640
   Leu Ala Pro Met Glu Leu Glu Pro Met Tyr Ser Asn Val Asn Pro Gly
   645 650 655
   Asp Ser Asn Pro Ile Tyr Ser Gln Ile Trp Ser Ile Gln His Thr Lys
   660 665 670
   Glu Asn Ser Ala Asn Cys Pro Met Met His Gln Glu His Glu Glu Leu
   675 680 685
   Thr Val Leu Tyr Ser Glu Leu Lys Lys Thr His Pro Asp Asp Ser Ala
   690 695 700
   Gly Glu Ala Ser Ser Arg Gly Arg Ala His Glu Glu Asp Asp Glu Glu
   705 710 715 720
   Asn Tyr Glu Asn Val Pro Arg Val Leu Leu Ala Ser Asp His
   725 730
<210> 6
   <211> 2970
   <212> DNA
   <213> Homo Sapiens
<400> 6
   agtgaagggg tttcccatat gaaaaataca gaaagaatta tttgaatact agcaaataca 60
   caacttgata tttctagaga acccaggcac agtcttggag acattactcc tgagagactg 120
   cagctgatgg aagatgagcc ccaacttcta aaaatgtatc actaccggga ttgagataca 180
   aacagcattt aggaaggtct catctgagta gcagcttcct gccctccttc ttggagataa 240
   gtcgggcttt tggtgagaca gactttccca accctctgcc cggccggtgc ccatgcttct 300
   gtggctgctg ctgctgatcc tgactcctgg aagagaacaa tcaggggtgg ccccaaaagc 360
   tgtacttctc ctcaatcctc catggtccac agccttcaaa ggagaaaaag tggctctcat 420
   atgcagcagc atatcacatt ccctagccca gggagacaca tattggtatc acgatgagaa 480
   gttgttgaaa ataaaacatg acaagatcca aattacagag cctggaaatt accaatgtaa 540
   gacccgagga tcctccctca gaattacaga gcctggaaat taccaatgta agacccgagg 600
   atcctccctc agacatcctg tctttgaagg agacaatgtc attctgagat gtcaggggaa 660
   agacaacaaa aacactcatc aaaaggttta ctacaaggat ggaaaacagc ttcctaatag 720
   ttataattta gagaagatca cagtgaattc agtctccagg gataatagca aatatcattg 780
   tactgcttat aggaagtttt acatacttga cattgaagta acttcaaaac ccctaaatat 840
   ccaagttcaa gagctgtttc tacatcctgt gctgagagcc agctcttcca cgcccataga 900
   ggggagtccc atgaccctga cctgtgagac ccagctctct ccacagaggc cagatgtcca     960
   gctgcaattc tccctcttca gagatagcca gaccctcgga ttgggctgga gcaggtcccc    1020
   cagactccag atccctgcca tgtggactga agactcaggg tcttactggt gtgaggtgga    1080
   gacagtgact cacagcatca aaaaaaggag cctgagatct cagatacgtg tacagagagt    1140
   ccctgtgtct aatgtgaatc tagagatccg gcccaccgga gggcagctga ttgaaggaga    1200
   aaatatggtc cttatttgct cagtagccca gggttcaggg actgtcacat tctcctggca    1260
   caaagaagga agagtaagaa gcctgggtag aaagacccag cgttccctgt tggcagagct    1320
   gcatgttctc accgtgaagg agagtgatgc agggagatac tactgtgcag ctgataacgt    1380
   tcacagcccc atcctcagca cgtggattcg agtcaccgtg agaattccgg tatctcaccc    1440
   tgtcctcacc ttcagggctc ccagggccca cactgtggtg ggggacctgc tggagcttca    1500
   ctgtgagtcc ctgagaggct ctcccccgat cctgtaccga ttttatcatg aggatgtcac    1560
   cctggggaac agctcagccc cctctggagg aggagcctcc ttcaacctct ctctgactgc    1620
   agaacattct ggaaactact cctgtgatgc agacaatggc ctgggggccc agcacagtca    1680
   tggagtgagt ctcagggtca cagttccggt gtctcgcccc gtcctcaccc tcagggctcc    1740
   cggggcccag gctgtggtgg gggacctgct ggagcttcac tgtgagtccc tgagaggctc    1800
   cttcccgatc ctgtactggt tttatcacga ggatgacacc ttggggaaca tctcggccca    1860
   ctctggagga ggggcatcct tcaacctctc tctgactaca gaacattctg gaaactactc    1920
   atgtgaggct gacaatggcc tgggggccca gcacagtaaa gtggtgacac tcaatgttac    1980
   aggaacttcc aggaacagaa caggccttac cgctgcggga atcacggggc tggtgctcag    2040
   catcctcgtc cttgctgctg ctgctgctct gctgcattac gccagggccc gaaggaaacc    2100
   aggaggactt tctgccactg gaacatctag tcacagtcct agtgagtgtc aggagccttc    2160
   ctcgtccagg ccttccagga tagaccctca agagcccact cactctaaac cactagcccc    2220
   aatggagctg gagccaatgt acagcaatgt aaatcctgga gatagcaacc cgatttattc    2280
   ccagatctgg agcatccagc atacaaaaga aaactcagct aattgtccaa tgatgcatca    2340
   agagcatgag gaacttacag tcctctattc agaactgaag aagacacacc cagacgactc    2400
   tgcaggggag gctagcagca gaggcagggc ccatgaagaa gatgatgaag aaaactatga    2460
   gaatgtacca cgtgtattac tggcctcaga ccactagccc cttacccaga gtggcccaca    2520
   ggaaacagcc tgcaccattt ttttttctgt tctctccaac cacacatcat ccatctctcc    2580
   agactctgcc tcctacgagg ctgggctgca gggtatgtga ggctgagcaa aaggtctgca    2640
   aatctcccct gtgcctgatc tgtgtgttcc ccaggaagag agcaggcagc ctctgagcaa    2700
   gcactgtgtt attttcacag tggagacacg tggcaaggca ggagggccct cagctcctag    2760
   ggctgtcgaa tagaggagga gagagaaatg gtctagccag ggttacaagg gcacaatcat 2820
   gaccatttga tccaagtgtg atcgaaagct gttaatgtgc tctctgtata aacaatttgc 2880
   tccaaatatt ttgtttccct tttttgtgtg gctggtagtg gcattgctga tgttttggtg 2940
   tatatgctgt atccttgcta ccatattggg 2970
<210> 7
   <211> 508
   <212> PRT
   <213> Homo Sapiens
<400> 7
   Met Leu Leu Trp Ser Leu Leu Val Ile Phe Asp Ala Val Thr Glu Gln
   1 5 10 15
   Ala Asp Ser Leu Thr Leu Val Ala Pro Ser Ser Val Phe Glu Gly Asp
   20 25 30
   Ser Ile Val Leu Lys Cys Gln Gly Glu Gln Asn Trp Lys Ile Gln Lys
   35 40 45
   Met Ala Tyr His Lys Asp Asn Lys Glu Leu Ser Val Phe Lys Lys Phe
   50 55 60
   Ser Asp Phe Leu Ile Gln Ser Ala Val Leu Ser Asp Ser Gly Asn Tyr
   65 70 75 80
   Phe Cys Ser Thr Lys Gly Gln Leu Phe Leu Trp Asp Lys Thr Ser Asn
   85 90 95
   Ile Val Lys Ile Lys Val Gln Glu Leu Phe Gln Arg Pro Val Leu Thr
   100 105 110
   Ala Ser Ser Phe Gln Pro Ile Glu Gly Gly Pro Val Ser Leu Lys Cys
   115 120 125
   Glu Thr Arg Leu Ser Pro Gln Arg Leu Asp Val Gln Leu Gln Phe Cys
   130 135 140
   Phe Phe Arg Glu Asn Gln Val Leu Gly Ser Gly Trp Ser Ser Ser Pro
   145 150 155 160
   Glu Leu Gln Ile Ser Ala Val Trp Ser Glu Asp Thr Gly Ser Tyr Trp
   165 170 175
   Cys Lys Ala Glu Thr Val Thr His Arg Ile Arg Lys Gln Ser Leu Gln
   180 185 190
   Ser Gln Ile His Val Gln Arg Ile Pro Ile Ser Asn Val Ser Leu Glu
   195 200 205
   Ile Arg Ala Pro Gly Gly Gln Val Thr Glu Gly Gln Lys Leu Ile Leu
   210 215 220
   Leu Cys Ser Val Ala Gly Gly Thr Gly Asn Val Thr Phe Ser Trp Tyr
   225 230 235 240
   Arg Glu Ala Thr Gly Thr Ser Met Gly Lys Lys Thr Gln Arg Ser Leu
   245 250 255
   Ser Ala Glu Leu Glu Ile Pro Ala Val Lys Glu Ser Asp Ala Gly Lys
   260 265 270
   Tyr Tyr Cys Arg Ala Asp Asn Gly His Val Pro Ile Gln Ser Lys Val
   275 280 285
   Val Asn Ile Pro Val Arg Ile Pro Val Ser Arg Pro Val Leu Thr Leu
   290 295 300
   Arg Ser Pro Gly Ala Gln Ala Ala Val Gly Asp Leu Leu Glu Leu His
   305 310 315 320
   Cys Glu Ala Leu Arg Gly Ser Pro Pro Ile Leu Tyr Gln Phe Tyr His
   325 330 335
   Glu Asp Val Thr Leu Gly Asn Ser Ser Ala Pro Ser Gly Gly Gly Ala
   340 345 350
   Ser Phe Asn Leu Ser Leu Thr Ala Glu His Ser Gly Asn Tyr Ser Cys
   355 360 365
   Glu Ala Asn Asn Gly Leu Gly Ala Gln Cys Ser Glu Ala Val Pro Val
   370 375 380
   Ser Ile Ser Gly Pro Asp Gly Tyr Arg Arg Asp Leu Met Thr Ala Gly
   385 390 395 400
   Val Leu Trp Gly Leu Phe Gly Val Leu Gly Phe Thr Gly Val Ala Leu
   405 410 415
   Leu Leu Tyr Ala Leu Phe His Lys Ile Ser Gly Glu Ser Ser Ala Thr
   420 425 430
   Asn Glu Pro Arg Gly Ala Ser Arg Pro Asn Pro Gln Glu Phe Thr Tyr
   435 440 445
   Ser Ser Pro Thr Pro Asp Met Glu Glu Leu Gln Pro Val Tyr Val Asn
   450 455 460
   Val Gly Ser Val Asp Val Asp Val Val Tyr Ser Gln Val Trp Ser Met
   465 470 475 480
   Gln Gln Pro Glu Ser Ser Ala Asn Ile Arg Thr Leu Leu Glu Asn Lys
   485 490 495
   Asp Ser Gln Val Ile Tyr Ser Ser Val Lys Lys Ser
   500 505
<210> 8
   <211> 2580
   <212> DNA
   <213> Homo Sapiens
<400> 8
   tggtgaccaa gagtacatct cttttcaaat agctggatta ggtcctcatg ctgctgtggt 60
   cattgctggt catctttgat gcagtcactg aacaggcaga ttcgctgacc cttgtggcgc 120
   cctcttctgt cttcgaagga gacagcatcg ttctgaaatg ccagggagaa cagaactgga 180
   aaattcagaa gatggcttac cataaggata acaaagagtt atctgttttc aaaaaattct 240
   cagatttcct tatccaaagt gcagttttaa gtgacagtgg taactatttc tgtagtacca 300
   aaggacaact ctttctctgg gataaaactt caaatatagt aaagataaaa gtccaagagc 360
   tctttcaacg tcctgtgctg actgccagct ccttccagcc catcgaaggg ggtccagtga 420
   gcctgaaatg tgagacccgg ctctctccac agaggttgga tgttcaactc cagttctgct 480
   tcttcagaga aaaccaggtc ctggggtcag gctggagcag ctctccggag ctccagattt 540
   ctgccgtgtg gagtgaagac acagggtctt actggtgcaa ggcagaaacg gtgactcaca 600
   ggatcagaaa acagagcctc caatcccaga ttcacgtgca gagaatcccc atctctaatg 660
   taagcttgga gatccgggcc cccgggggac aggtgactga aggacaaaaa ctgatcctgc 720
   tctgctcagt ggctgggggt acaggaaatg tcacattctc ctggtacaga gaggccacag 780
   gaaccagtat gggaaagaaa acccagcgtt ccctgtcagc agagctggag atcccagctg 840
   tgaaagagag tgatgccggc aaatattact gtagagctga caacggccat gtgcctatcc 900
   agagcaaggt ggtgaatatc cctgtgagaa ttccagtgtc tcgccctgtc ctcaccctca 960
   ggtctcctgg ggcccaggct gcagtggggg acctgctgga gcttcactgt gaggccctga 1020
   gaggctctcc cccaatcttg taccaatttt atcatgagga tgtcaccctt gggaacagct    1080
   cggccccctc tggaggaggg gcctccttca acctctcttt gactgcagaa cattctggaa    1140
   actactcctg tgaggccaac aacggcctgg gggcccagtg cagtgaggca gtgccagtct 1200
   ccatctcagg acctgatggc tatagaagag acctcatgac agctggagtt ctctggggac 1260
   tgtttggtgt ccttggtttc actggtgttg ctttgctgtt gtatgccttg ttccacaaga 1320
   tatcaggaga aagttctgcc actaatgaac ccagaggggc ttccaggcca aatcctcaag 1380
   agttcaccta ttcaagccca accccagaca tggaggagct gcagccagtg tatgtcaatg 1440
   tgggctctgt agatgtggat gtggtttatt ctcaggtctg gagcatgcag cagccagaaa 1500
   gctcagcaaa catcaggaca cttctggaga acaaggactc ccaagtcatc tactcttctg 1560
   tgaagaaatc ataacacttg gaggaatcag aagggaagat caacagcaag gatggggcat 1620
   cattaagact tgctataaaa ccttatgaaa atgcttgagg cttatcacct gccacagcca 1680
   gaacgtgcct caggaggcac ctcctgtcat ttttgtcctg atgatgtttc ttctccaata 1740
   tcttctttta cctatcaata ttcattgaac tgctgctaca tccagacact gtgcaaataa 1800
   attatttctg ctaccttctc ttaagcaatc agtgtgtaaa gatttgaggg aagaatgaat 1860
   aagagataca aggtctcacc ttcatctact gtgaagtgat gagaacagga cttgatagtg 1920
   gtgtattaac ttatttatgt gctgctggat acagtttgct aatattttgt tgagaatttt 1980
   tgcaaatatg ttcattggga atattggcct gaaattttct tttccactgt gtctctgcca 2040
   gaatgtttgt atcaggctga tgctggcttc atagaatgag ttaggcagga gcccttcctc 2100
   cttgattttt tggcatagtt tcagcaggat tggtaccagt tattctttct gcatcttgta 2160
   gaattcagct atgaatccat ctggtctagg gcttttgtgt tggttggtaa gttttttatt 2220
   actaattcaa cttcagcgct tgatattggt ctaggagggg tttctgtctc ttcctggttc 2280
   aatcttggga gattgtgtgt ttccaggaat ttagccgttt cctccagatt ttcttcttta 2340
   tgtgcatcga cttgagtgta aacataactt atatgcactg ggaaaccaaa aaatctgtgt 2400
   gacttgcttt attgcagcat ttgttttatt ttggtagtct ggaactgaac ctgcaatatc 2460
   accaaagtat gcatatagtt gcaaaaatgt gatttttgac atagtaaata tgagtatttg 2520
   caataaacta tgatattact tttgtaagta tatagaataa aatgtaaata atctataaaa 2580
<210> 9
   <211> 429
   <212> PRT
   <213> Homo Sapiens
<400> 9
   Met Leu Pro Arg Leu Leu Leu Leu Ile Cys Ala Pro Leu Cys Glu Pro
   1               5                   10                  15
   Ala Glu Leu Phe Leu Ile Ala Ser Pro Ser His Pro Thr Glu Gly Ser
               20                  25                  30
   Pro Val Thr Leu Thr Cys Lys Met Pro Phe Leu Gln Ser Ser Asp Ala
           35                  40                  45
   Gln Phe Gln Phe Cys Phe Phe Arg Asp Thr Arg Ala Leu Gly Pro Gly
       50                  55                  60
   Trp Ser Ser Ser Pro Lys Leu Gln Ile Ala Ala Met Trp Lys Glu Asp
   65                  70                  75                  80
   Thr Gly Ser Tyr Trp Cys Glu Ala Gln Thr Met Ala Ser Lys Val Leu
                   85                  90                  95
   Arg Ser Arg Arg Ser Gln Ile Asn Val His Arg Val Pro Val Ala Asp
               100                 105                 110
   Val Ser Leu Glu Thr Gln Pro Pro Gly Gly Gln Val Met Glu Gly Asp
           115                 120                 125
   Arg Leu Val Leu Ile Cys Ser Val Ala Met Gly Thr Gly Asp Ile Thr
       130                 135                 140
   Phe Leu Trp Tyr Lys Gly Ala Val Gly Leu Asn Leu Gln Ser Lys Thr
   145                 150                 155                 160
   Gln Arg Ser Leu Thr Ala Glu Tyr Glu Ile Pro Ser Val Arg Glu Ser
                   165                 170                 175
   Asp Ala Glu Gln Tyr Tyr Cys Val Ala Glu Asn Gly Tyr Gly Pro Ser
               180                 185                 190
   Pro Ser Gly Leu Val Ser Ile Thr Val Arg Ile Pro Val Ser Arg Pro
           195                 200                 205
   Ile Leu Met Leu Arg Ala Pro Arg Ala Gln Ala Ala Val Glu Asp Val
       210                 215                 220
   Leu Glu Leu His Cys Glu Ala Leu Arg Gly Ser Pro Pro Ile Leu Tyr
   225                 230                 235                 240
   Trp Phe Tyr His Glu Asp Ile Thr Leu Gly Ser Arg Ser Ala Pro Ser
                   245                 250                 255
   Gly Gly Gly Ala Ser Phe Asn Leu Ser Leu Thr Glu Glu His Ser Gly
               260                 265                 270
   Asn Tyr Ser Cys Glu Ala Asn Asn Gly Leu Gly Ala Gln Arg Ser Glu
           275                 280                 285
   Ala Val Thr Leu Asn Phe Thr Val Pro Thr Gly Ala Arg Ser Asn His
       290                 295                 300
   Leu Thr Ser Gly Val Ile Glu Gly Leu Leu Ser Thr Leu Gly Pro Ala
   305                 310                 315                 320
   Thr Val Ala Leu Leu Phe Cys Tyr Gly Leu Lys Arg Lys Ile Gly Arg
                   325                 330                 335
   Arg Ser Ala Arg Asp Pro Leu Arg Ser Leu Pro Ser Pro Leu Pro Gln
               340                 345                 350
   Glu Phe Thr Tyr Leu Asn Ser Pro Thr Pro Gly Gln Leu Gln Pro Ile
           355                 360                 365
   Tyr Glu Asn Val Asn Val Val Ser Gly Asp Glu Val Tyr Ser Leu Ala
       370                 375                 380
   Tyr Tyr Asn Gln Pro Glu Gln Glu Ser Val Ala Ala Glu Thr Leu Gly
   385                 390                 395                 400
   Thr His Met Glu Asp Lys Val Ser Leu Asp Ile Tyr Ser Arg Leu Arg
                   405                 410                 415
   Lys Ala Asn Ile Thr Asp Val Asp Tyr Glu Asp Ala Met
               420                 425
<210> 10
   <211> 2303
   <212> DNA
   <213> Homo Sapiens
<900> 10
<210> 11
   <211> 90
   <212> DNA
   <213> Homo Sapiens
<400> 11
   gggcctgaca gcaacttttc ttctactagt tcatcttaac tttatcctgg taactggcga 60
   gacaacctgt cttaagtaac tgaagggaaa 90
<210> 13
   <211> 90
   <212> DNA
   <213> Homo Sapiens
<400> 13
   tcccactgac gcatgcagga aggggcacct ccccttaacc acactgctct gtacggggca 60
   cgtgggcaca ggtgcacact cacactcaca 90
<210> 14
   <211> 2499
   <212> DNA
   <213> HOMO SAPIENS
<400> 14
   ctcaatcagc tttatgcaga gaagaagctt actgagctca ctgctggtgc tggtgtaggc 60
   aagtgctgct ttggcaatct gggctgacct ggcttgtctc ctcagaactc cttctccaac 120
   cctggagcag gcttccatgc tgctgtgggc gtccttgctg gcctttgctc cagtctgtgg 180
   acaatctgca gctgcacaca aacctgtgat ttccgtccat cctccatgga ccacattctt 240
   caaaggagag agagtgactc tgacttgcaa tggatttcag ttctatgcaa cagagaaaac 300
   aacatggtat catcggcact actggggaga aaagttgacc ctgaccccag gaaacaccct 360
   cgaggttcgg gaatctggac tgtacagatg ccaggcccgg ggctccccac gaagtaaccc 420
   tgtgcgcttg ctcttttctt cagactcctt aatcctgcag gcaccatatt ctgtgtttga 480
   aggtgacaca ttggttctga gatgccacag aagaaggaaa gagaaattga ctgctgtgaa 540
   atatacttgg aatggaaaca ttctttccat ttctaataaa agctgggatc ttcttatccc 600
   acaagcaagt tcaaataaca atggcaatta tcgatgcatt ggatatggag atgagaatga 660
   tgtatttaga tcaaatttca aaataattaa aattcaagaa ctatttccac atccagagct 720
   gaaagctaca gactctcagc ctacagaggg gaattctgta aacctgagct gtgaaacaca 780
   gcttcctcca gagcggtcag acaccccact tcacttcaac ttcttcagag atggcgaggt     840
   catcctgtca gactggagca cgtacccgga actccagctc ccaaccgtct ggagagaaaa     900
   ctcaggatcc tattggtgtg gtgctgaaac agtgaggggt aacatccaca agcacagtcc     960
   ctcgctacag atccatgtgc agcggatccc tgtgtctggg gtgctcctgg agacccagcc    1020
   ctcagggggc caggctgttg aaggggagat gctggtcctt gtctgctccg tggctgaagg    1080
   cacaggggat accacattct cctggcaccg agaggacatg caggagagtc tggggaggaa    1140
   aactcagcgt tccctgagag cagagctgga gctccctgcc atcagacaga gccatgcagg    1200
   gggatactac tgtacagcag acaacagcta cggccctgtc cagagcatgg tgctgaatgt    1260
   cactgtgaga gagaccccag gcaacagaga tggccttgtc gccgcgggag ccactggagg    1320
   gctgctcagt gctcttctcc tggctgtggc cctgctgttt cactgctggc gtcggaggaa    1380
   gtcaggagtt ggtttcttgg gagacgaaac caggctccct cccgctccag gcccaggaga    1440
   gtcctcccat tccatctgcc ctgcccaggt ggagcttcag tcgttgtatg ttgatgtaca    1500
   ccccaaaaag ggagatttgg tatactctga gatccagact actcagctgg gagaagaaga    1560
   ggaagctaat acctccagga cacttctaga ggataaggat gtctcagttg tctactctga    1620
   ggtaaagaca caacacccag ataactcagc tggaaagatc agctctaagg atgaagaaag    1680
   ttaagagaat gaaaagttac gggaacgtcc tactcatgtg atttctccct tgtccaaagt    1740
   cccaggccca gtgcagtcct tgcggcacct ggaatgatca actcattcca gctttctaat    1800
   tcttctcatg catatgcatt cactcccagg aatactcatt cgtctactct gatgttggga    1860
   tggaatggcc tctgaaagac ttcactaaaa tgaccaggat ccacagttaa gagaagaccc    1920
   tgtagtattt gctgtgggcc tgacctaatg cattccctag ggtctgcttt agagaagggg    1980
   gataaagaga gagaaggact gttatgaaaa acagaagcac aaattttggt gaattgggat    2040
   ttgcagagat gaaaaagact gggtgacctg gatctctgct taatacatct acaaccattg    2100
   tctcactgga gactcacttg catcagtttg tttaactgtg agtggctgca caggcactgt    2160
   gcaaacaatg aaaagcccct tcacttctgc ctgcacagct tacactgtca ggattcagtt    2220
   gcagattaaa gaacccatct ggaatggttt acagagagag gaatttaaaa gaggacatca    2280
   gaagagctgg agatgcaagc tctaggctgc gcttccaaaa gcaaatgata attatgttaa    2340
   tgtcattagt gacaaagatt tgcaacatta gagaaaagag acacaaatat aaaattaaaa    2400
   acttaagtac caactctcca aaactaaatt tgaacttaaa atattagtat aaactcataa    2460
   taaactctgc ctttaaataa aaaaaaaaaa aaaaaaaaa                           2499
<210> 15
   <211> 515
   <212> PRT
   <213> Homo Sapiens
<900> 15
<210> 16
   <211> 2805
   <212> DNA
   <213> Homo Sapiens
<210> 17
   <211> 759
   <212> PRT
   <213> Homo Sapiens
<400> 17
   Met Leu Leu Trp Val Ile Leu Leu Val Leu Ala Pro Val Ser Gly Gln
   1 5 10 15
   Phe Ala Arg Thr Pro Arg Pro Ile Ile Phe Leu Gln Pro Pro Trp Thr
   20 25 30
   Thr Val Phe Gln Gly Glu Arg Val Thr Leu Thr Cys Lys Gly Phe Arg
   35 40 45
   Phe Tyr Ser Pro Gln Lys Thr Lys Trp Tyr His Arg Tyr Leu Gly Lys
   50 55 60
   Glu Ile Leu Arg Glu Thr Pro Asp Asn Ile Leu Glu Val Gln Glu Ser
   65 70 75 80
   Gly Glu Tyr Arg Cys Gln Ala Gln Gly Ser Pro Leu Ser Ser Pro Val
   85 90 95
   His Leu Asp Phe Ser Ser Ala Ser Leu Ile Leu Gln Ala Pro Leu Ser
   100 105 110
   Val Phe Glu Gly Asp Ser Val Val Leu Arg Cys Arg Ala Lys Ala Glu
   115 120 125
   Val Thr Leu Asn Asn Thr Ile Tyr Lys Asn Asp Asn Val Leu Ala Phe
   130 135 140
   Leu Asn Lys Arg Thr Asp Phe His Ile Pro His Ala Cys Leu Lys Asp
   145 150 155 160
   Asn Gly Ala Tyr Arg Cys Thr Gly Tyr Lys Glu Ser Cys Cys Pro Val
   165 170 175
   Ser Ser Asn Thr Val Lys Ile Gln Val Gln Glu Pro Phe Thr Arg Pro
   180 185 190
   Val Leu Arg Ala Ser Ser Phe Gln Pro Ile Ser Gly Asn Pro Val Thr
   195 200 205
   Leu Thr Cys Glu Thr Gln Leu Ser Leu Glu Arg Ser Asp Val Pro Leu
   210 215 220
   Arg Phe Arg Phe Phe Arg Asp Asp Gln Thr Leu Gly Leu Gly Trp Ser
   225 230 235 240
   Leu Ser Pro Asn Phe Gln Ile Thr Ala Met Trp Ser Lys Asp Ser Gly
   245 250 255
   Phe Tyr Trp Cys Lys Ala Ala Thr Met Pro His Ser Val Ile Ser Asp
   260 265 270
   Ser Pro Arg Ser Trp Ile Gln Val Gln Ile Pro Ala Ser His Pro Val
   275 280 285
   Leu Thr Leu Ser Pro Glu Lys Ala Leu Asn Phe Glu Gly Thr Lys Val
   290 295 300
   Thr Leu His Cys Glu Thr Gln Glu Asp Ser Leu Arg Thr Leu Tyr Arg
   305 310 315 320
   Phe Tyr His Glu Gly Val Pro Leu Arg His Lys Ser Val Arg Cys Glu
   325 330 335
   Arg Gly Ala Ser Ile Ser Phe Ser Leu Thr Thr Glu Asn Ser Gly Asn
   340 345 350
   Tyr Tyr Cys Thr Ala Asp Asn Gly Leu Gly Ala Lys Pro Ser Lys Ala
   355 360 365
   Val Ser Leu Ser Val Thr Val Pro Val Ser His Pro Val Leu Asn Leu
   370 375 380
   Ser Ser Pro Glu Asp Leu Ile Phe Glu Gly Ala Lys Val Thr Leu His
   385 390 395 400
   Cys Glu Ala Gln Arg Gly Ser Leu Pro Ile Leu Tyr Gln Phe His His
   405 410 415
   Glu Asp Ala Ala Leu Glu Arg Arg Ser Ala Asn Ser Ala Gly Gly Val
               420                     425                     430
   Ala Ile Ser Phe Ser Leu Thr Ala Glu His Ser Gly Asn Tyr Tyr Cys
           435                     440                     445
   Ala Thr Asp Asn Gly Phe Gly Pro Gln Arg Ser Lys Ala Val Ser Leu
       450                     455                     460
   Ser Ile Thr Val Pro Val Ser His Pro Val Leu Thr Leu Ser Ser Ala
   465                     470                     475                     480
   Glu Ala Leu Thr Phe Glu Gly Ala Thr Val Thr Leu His Cys Glu Val
                   485                     490                     495
   Gln Arg Gly Ser Pro Gln Ile Leu Tyr Gln Phe Tyr His Glu Asp Met
               500                     505                     510
   Pro Leu Trp Ser Ser Ser Thr Pro Ser Val Gly Arg Val Ser Phe Ser
           515                     520                     525
   Phe Ser Leu Thr Glu Gly His Ser Gly Asn Tyr Tyr Cys Thr Ala Asp
       530                     535                     540
   Asn Gly Phe Gly Pro Gln Arg Ser Glu Val Val Ser Leu Phe Val Thr
   545                     550                     555                     560
   Val Pro Val Ser Arg Pro Ile Leu Thr Leu Arg Val Pro Arg Ala Gln
                   565                     570                     575
   Ala Val Val Gly Asp Leu Leu Glu Leu His Cys Glu Ala Pro Arg Gly
               580                     585                     590
   Ser Pro Pro Ile Leu Tyr Trp Phe Tyr His Glu Asp Val Thr Leu Gly
           595                     600                     605
   Ser Ser Ser Ala Pro Ser Gly Gly Glu Ala Ser Phe Asn Leu Ser Leu
       610                     615                     620
   Thr Ala Glu His Ser Gly Asn Tyr Ser Cys Glu Ala Asn Asn Gly Leu
   625                     630                     635                     640
   Val Ala Gln His Ser Asp Thr Ile Ser Leu Ser Val Ile Val Pro Val
                   645                     650                     655
   Ser Arg Pro Ile Leu Thr Phe Arg Ala Pro Arg Ala Gln Ala Val Val
               660                 665                 670
   Gly Asp Leu Leu Glu Leu His Cys Glu Ala Leu Arg Gly Ser Ser Pro
           675                 680                 685
   Ile Leu Tyr Trp Phe Tyr His Glu Asp Val Thr Leu Gly Lys Ile Ser
       690                 695                 700
   Ala Pro Ser Gly Gly Gly Ala Ser Phe Asn Leu Ser Leu Thr Thr Glu
   705                 710                 715                 720
   His Ser Gly Ile Tyr Ser Cys Glu Ala Asp Asn Gly Leu Glu Ala Gln
                   725                 730                 735
   Arg Ser Glu Met Val Thr Leu Lys Val Ala Gly Glu Trp Ala Leu Pro
               740                 745                 750
   Thr Ser Ser Thr Ser Glu Asn
           755
<210> 18
   <211> 4448
   <212> DNA
   <213> Homo Sapiens
<400> 18
   cggtgcagtg tcctgactgt aagatcaagt ccaaacctgt tttggaattg aggaaacttc      60
   tcttttgatc tcagcccttg gtggtccagg tcttcatgct gctgtgggtg atattactgg     120
   tcctggctcc tgtcagtgga cagtttgcaa ggacacccag gcccattatt ttcctccagc     180
   ctccatggac cacagtcttc caaggagaga gagtgaccct cacttgcaag ggatttcgct     240
   tctactcacc acagaaaaca aaatggtacc atcggtacct tgggaaagaa atactaagag     300
   aaaccccaga caatatcctt gaggttcagg aatctggaga gtacagatgc caggcccagg     360
   gctcccctct cagtagccct gtgcacttgg atttttcttc agcttcgctg atcctgcaag     420
   ctccactttc tgtgtttgaa ggagactctg tggttctgag gtgccgggca aaggcggaag     480
   taacactgaa taatactatt tacaagaatg ataatgtcct ggcattcctt aataaaagaa     540
   ctgacttcca tattcctcat gcatgtctca aggacaatgg tgcatatcgc tgtactggat     600
   ataaggaaag ttgttgccct gtttcttcca atacagtcaa aatccaagtc caagagccat 660
   ttacacgtcc agtgctgaga gccagctcct tccagcccat cagcgggaac ccagtgaccc 720
   tgacctgtga gacccagctc tctctagaga ggtcagatgt cccgctccgg ttccgcttct     780
   tcagagatga ccagaccctg ggattaggct ggagtctctc cccgaatttc cagattactg     840
   ccatgtggag taaagattca gggttctact ggtgtaaggc agcaacaatg cctcacagcg 900
   tcatatctga cagcccgaga tcctggatac aggtgcagat ccctgcatct catcctgtcc 960
   tcactctcag ccctgaaaag gctctgaatt ttgagggaac caaggtgaca cttcactgtg 1020
   aaacccagga agattctctg cgcactttgt acaggtttta tcatgagggt gtccccctga 1080
   ggcacaagtc agtccgctgt gaaaggggag catccatcag cttctcactg actacagaga 1140
   attcagggaa ctactactgc acagctgaca atggccttgg cgccaagccc agtaaggctg 1200
   tgagcctctc agtcactgtt cccgtgtctc atcctgtcct caacctcagc tctcctgagg 1260
   acctgatttt tgagggagcc aaggtgacac ttcactgtga agcccagaga ggttcactcc 1320
   ccatcctgta ccagtttcat catgaggatg ctgccctgga gcgtaggtcg gccaactctg 1380
   caggaggagt ggccatcagc ttctctctga ctgcagagca ttcagggaac tactactgca 1440
   cagctgacaa tggctttggc ccccagcgca gtaaggcggt gagcctctcc atcactgtcc 1500
   ctgtgtctca tcctgtcctc accctcagct ctgctgaggc cctgactttt gaaggagcca 1560
   ctgtgacact tcactgtgaa gtccagagag gttccccaca aatcctatac cagttttatc 1620
   atgaggacat gcccctgtgg agcagctcaa caccctctgt gggaagagtg tccttcagct 1680
   tctctctgac tgaaggacat tcagggaatt actactgcac agctgacaat ggctttggtc 1740
   cccagcgcag tgaagtggtg agcctttttg tcactggtaa gtgctgggtt cttgccagtc 1800
   acccacccct ggctgagttc tctctcaccc attcctttaa aaatctgttt gcactgtcca 1860
   gtttcctccc ctaatcaact taatcccctt cttggcttcc tcctcaacta actagctggg 1920
   gttttccgta ctcataagtc ctggctcagc cagaccccta aaacagctca gtagattccc 1980
   cagcttttac caaatgaatt tatttattgt attttctcct cattccttgt atgttccaac 2040
   agtacgccaa tttttcttga tgcacggagc gtgtcctact tctctactga catttacata 2100
   ttaacttagc tacaagcaca gtcttataga taaatattgg tcaagacctt aaattctcca 2160
   aaggatttcc aatcttatgg tagatttgga gaaagctgct ggtgaacaaa gggggaaatg 2220
   gctccctagg aaccaactcc tcaaacttct ggagttttta tgatcccttg ttttctaacc 2280
   tgctaaaatc agtatcattt tattgtatta ttttaaaaaa actattgttg aagtatgaca 2340
   tacattcaag aaacgtgtgc aaattgtatg tgtacgattt ggtgtctttt taggagctaa 2400
   gttgcttctg tttttacttg aatctttgtt tatagaaact gggggaaagt ttactttctt 2460
   ttcagagaag ccaaatggta tgatagaaaa atcttgagcc tgatgtgtca gacatgcccc 2520
   tagcataact tgttgagtaa agaggttatt tttaaaatgt gaatgttctg agactactcc 2580
   aaagtcagag ccaaatctac taggaagctt ctagacttca ctcattctgc atcccattac 2640
   tatcttttta tccatgtttt actttcttct catattcagc agcatcttaa gcctctttat 2700
   tttctgtttc ttgactgtca cccttaatgc cagtagaatg taagcttcat gagaacagaa 2760
   ctgcatccat cttggtcttc acaacatccc tgtgcctact cagtgtttgg cacacagtag 2820
   gtcctcagtc aacatttgta atttagtgga cagatgatat gacaagatga taagagggga 2880
   tttaaaaaaa tcatctagca aagcccaaga ggaaaaaaaa caaagctatt ttagaaatga 2940
   aataccaatt tgaagcagta agaatagatt ggatatcttt gaaaaccatt aattgaatga 3000
   agaaccaatt tgagaaaaca atacagaatg caaagtagaa agatacagaa ataaaggcaa 3060
   aagttataat atggaaatca gacaatggat ttgtctgtat ccagttatgt ggataattaa 3120
   aatggagacc ctcagaaaat tgaaccgaag agtaaaatga aactcaaaaa tgtagtagaa 3180
   attgttggga agtaaagaaa acttgaatat gtagatcaga acatatatgt tgatgacgtt 3240
   attgactttg aggttaaaaa tatatatatg tgcctatgat tatggggaaa aaagcagtcg 3300
   tctcagaaag aaaaacatca agttagtctt agactttgca gtgcactcag taccaaagag 3360
   agaggaggcc agacttggac ctgcgaggga agaataataa ccgaaaattt tatatcaatt 3420
   caaaaagaca ttgtcaaaaa tacagggatt caggaaactg agaatgcact aagccttctg 3480
   gaaaaaacac ctaatgacaa aatctagccc aacaagatgt aaatgaatat aaaggactca 3540
   taatgaggaa accgcattat gactggctct caaccctggc cgcatattag actcgtcaaa 3600
   gacctttgta aaaggtcaca cattgactcg tcaaagcccc tctccagact aattcaattc 3660
   agaatctcac agatggggcc acagaatcag tattttttga cacaacctca agtgagaata 3720
   ttgtgtagac aagattggaa accactgatt tagatataga aacaaaggct aatcaactgt 3780
   gagaattatg gtcacagaat agaaagtaac tattatgaac actgaaaatg taaaaaaaat 3840
   gtaacaaaga aaaatagtta gaggaaggag aggaagtaaa ggaacaatca ttttctcatg 3900
   attattatta tttcagagta aattgtgagt tatttcacaa ttcaaaaaga atggactgtt 3960
   ttaaaaaatt agtaatagat ttcaaaatgt ccattttgta aatcgtttct gaatactttg 4020
   tcaacagtta ctcatcatta atggcttata cttcactaaa attccatgga aaaccaacta 4080
   gtagcctgta gagtcacata ggagagaaca agtgaattct ttgggtggcg caagcataga 4140
   tgttaggact gacaaaaaaa aataataaaa ataaacctgt gcattgatat gatcacaaat 4200
   gatcagggaa agaggaaaca gaaactctca tacgccatta ttacaagtgt aaattggttc 4260
   aaccttttcg tcttaattga cacattgtaa ttgtatatat ttatggaagc acagtttgat   4320
   attttgatat acatacatgg tatataacga tcaaattagg atatttaatg tacccatcat   4380
   ctcatgcatt tatcatttct ttggaataaa aacattcaaa agccaaaaaa aaaaaaaaaa   4440
   aaaaaaaa                                                             4448
<210> 19
   <211> 592
   <212> PRT
   <213> Homo Sapiens
<400> 19
   Met Leu Leu Trp Val Ile Leu Leu Val Leu Ala Pro Val Ser Gly Gln
   1               5                   10                  15
   Phe Ala Arg Thr Pro Arg Pro Ile Ile Phe Leu Gln Pro Pro Trp Thr
               20                  25                  30
   Thr Val Phe Gln Gly Glu Arg Val Thr Leu Thr Cys Lys Gly Phe Arg
           35                  40                  45
   Phe Tyr Ser Pro Gln Lys Thr Lys Trp Tyr His Arg Tyr Leu Gly Lys
       50                  55                  60
   Glu Ile Leu Arg Glu Thr Pro Asp Asn Ile Leu Glu Val Gln Glu Ser
   65                  70                  75                  80
   Gly Glu Tyr Arg Cys Gln Ala Gln Gly Ser Pro Leu Ser Ser Pro Val
                   85                  90                  95
   His Leu Asp Phe Ser Ser Ala Ser Leu Ile Leu Gln Ala Pro Leu Ser
               100                 105                 110
   Val Phe Glu Gly Asp Ser Val Val Leu Arg Cys Arg Ala Lys Ala Glu
           115                 120                 125
   Val Thr Leu Asn Asn Thr Ile Tyr Lys Asn Asp Asn Val Leu Ala Phe
       130                 135                 140
   Leu Asn Lys Arg Thr Asp Phe His Ile Pro His Ala Cys Leu Lys Asp
   145                 150                 155                 160
   Asn Gly Ala Tyr Arg Cys Thr Gly Tyr Lys Glu Ser Cys Cys Pro Val
   165 170 175
   Ser Ser Asn Thr Val Lys Ile Gln Val Gln Glu Pro Phe Thr Arg Pro
   180 185 190
   Val Leu Arg Ala Ser Ser Phe Gln Pro Ile Ser Gly Asn Pro Val Thr
   195 200 205
   Leu Thr Cys Glu Thr Gln Leu Ser Leu Glu Arg Ser Asp Val Pro Leu
   210 215 220
   Arg Phe Arg Phe Phe Arg Asp Asp Gln Thr Leu Gly Leu Gly Trp Ser
   225 230 235 240
   Leu Ser Pro Asn Phe Gln Ile Thr Ala Met Trp Ser Lys Asp Ser Gly
   245 250 255
   Phe Tyr Trp Cys Lys Ala Ala Thr Met Pro His Ser Val Ile Ser Asp
   260 265 270
   Ser Pro Arg Ser Trp Ile Gln Val Gln Ile Pro Ala Ser His Pro Val
   275 280 285
   Leu Thr Leu Ser Pro Glu Lys Ala Leu Asn Phe Glu Gly Thr Lys Val
   290 295 300
   Thr Leu His Cys Glu Thr Gln Glu Asp Ser Leu Arg Thr Leu Tyr Arg
   305 310 315 320
   Phe Tyr His Glu Gly Val Pro Leu Arg His Lys Ser Val Arg Cys Glu
   325 330 335
   Arg Gly Ala Ser Ile Ser Phe Ser Leu Thr Thr Glu Asn Ser Gly Asn
   340 345 350
   Tyr Tyr Cys Thr Ala Asp Asn Gly Leu Gly Ala Lys Pro Ser Lys Ala
   355 360 365
   Val Ser Leu Ser Val Thr Val Pro Val Ser His Pro Val Leu Asn Leu
   370 375 380
   Ser Ser Pro Glu Asp Leu Ile Phe Glu Gly Ala Lys Val Thr Leu His
   385 390 395 400
   Cys Glu Ala Gln Arg Gly Ser Leu Pro Ile Leu Tyr Gln Phe His His
                   405                 410                 415
   Glu Asp Ala Ala Leu Glu Arg Arg Ser Ala Asn Ser Ala Gly Gly Val
               420                 425                 430
   Ala Ile Ser Phe Ser Leu Thr Ala Glu His Ser Gly Asn Tyr Tyr Cys
           435                 440                 445
   Thr Ala Asp Asn Gly Phe Gly Pro Gln Arg Ser Lys Ala Val Ser Leu
       450                 455                 460
   Ser Ile Thr Val Pro Val Ser His Pro Val Leu Thr Leu Ser Ser Ala
   465                 470                 475                 480
   Glu Ala Leu Thr Phe Glu Gly Ala Thr Val Thr Leu His Cys Glu Val
                   485                 490                 495
   Gln Arg Gly Ser Pro Gln Ile Leu Tyr Gln Phe Tyr His Glu Asp Met
               500                 505                 510
   Pro Leu Val Ser Ser Ser Thr Pro Ser Val Gly Arg Val Ser Phe Ser
           515                 520                 525
   Phe Ser Leu Thr Glu Gly His Ser Gly Asn Tyr Tyr Cys Thr Ala Asp
       530                 535                 540
   Asn Gly Phe Gly Pro Gln Arg Ser Glu Val Val Ser Leu Phe Val Thr
   545                 550                 555                 560
   Gly Lys Cys Trp Val Leu Ala Ser Lys Pro Pro Leu Ala Glu Phe Ser
                   565                 570                 575
   Leu Thr His Ser Phe Lys Asn Leu Phe Ala Leu Ser Ser Phe Leu Pro
               580                 585                 590
<210> 20
   <211> 5323
   <212> DNA
   <213> Homo Sapiens
<900> 20
<210> 21
   <211> 977
   <212> PRT
   <213> Homo sapiens
<400> 21
   Met Leu Leu Trp Val Ile Leu Leu Val Leu Ala Pro Tyr Ser Gly Gln
   1               5                   10                  15
   Phe Ala Arg Thr Pro Arg Pro Ile Ile Phe Leu Gln Pro Pro Trp Thr
               20                  25                  30
   Thr Val Phe Gln Gly Glu Arg Val Thr Leu Thr Cys Lys Gly Phe Arg
           35                  40                  45
   Phe Tyr Ser Pro Gln Lys Thr Lys Trp Tyr His Arg Tyr Leu Gly Lys
       50                  55                  60
   Glu Ile Leu Arg Glu Thr Pro Asp Asn Ile Leu Glu Val Gln Glu Ser
   65                  70                  75                  80
   Gly Ser Tyr Arg Cys Gln Ala Gln Gly Ser Pro Leu Ser Ser Pro Val
                   85                  90                  95
   His Leu Asp Phe Ser Ser Ala Ser Leu Ile Leu Gln Ala Pro Leu Ser
               100                 105                 110
   Val Phe Glu Gly Asp Ser Val Val Leu Arg Cys Arg Ala Lys Ala Glu
           115                 120                 125
   Val Thr Leu Asn Asn Thr Ile Tyr Lys Asn Asp Asn Val Leu Ala Phe
       130                 135                 140
   Leu Asn Lys Arg Thr Asp Phe His Ile Pro His Ala Cys Leu Lys Asp
   145                 150                 155                 160
   Asn Gly Ala Tyr Arg Cys Thr Gly Tyr Lys Glu Ser Cys Cys Pro Val
                   165                 170                 175
   Ser Ser Asn Thr Val Lys Ile Gln Val Gln Glu Pro Phe Thr Arg Pro
               180                 185                 190
   Val Leu Arg Ala Ser Ser Phe Gln Pro Ile Ser Gly Asn Pro Val Thr
           195                 200                 205
   Leu Thr Cys Glu Thr Gln Leu Ser Leu Glu Arg Ser Asp Val Pro Leu
   210 215 220
   Arg Phe Arg Phe Phe Arg Asp Asp Gln Thr Leu Gly Leu Gly Trp Ser
   225 230 235 240
   Leu Ser Pro Asn Phe Gln Ile Thr Ala Met Trp Ser Lys Asp Ser Gly
   245 250 255
   Phe Tyr Trp Cys Lys Ala Ala Thr Met Pro His Ser Val Ile Ser Asp
   260 265 270
   Ser Pro Arg Ser Trp Ile Gln Val Gln Ile Pro Ala Ser His Pro Val
   275 280 285
   Leu Thr Leu Ser Pro Glu Lys Ala Leu Asn Phe Glu Gly Thr Lys Val
   290 295 300
   Thr Leu His Cys Glu Thr Gln Glu Asp Ser Leu Arg Thr Leu Tyr Arg
   305 310 315 320
   Phe Tyr His Glu Gly Val Pro Leu Arg His Lys Ser Val Arg Cys Glu
   325 330 335
   Arg Gly Ala Ser Ile Ser Phe Ser Leu Thr Thr Glu Asn Ser Gly Asn
   340 345 350
   Tyr Tyr Cys Thr Ala Asp Asn Gly Leu Gly Ala Lys Pro Ser Lys Ala
   355 360 365
   Val Ser Leu Ser Val Thr Val Pro Val Ser His Pro Val Leu Asn Leu
   370 375 380
   Ser Ser Pro Glu Asp Leu Ile Phe Glu Gly Ala Lys Val Thr Leu His
   385 390 395 400
   Cys Glu Ala Gln Arg Gly Ser Leu Pro Ile Leu Tyr Gln Phe His His
   405 410 415
   Glu Asp Ala Ala Leu Glu Arg Arg Ser Ala Asn Ser Ala Gly Gly Val
   420 425 430
   Ala Ile Ser Phe Ser Leu Thr Ala Glu His Ser Gly Asn Tyr Tyr Cys
   435 440 445
   Thr Ala Asp Asn Gly Phe Gly Pro Gln Arg Ser Lys Ala Val Ser Leu
   450 455 460
   Ser Ile Thr Val Pro Val Ser His Pro Val Leu Thr Leu Ser Ser Ala
   465 470 475 480
   Glu Ala Leu Thr Phe Glu Gly Ala Thr Val Thr Leu His Cys Glu Val
   485 490 495
   Gln Arg Gly Ser Pro Gln Ile Leu Tyr Gln Phe Tyr His Glu Asp Met
   500 505 510
   Pro Leu Trp Ser Ser Ser Thr Pro Ser Val Gly Arg Val Ser Phe Ser
   515 520 525
   Phe Ser Leu Thr Glu Gly His Ser Gly Asn Tyr Tyr Cys Thr Ala Asp
   530 535 540
   Asn Gly Phe Gly Pro Gln Arg Ser Glu Val Val Ser Leu Phe Val Thr
   545 550 555 560
   Val Pro Val Ser Arg Pro Ile Leu Thr Leu Arg Val Pro Arg Ala Gln
   565 570 575
   Ala Val Val Gly Asp Leu Leu Glu Leu His Cys Glu Ala Pro Arg Gly
   580 585 590
   Ser Pro Pro Ile Leu Tyr Trp Phe Tyr His Glu Asp Val Thr Leu Gly
   595 600 605
   Ser Ser Ser Ala Pro Ser Gly Gly Glu Ala Ser Phe Asn Leu Ser Leu
   610 615 620
   Thr Ala Glu His Ser Gly Asn Tyr Ser Cys Glu Ala Asn Asn Gly Leu
   625 630 635 640
   Val Ala Gln His Ser Asp Thr Ile Ser Leu Ser Val Ile Val Pro Val
   645 650 655
   Ser Arg Pro Ile Leu Thr Phe Arg Ala Pro Arg Ala Gln Ala Val Val
   660 665 670
   Gly Asp Leu Leu Glu Leu His Cys Glu Ala Leu Arg Gly Ser Ser Pro
   675 680 685
   Ile Leu Tyr Trp Phe Tyr His Glu Asp Val Thr Leu Gly Lys Ile Ser
       690                 695                 700
   Ala Pro Ser Gly Gly Gly Ala Ser Phe Asn Leu Ser Leu Thr Thr Glu
   705                 710                 715                 720
   His Ser Gly Ile Tyr Ser Cys Glu Ala Asp Asn Gly Leu Glu Ala Gln
                   725                 730                 735
   Arg Ser Glu Met Val Thr Leu Lys Val Ala Val Pro Val Ser Arg Pro
               740                 745                 750
   Val Leu Thr Leu Arg Ala Pro Gly Thr His Ala Ala Val Gly Asp Leu
           755                 760                 765
   Leu Thr Glu Leu His Cys Glu Ala Leu Arg Gly Ser Pro Leu Ile Leu
       770                 775                 780
   Tyr Arg Phe Phe His Glu Asp Val Thr Leu Gly Asn Glu Leu His Cys
   785                 790                 795                 800
   Glu Ala Leu Arg Gly Ser Pro Leu Ile Leu Tyr Arg Phe Phe His Glu
                   805                 810                 815
   Asp Val Thr Leu Gly Asn Asn Gly Leu Gly Ala Gln Arg Ser Glu Thr
               820                 825                 830
   Val Thr Leu Tyr Ile Thr Gly Leu Thr Ala Asn Arg Ser Gly Pro Phe
           835                 840                 845
   Ala Thr Gly Val Ala Gly Gly Leu Leu Ser Ile Ala Gly Leu Ala Ala
       850                 855                 860
   Gly Ala Leu Leu Leu Tyr Cys Trp Leu Ser Arg Lys Ala Gly Arg Lys
   865                 870                 875                 880
   Pro Ala Ser Asp Pro Ala Arg Ser Pro Ser Asp Ser Asp Ser Gln Glu
                   885                 890                 895
   Pro Thr Tyr His Met Val Pro Ala Trp Glu Glu Leu Gln Pro Val Tyr
               900                 905                 910
   Thr Asn Ala Asn Pro Arg Gly Glu Asn Val Val Tyr Ser Glu Val Arg
           915                 920                 925
   Ile Ile Gln Glu Lys Lys Lys His Ala Val Ala Ser Asp Pro Arg His
       930                 935                 940
   Leu Arg Asn Lys Gly Ser Pro Ile Ile Tyr Ser Glu Val Lys Val Ala
   945                 950                 955                 960
   Ser Thr Pro Val Ser Gly Ser Leu Phe Leu Ala Ser Ser Ala Pro His
                   965                 970                 975
   Arg
<210> 22
   <211> 88
   <212> PRT
   <213> Homo Sapiens
<400> 22
   Met Leu Leu Trp Ala Ser Leu Leu Ala Phe Ala Pro Val Cys Gly Gln
   1               5                   10                  15
   Ser Gly Ser Cys Ser Val Ala Asp Trp Gln Met Pro Pro Pro Tyr Val
               20                  25                  30
   Val Leu Asp Leu Pro Gln Glu Thr Leu Glu Glu Glu Thr Pro Gly Ala
           35                  40                  45
   Asn Leu Trp Pro Thr Thr Ile Thr Phe Leu Thr Leu Phe Leu Leu Ser
       50                  55                  60
   Leu Phe Tyr Ser Thr Ala Leu Thr Val Thr Ser Val Arg Gly Pro Ser
   65                  70                  75                  80
   Gly Asn Arg Glu Gly Pro Gln Tyr
                   85
<210> 23
   <211> 837
   <212> DNA
   <213> Homo Sapiens
<400> 23
   ctcaatcagc tttatgcaga gaagaagctt actgagctca ctgctggtgc tggtgtaggc 60
   aagtgctgct ttggcaatct gggctgacct ggcttgtctc ctcagaactc cttctccaac 120
   cctggagcag gcttccatgc tgctgtgggc gtccttgctg gcctttgctc cagtctgtgg 180
   acaatctggc tcttgctctg ttgcagattg gcagatgccg cctccctatg tggtgctgga 240
   cttgccgcag gagaccctgg aggaggagac ccccggcgcc aacctgtggc ccaccaccat 300
   caccttcctc accctcttcc tgctgagcct gttctatagc acagcactga ccgtgaccag 360
   cgtccggggc ccatctggca acagggaggg cccccagtac tgagcgggag ccggcaaggc 420
   acaggtggga gcccaggagg gggatgagcc cacagtggat gaggtgggct gcagtgcttg 480
   gctaagagga gagcaccacc tgctcccact gtggggggac gtgctctcct ggggggccct 540
   tcacagacac tgaggacacg cgcaggccca gggtcagggc tgagcttccc tccagtgcag 600
   taacgaggat tccgtccagg ctcccatgag caggccaggg ctgagacaga gggcgttggc 660
   aaggatgctg ctcttcaggc tgtgacccct ctgtctttgc agggaggaag tgtggaggaa 720
   cctcttggag aagccagcta tgcttgccag aactcagccc tttcagacgt caccgacccg 780
   cccttactca catgccttcc aggtgcaata aagtggcccc aaggaaaaaa aaaaaaa 837
<210> 24
   <211> 90
   <212> DNA
   <213> Homo Sapiens
<400> 24
   tcccactgac gcatgcagga aggggcacct ccccttaacc acactgctct gtacggggca 60
   cgtgggcaca ggtgcacact cacactcaca 90
<210> 25
   <211> 89
   <212> DNA
   <213> Homo Sapiens
<400> 25
   ggcctgacag caacttttct tctactagtt catcttaaca cactgctctg tacggggcac 60
   gtgggcacag gtgcacactc acactcaca 89
<210> 26
   <211> 89
   <212> DNA.
   <213> Homo Sapiens.
<400> 26
   ggcctgacag caacttttct tctactagtt catcttaact ttatcctggt aactggcgag 60
   acaacctgtc ttaagtaact gaagggaaa 89
<210> 27
   <211> 77
   <212> DNA
   <213> Homo Sapiens
<400> 27
   tcccactgac gcaggaagga tcttaagttt atcctggtaa ctggcgagac aacctgtctt 60
   aagtaactga agggaaa 77
<210> 28
   <211> 200
   <212> PRT
   <213> Homo Sapiens
<400> 28
   Met Ala Met Glu Thr Gln Met Ser Gln Asn Val Cys Pro Arg Asn Leu
   1               5                   10                  15
   Trp Leu Leu Gln Pro Leu Thr Val Leu Leu Leu Leu Ala Ser Ala Asp
               20                  25                  30
   Ser Gln Ala Ala Ala Pro Pro Lys Ala Val Leu Lys Leu Glu Pro Pro
           35                  40                  45
   Trp Ile Asn Val Leu Gln Glu Asp Ser Val Thr Leu Thr Cys Cys Gly
       50                  55                  60
   Ala Arg Ser Pro Glu Ser Pro Ser Ile Gln Trp Phe His His Asn Gly
   65 70 75 80
   Asn Leu Ile Pro Ile His Thr Gln Ser Ser Tyr Arg Phe Lys Ala Asn
   85 90 95
   Asn Asn Asp Ser Gly Glu Tyr Thr Cys Gln Thr Gly Gln Thr Ser Leu
   100 105 110
   Ser Asp Pro Val His Leu Thr Val Leu Ser Glu Trp Leu Leu Leu Gln
   115 120 125
   Thr Pro His Leu Glu Phe Gln Glu Gly Glu Thr Ile Asn Leu Arg Cys
   130 135 140
   His Ser Trp Lys Asp Lys Pro Leu Val Lys Val Thr Glu Glu Gln Asn
   145 150 155 160
   Gly Lys Ser Gln Lys Phe Ser Arg Leu Asp Pro Thr Phe Ser Ile Pro
   165 170 175
   Gln Ala Asn His Ser His Ser Gly Asp Tyr His Cys Thr Gly Asn Cys
   180 185 190
   Gly Tyr Thr Leu Phe Ser Ser Lys
   195 200
<210> 29
   <211> 184
   <212> PRT
   <213> Homo Sapiens
<400> 29
   Met Trp Gln Leu Leu Leu Pro Thr Ala Leu Leu Leu Leu Val Ser Ala
   1 5 10 15
   Gly Met Arg Thr Glu Asp Leu Pro Lys Ala Val Val Phe Leu Glu Pro
   20 25 30
   Gln Trp Tyr Arg Val Leu Glu Lys Asp Ser Val Thr Leu Lys Cys Cys
   35 40 45
   Gly Ala Tyr Ser Pro Glu Leu Asn Ser Thr Gln Trp Phe His Asn Glu
       50                  55                  60
   Ser Leu Ile Ser Glu Gln Ala Ser Ser Tyr Phe Ile Asp Ala Ala Thr
   65                  70                  75                  80
   Val Asp Asp Ser Gly Glu Tyr Arg Cys Gln Thr Asn Leu Ser Thr Leu
                   85                  90                  95
   Ser Asp Pro Val Gln Leu Glu Val His Ile Gly Trp Leu Leu Leu Gln
               100                 105                 110
   Ala Pro Arg Trp Val Phe Lys Glu Glu Asp Pro Ile His Leu Arg Cys
           115                 120                 125
   His Ser Trp Lys Asn Thr Ala Leu His Lys Val Thr Tyr Leu Gln Asn
       130                 135                 140
   Gly Lys Gly Arg Lys Tyr Phe His His Asn Ser Asp Phe Tyr Ile Pro
   145                 150                 155                 160
   Gln Ala Thr Leu Lys Asp Ser Gly Ser Tyr Phe Cys Arg Gly Leu Phe
                   165                 170                 175
   Gly Ser Lys Asn Val Ser Ser Glu
               180
<210> 30
   <211> 188
   <212> PRT
   <213> Homo Sapiens
<400> 30
   Met Ala Pro Ala Met Glu Ser Pro Thr Leu Leu Cys Val Ala Leu Leu
   1               5                   10                  15
   Phe Phe Ala Asp Asp Gly Val Leu Ala Val Pro Gln Lys Pro Lys Val
               20                  25                  30
   Ser Leu Asn Pro Pro Trp Asn Arg Ile Phe Lys Gly Glu Asn Val Thr
           35                  40                  45
   Leu Thr Cys Asn Gly Asn Asn Phe Phe Glu Val Ser Ser Thr Lys Trp
   50 55 60
   Phe His Asn Gly Ser Leu Ser Glu Ser Thr Asn Ser Ser Leu Asn Ile
   65 70 75 80
   Val Asn Ala Lys Phe Glu Asp Ser Gly Glu Tyr Lys Cys Gln His Gln
   85 90 95
   Gln Val Asn Glu Ser Glu Pro Val Tyr Leu Glu Val Phe Ser Asp Trp
   100 105 110
   Leu Leu Leu Gln Ala Ser Ala Glu Val Val Met Glu Gly Gln Pro Leu
   115 120 125
   Phe Leu Arg Cys His Gly Trp Arg Asn Trp Pro Val Tyr Lys Val Ile
   130 135 140
   Tyr Tyr Lys Asp Gly Glu Ala Leu Lys Tyr Trp Tyr Glu Asn His Asn
   145 150 155 160
   Ile Ser Ile Thr Asn Ala Thr Val Glu Asp Ser Gly Thr Tyr Tyr Cys
   165 170 175
   Thr Gly Lys Val Trp Gln Leu Asp Tyr Glu Ser Glu
   180 185
<210> 31
   <211> 378
   <212> PRT
   <213> Homo Sapiens
<400> 31
   Met Trp Phe Leu Thr Thr Leu Leu Leu Trp Val Pro Val Asp Gly Gln
   1 5 10 15
   Val Asp Thr Thr Lys Ala Val Ile Ser Leu Gln Pro Pro Trp Val Ser
   20 25 30
   Phe Val Gln Glu Glu Thr Val Thr Leu His Cys Glu Val Leu His Leu
   35 40 45
   Pro Gly Ser Ser Ser Thr Gln Trp Phe Leu Asn Gly Thr Ala Thr Gln
   50 55 60
   Thr Ser Thr Pro Ser Tyr Arg Ile Thr Ser Ala Ser Val Asn Asp Ser
   65 70 75 80
   Gly Glu Tyr Arg Cys Gln Arg Gly Leu Ser Gly Arg Ser Asp Pro Thr
   85 90 95
   Trp Leu Glu Thr His Arg Gly Trp Leu Leu Leu Gln Tyr Ser Ser Arg
   100 105 110
   Val Phe Thr Glu Gly Glu Pro Leu Ala Leu Arg Cys His Ala Trp Lys
   115 120 125
   Asp Lys Leu Val Tyr Asn Val Leu Tyr Tyr Arg Asn Gly Lys Ala Phe
   130 135 140
   Lys Phe Phe His Trp Asn Ser Asn Leu Ile Ile Leu Lys Ile Asn Ile
   145 150 155 160
   Ser Ser His Asn Gly Thr Tyr His Cys Ser Gly Asn Gly Lys His Arg
   165 170 175
   Tyr Thr Ser Ala Gly Lys His Arg Tyr Thr Ser Ala Gly Ile Ser Val
   180 185 190
   Thr Val Lys Glu Leu Phe Pro Ala Pro Val Leu Asn Ala Ser Val Thr
   195 200 205
   Ser Pro Leu Leu Glu Gly Asn Leu Val Thr Leu Ser Cys Glu Thr Lys
   210 215 220
   Leu Leu Leu Gln Arg Pro Gly Leu Gln Leu Tyr Phe Ser Phe Tyr Met
   225 230 235 240
   Gly Ser Leu Thr Leu Arg Gly Arg Asn Thr Ser Ser Glu Tyr Gln Ile
   245 250 255
   Leu Thr Ala Arg Arg Glu Asp Ser Gly Leu Tyr Trp Cys Glu Ala Ala
   260 265 270
   Thr Glu Asp Gly Asn Val Leu Lys Arg Ser Pro Glu Leu Glu Leu Gln
   275 280 285
   Val Leu Gly Leu Gln Leu Pro Thr Pro Val Val Trp Phe His Val Leu
       290                 295                 300
   Gly Tyr Leu Ala Val Gly Ile Met Phe Leu Val Asn Thr Val Leu Trp
   305                 310                 315                 320
   Val Val Thr Ile Arg Lys Glu Leu Lys Arg Lys Lys Lys Trp Asp Leu
                   325                 330                 335
   Glu Ile Ser Leu Asp Ser Gly His Glu Lys Lys Val Thr Ser Ser Leu
               340                 345                 350
   Gln Glu Asp Arg His Glu Glu Glu Glu Leu Lys Cys Gln Glu Gln Lys
           355                 360                 365
   Gly Glu Gln Leu Gln Glu Gly Val His Arg
       370                 375
<210> 32
   <211> 376
   <212> PRT
   <213> Homo Sapiens
<400> 32
   Met Leu Leu Trp Ala Ser Leu Leu Ala Phe Ala Pro Val Cys Gly Gln
   1               5                   10                  15
   Ser Ala Ala Ala His Lys Pro Val Ile Ser Val His Pro Pro Trp Thr
               20                  25                  30
   Thr Phe Phe Lys Gly Glu Arg Val Thr Leu Thr Cys Asn Gly Phe Gln
           35                  40                  45
   Phe Tyr Ala Thr Glu Lys Thr Thr Trp Tyr His Arg His Tyr Trp Gly
       50                  55                  60
   Glu Lys Leu Thr Leu Thr Pro Gly Asn Thr Leu Glu Val Arg Ala Ser
   65                  70                  75                  80
   Gly Leu Tyr Arg Cys Gln Ala Arg Gly Ser Pro Arg Ser Asn Pro Val
                   85                  90                  95
   Arg Leu Leu Phe Ser Ser Asp Ser Leu Ile Leu Gln Ala Pro Tyr Ser
               100                 105                 110
   Val Phe Glu Gly Asp Thr Leu Val Leu Arg Cys His Arg Arg Arg Lys
           115                 120                 125
   Glu Lys Leu Thr Ala Val Lys Tyr Thr Trp Asn Gly Asn Ile Leu Ser
       130                 135                 140
   Ile Ser Asn Lys Ser Trp Asp Leu Leu Ile Pro Gln Ala Ser Ser Asn
   145                 150                 155                 160
   Asn Asn Gly Asn Tyr Arg Cys Ile Gly Tyr Gly Val Glu Asn Asp Val
                   165                 170                 175
   Phe Arg Ser Asn Gly Asp Glu Asn Asp Val Phe Arg Ser Asn Phe Lys
               180                 185                 190
   Ile Ile Lys Ile Gln Glu Leu Phe Pro His Pro Glu Leu Lys Ala Thr
           195                 200                 205
   Asp Ser Gln Pro Thr Glu Gly Asn Ser Val Asn Leu Ser Cys Glu Thr
       210                 215                 220
   Gln Leu Pro Pro Glu Arg Ser Asp Thr Pro Leu His Phe Asn Phe Phe
   225                 230                 235                 240
   Arg Asp Gly Glu Val Ile Leu Ser Asp Trp Ser Thr Tyr Pro Glu Leu
                   245                 250                 255
   Gln Leu Pro Thr Val Trp Arg Glu Asn Ser Gly Ser Tyr Trp Cys Gly
               260                 265                 270
   Ala Glu Thr Val Arg Gly Asn Ile His Lys His Ser Pro Ser Leu Gln
           275                 280                 285
   Ile His Val Gln Arg Ile Pro Val Ser Gly Val Leu Leu Glu Thr Gln
       290                 295                 300
   Pro Ser Gly Gly Gln Ala Val Glu Gln Glu Met Leu Val Leu Val Cys
   305                 310                 315                 320
   Ser Val Ala Glu Gly Thr Gly Asp Thr Thr Phe Ser Trp His Arg Glu
                   325                 330                 335
   Asp Met Gln Glu Ser Leu Gly Arg Lys Thr Gln Arg Ser Leu Arg Ala
   340 345 350
   Glu Leu Glu Leu Pro Ala Ile Arg Gln Ser His Ala Gly Gly Tyr Tyr
   355 360 365
   Cys Thr Ala Asp Asn Ser Tyr Gly
   370 375
<210> 33
   <211> 373
   <212> PRT
   <213> Homo Sapiens
<400> 33
   Met Leu Leu Trp Val Ile Leu Leu Val Leu Ala Pro Val Ser Gly Gln
   1 5 10 15
   Phe Ala Arg Thr Pro Arg Pro Ile Ile Phe Leu Gln Pro Pro Trp Thr
   20 25 30
   Thr Val Phe Gln Gly Glu Arg Val Thr Leu Thr Cys Lys Gly Phe Arg
   35 40 45
   Phe Tyr Ser Pro Gln Arg Thr Arg Trp Tyr His Arg Tyr Leu Gly Lys
   50 55 60
   Glu Ile Leu Arg Glu Thr Pro Asp Asn Ile Leu Glu Val Gln Glu Ser
   65 70 75 80
   Gly Glu Tyr Arg Cys Gln Ala Gln Gly Ser Pro Leu Ser Ser Pro Val
   85 90 95
   His Leu Asp Phe Ser Ser Ala Ser Leu Ile Leu Gln Ala Pro Leu Ser
   100 105 110
   Val Phe Glu Gly Asp Ser Val Val Leu Arg Cys Arg Ala Lys Ala Glu
   115 120 125
   Val Thr Leu Asn Asn Thr Ile Tyr Lys Asn Asp Asn Val Leu Ala Phe
   130 135 140
   Leu Asn Lys Arg Thr Asp Phe His Ile Pro His Ala Cys Leu Lys Asp
   145 150 155 160
   Asn Gly Ala Tyr Arg Cys Thr Gly Tyr Lys Glu Ser Cys Cys Pro Val
   165 170 175
   Ser Ser Asn Lys Glu Ser Cys Cys Pro Val Ser Ser Asn Thr Val Lys
   180 185 190
   Ile Gln Val Gln Glu Pro Phe Thr Arg Pro Val Leu Arg Ala Ser Ser
   195 200 205
   Phe Gln Pro Thr Ser Gly Asn Pro Val Thr Leu Thr Cys Glu Thr Gln
   210 215 220
   Leu Ser Leu Glu Arg Ser Asp Val Pro Leu Arg Phe Arg Phe Phe Arg
   225 230 235 240
   Asp Asp Gln Thr Leu Gly Leu Gly Trp Ser Leu Ser Pro Asn Phe Gln
   245 250 255
   Ile Thr Ala Met Trp Ser Lys Asp Ser Gly Phe Tyr Trp Cys Lys Ala
   260 265 270
   Ala Thr Met Pro His Ser Val Ile Ser Asp Ser Pro Arg Ser Trp Ile
   275 280 285
   Gln Val Gln Ile Pro Ala Ser His Pro Val Leu Thr Leu Ser Pro Glu
   290 295 300
   Lys Ala Leu Asn Phe Glu Gly Thr Lys Val Thr Leu His Cys Glu Thr
   305 310 315 320
   Gln Glu Asp Ser Leu Arg Thr Leu Tyr Arg Phe Tyr His Glu Gly Val
   325 330 335
   Pro Leu Arg His Lys Ser Val Arg Cys Glu Arg Gly Ala Ser Ile Ser
   340 345 350
   Phe Ser Leu Thr Thr Glu Asn Ser Gly Asn Tyr Tyr Cys Thr Ala Asp
   355 360 365
   Asn Gly Leu Gly Ala
   370
<210> 34
   <211> 26
   <212> PRT
   <213> Homo Sapiens
<220>
   <221> MISC_FEATURE
   <222> (2)..(8)
   <223> Xaa = Any amino acid
<220>
   <221> MISC-FEATURE
   <222> (26)..(26)
   <223> Xaa = I or L
<220>
   <221> MISC_FEATURE
   <222> (15)..(15)
   <223> Xaa = I or L
<220>
   <221> MISC_FEATURE
   <222> (9)..(9)
   <223> Xaa = D or E
<220>
<221> MISC_FEATURE
   <222> (1)..(1)
   <223> Xaa = D or E
<220>
   <221> MISC_FEATURE
   <222> (10)..(11)
   <223> Xaa = Any amino acid
<220>
   <221> MISC_FEATURE
   <222> (13)..(14)
   <223> Xaa = Any amino acid
<220>
   <221> MISC_FEATURE
   <222> (16)..(22)
   <223> Xaa = Any amino acid
<220>
   <221> MISC_FEATURE
   <222> (24)..(25)
   <223> Xaa = Any amino acid
<900> 34
<210> 35
   <211> 63
   <212> PRT
   <213> Homo Sapiens
<400> 35
   Glu Ser Ser His Ser Ile Cys Pro Ala Gln Val Glu Leu Gln Ser Leu
   1               5                   10                  15
   Tyr Val Asp Val His Pro Lys Lys Gly Asp Leu Val Tyr Ser Glu Ile
               20                  25                  30
   Gln Thr Thr Thr Leu Gly Glu Glu Glu Glu Glu Ala Asn Thr Ser Arg
           35                  40                  45
   Thr Leu Leu Glu Asp Lys Asp Val Ser Val Val Tyr Ser Glu Val
       50                  55                  60
<210> 36
   <211> 39
   <212> PRT
   <213> Homo Sapiens
<400> 36
   Asp Asn Lys Glu Pro Leu Asn Ser Asp Val Gln Tyr Thr Glu Val Gln
   1               5                   10                  15
   Val Ser Ser Ala Glu Trp Ser His Lys Asp Leu Gly Lys Lys Asp Thr
               20                  25                  30
   Glu Thr Val Tyr Ser Glu Val
           35
<210> 38
   <211> 65
   <212> PRT
   <213> Homo Sapiens
<220>
   <221> MISC_FEATURE
   <222> (38)..(58)
   <223> Xaa = Any amino acid
<900> 38
<210> 39
   <211> 6
   <212> PRT
   <213> Artificial Sequence
<220>
   <223> Immune-receptor Tyrosine-based Inhibition Motif
   <220>
   <221> MISC_FEATURE
   <222> (1)..(1)
   <223> Xaa = S, V, L or I
<220>
   <221> MISC_FEATURE
   <222> (2)..(2)
   <223> Xaa = any amino acid
<220>
   <221> MISC_FEATURE
   <222> (4)..(5)
   <223> Xaa = any amino acid
<220>
   <221> MISC_FEATURE
   <222> (6)..(6)
   <223> Xaa = L or V
<400> 39
   Xaa Xaa Tyr Xaa Xaa Xaa
<210> 40
   <211> 5321
   <212> DNA
   <213> HOMO SAPIENS
<400> 40
   gtgcagtgtc ctgactgtaa gatcaagtcc aaacctgttt tggaattgag gaaacttctc     60
   ttttgatctc agcccttggt ggtccaggtc ttcatgctgc tgtgggtgat attactggtc    120
   ctggctcctg tcagtggaca gtttgcaagg acacccaggc ccattatttt cctccagcct    180
   ccatggacca cagtcttcca aggagagaga gtgaccctca cttgcaaggg atttcgcttc    240
   tactcaccac agaaaacaaa atggtaccat cggtaccttg ggaaagaaat actaagagaa    300
   accccagaca atatccttga ggttcaggaa tctggagagt acagatgcca ggcccagggc    360
   tcccctctca gtagccctgt gcacttggat ttttcttcag cttcgctgat cctgcaagct    420
   ccactttctg tgtttgaagg agactctgtg gttctgaggt gccgggcaaa ggcggaagta    480
   acactgaata atactattta caagaatgat aatgtcctgg cattccttaa taaaagaact    540
   gacttccata ttcctcatgc atgtctcaag gacaatggtg catatcgctg tactggatat    600
   aaggaaagtt gttgccctgt ttcttccaat acagtcaaaa tccaagtcca agagccattt    660
   acacgtccag tgctgagagc cagctccttc cagcccatca gcgggaaccc agtgaccctg    720
   acctgtgaga cccagctctc tctagagagg tcagatgtcc cgctccggtt ccgcttcttc    780
   agagatgacc agaccctggg attaggctgg agtctctccc cgaatttcca gattactgcc    840
   atgtggagta aagattcagg gttctactgg tgtaaggcag caacaatgcc tcacagcgtc    900
   atatctgaca gcccgagatc ctggatacag gtgcagatcc ctgcatctca tcctgtcctc    960
   actctcagcc ctgaaaaggc tctgaatttt gagggaacca aggtgacact tcactgtgaa   1020
   acccaggaag attctctgcg cactttgtac aggttttatc atgagggtgt ccccctgagg  1080
   cacaagtcag tccgctgtga aaggggagca tccatcagct tctcactgac tacagagaat   1140
   tcagggaact actactgcac agctgacaat ggccttggcg ccaagcccag taaggctgtg   1200
   agcctctcag tcactgttcc cgtgtctcat cctgtcctca acctcagctc tcctgaggac   1260
   ctgatttttg agggagccaa ggtgacactt cactgtgaag cccagagagg ttcactcccc   1320
   atcctgtacc agtttcatca tgaggatgct gccctggagc gtaggtcggc caactctgca   1380
   ggaggagtgg ccatcagctt ctctctgact gcagagcatt cagggaacta ctactgcaca   1440
   gctgacaatg gctttggccc ccagcgcagt aaggcggtga gcctctccat cactgtccct 1500
   gtgtctcatc ctgtcctcac cctcagctct gctgaggccc tgacttttga aggagccact 1560
   gtgacacttc actgtgaagt ccagagaggt tccccacaaa tcctatacca gttttatcat 1620
   gaggacatgc ccctgtggag cagctcaaca ccctctgtgg gaagagtgtc cttcagcttc 1680
   tctctgactg aaggacattc agggaattac tactgcacag ctgacaatgg ctttggtccc 1740
   cagcgcagtg aagtggtgag cctttttgtc actgttccag tgtctcgccc catcctcacc 1800
   ctcagggttc ccagggccca ggctgtggtg ggggacctgc tggagcttca ctgtgaggcc 1860
   ccgagaggct ctcccccaat cctgtactgg ttttatcatg aggatgtcac cctggggagc 1920
   agctcagccc cctctggagg agaagcttct ttcaacctct ctctgactgc agaacattct 1980
   ggaaactact catgtgaggc caacaatggc ctagtggccc agcacagtga cacaatatca 2040
   ctcagtgtta tagttccagt atctcgtccc atcctcacct tcagggctcc cagggcccag 2100
   gctgtggtgg gggacctgct ggagcttcac tgtgaggccc tgagaggctc ctccccaatc 2160
   ctgtactggt tttatcatga agatgtcacc ctgggtaaga tctcagcccc ctctggagga 2220
   ggggcctcct tcaacctctc tctgactaca gaacattctg gaatctactc ctgtgaggca 2280
   gacaatggtc tggaggccca gcgcagtgag atggtgacac tgaaagttgc agttccggtg 2340
   tctcgcccgg tcctcaccct cagggctccc gggacccatg ctgcggtggg ggacctgctg 2400
   gagcttcact gtgaggccct gagaggctct cccctgatcc tgtaccggtt ttttcatgag 2460
   gatgtcaccc taggaaatag gtcgtccccc tctggaggag cgtccttaaa cctctctctg 2520
   actgcagagc actctggaaa ctactcctgt gaggccgaca atggcctcgg ggcccagcgc 2580
   agtgagacag tgacacttta tatcacaggg ctgaccgcga acagaagtgg cccttttgcc 2640
   acaggagtcg ccgggggcct gctcagcata gcaggccttg ctgcgggggc actgctgctc 2700
   tactgctggc tctcgagaaa agcagggaga aagcctgcct ctgaccccgc caggagccct 2760
   tcagactcgg actcccaaga gcccacctat cacaatgtac cagcctggga agagctgcaa 2820
   ccagtgtaca ctaatgcaaa tcctagagga gaaaatgtgg tttactcaga agtacggatc 2880
   atccaagaga aaaagaaaca tgcagtggcc tctgacccca ggcatctcag gaacaagggt 2940
   tcccctatca tctactctga agttaaggtg gcgtcaaccc cggtttccgg atccctgttc 3000
   ttggcttcct cagctcctca cagatgagtc cacacgtctc tccaactgct gtttcagcct 3060
   ctgcacccca aagttcccct tgggggagaa gcagcattga agtgggaaga tttaggctgc 3120
   cccagaccat atctactggc ctttgtttca catgtcctca ttctcagtct gaccagaatg 3180
   cagggccctg ctggactgtc acctgtttcc cagttaaagc cctgactggc aggtttttta 3240
   atccagtggc aaggtgctcc cactccaggg cccagcacat ctcctggatt ccttagtggg 3300
   cttcagctgt ggttgctgtt ctgagtactg ctctcatcac acccccacag agggggtctt 3360
   accacacaaa gggagagtgg gccttcagga gatgccgggc tggcctaaca gctcaggtgc 3420
   tcctaaactc cgacacagag ttcctgcttt gggtggatgc atttctcaat tgtcatcagc 3480
   ctggtggggc tactgcagtg tgctgccaaa tgggacagca cacagcctgt gcacatggga 3540
   catgtgatgg gtctccccac gggggctgca tttcacactc ctccacctgt ctcaaactct 3600
   aaggtcggca cttgacacca aggtaacttc tctcctgctc atgtgtcagt gtctacctgc 3660
   ccaagtaagt ggctttcata caccaagtcc cgaagttctt cccatcctaa cagaagtaac 3720
   ccagcaagtc aaggccagga ggaccagggg tgcagacaga acacatactg gaacacagga 3780
   ggtgctcaat tactatttga ctgactgact gaatgaatga atgaatgagg aagaaaactg 3840
   tgggtaatca aactggcata aaatccagtg cactccctag gaaatccggg aggtattctg 3900
   gcttcctaag aaacaacgga agagaaggag cttggatgaa gaaactgttc agcaagaaga 3960
   agggcttctt cacactttta tgtgcttgtg gatcacctga ggatctgtga aaatacagat 4020
   actgattcag tgggtctgtg tagagcctga gactgccatt ctaacatgtt cccaggggat 4080
   gctgatgctg ctggccctgg gactgcactg catgcatgtg aagccctata ggtctcagca 4140
   gaggcccatg gagagggaat gtgtggctct ggctgcccag ggcccaactc ggttcacacg 4200
   gatcgtgctg ctccctggcc agcctttggc cacagcacca ccagctgctg ttgctgagag 4260
   agcttcttct ctgtgacatg ttggctttca tcagccaccc tgggaagcgg aaagtagctg 4320
   ccactatctt tgtttcccca cctcaggcct cacactttcc catgaaaagg gtgaatgtat 4380
   ataacctgag ccctctccat tcagagttgt tctcccatct ctgagcaatg ggatgttctg 4440
   ttccgctttt atgatatcca tcacatctta tcttgatctt tgctcccagt ggattgtaca 4500
   gtgatgactt ttaagcccca cggccctgaa ataaaatcct tccaagggca ttggaagctc 4560
   actccacctg aaccatggct tttcatgctt ccaagtgtca gggccttgcc cagatagaca 4620
   gggctgactc tgctgcccca acctttcaag gaggaaacca gacacctgag acaggagcct 4680
   gtatgcagcc cagtgcagcc ttgcagagga caaggctgga ggcatttgtc atcactacag 4740
   atatgcaact aaaatagacg tggagcaaga gaaatgcatt cccaccgagg ccgctttttt 4800
   aggcctagtt gaaagtcaag aaggacagca gcaagcatag gctcaggatt aaagaaaaaa 4860
   atctgctcac agtctgttct ggaggtcaca tcaccaacaa agctcacgcc ctatgcagtt 4920
   ctgagaaggt ggaggcacca ggctcaaaag aggaaattta gaatttctca ttgggagagt 4980
   aaggtacccc catcccagaa tgataactgc acagtggcag aacaaactcc accctaatgt 5040
   gggtggaccc catccagtct gttgaaggcc tgaatgtaac aaaagggctt attcttcctc 5100
   aagtaagggg gaactcctgc tttgggctgg gacataagtt tttctgcttt cagacgcaaa   5160
   ctgaaaaatg gctcttcttg ggtcttgagc ttgctggcat atggactgaa agaaactatg   5220
   ctattggatc tcctggatct ccagcttgct gactgcagat cttgagatat gtcagcctct   5280
   acagtcacaa gagctaattc attctaataa accaatcttt c                       5321
<210> 41
   <211> 977
   <212> PRT
   <213> HOMO SAPIENS
<400> 41
   Met Leu Leu Trp Val Ile Leu Leu Val Leu Ala Pro Val Ser Gly Gln
   1               5                   10                  15
   Phe Ala Arg Thr Pro Arg Pro Ile Ile Phe Leu Gln Pro Pro Trp Thr
               20                  25                  30
   Thr Val Phe Gln Gly Glu Arg Val Thr Leu Thr Cys Lys Gly Phe Arg
           35                  40                  45
   Phe Tyr Ser Pro Gln Lys Thr Lys Trp Tyr His Arg Tyr Leu Gly Lys
       50                  55                  60
   Glu Ile Leu Arg Glu Thr Pro Asp Asn Ile Leu Glu Val Gln Glu Ser
   65                  70                  75                  80
   Gly Glu Tyr Arg Cys Gln Ala Gln Gly Ser Pro Leu Ser Ser Pro Val
                   85                  90                  95
   His Leu Asp Phe Ser Ser Ala Ser Leu Ile Leu Gln Ala Pro Leu Ser
               100                 105                 110
   Val Phe Glu Gly Asp Ser Val Val Leu Arg Cys Arg Ala Lys Ala Glu
           115                 120                 125
   Val Thr Leu Asn Asn Thr Ile Tyr Lys Asn Asp Asn Val Leu Ala Phe
       130                 135                 140
   Leu Asn Lys Arg Thr Asp Phe His Ile Pro His Ala Cys Leu Lys Asp
   145                 150                 155                 160
   Asn Gly Ala Tyr Arg Cys Thr Gly Tyr Lys Glu Ser Cys Cys Pro Val
                   165                 170                 175
   Ser Ser Asn Thr Val Lys Ile Gln Val Gln Glu Pro Phe Thr Arg Pro
               180                 185                 190
   Val Leu Arg Ala Ser Ser Phe Gln Pro Ile Ser Gly Asn Pro Val Thr
           195                 200                 205
   Leu Thr Cys Glu Thr Gln Leu Ser Leu Glu Arg Ser Asp Val Pro Leu
       210                 215                 220
   Arg Phe Arg Phe Phe Arg Asp Asp Gln Thr Leu Gly Leu Gly Trp Ser
   225                 230                 235                 240
   Leu Ser Pro Asn Phe Gln Ile Thr Ala Met Trp Ser Lys Asp Ser Gly
                   245                 250                 255
   Phe Tyr Trp Cys Lys Ala Ala Thr Met Pro His Ser Val Ile Ser Asp
               260                 265                 270
   Ser Pro Arg Ser Trp Ile Gln Val Gln Ile Pro Ala Ser His Pro Val
           275                 280                 285
   Leu Thr Leu Ser Pro Glu Lys Ala Leu Asn Phe Glu Gly Thr Lys Val
       290                 295                 300
   Thr Leu His Cys Glu Thr Gln Glu Asp Ser Leu Arg Thr Leu Tyr Arg
   305                 310                 315                 320
   Phe Tyr His Glu Gly Val Pro Leu Arg His Lys Ser Val Arg Cys Glu
                   325                 330                 335
   Arg Gly Ala Ser Ile Ser Phe Ser Leu Thr Thr Glu Asn Ser Gly Asn
               340                 345                 350
   Tyr Tyr Cys Thr Ala Asp Asn Gly Leu Gly Ala Lys Pro Ser Lys Ala
           355                 360                 365
   Val Ser Leu Ser Val Thr Val Pro Val Ser His Pro Val Leu Asn Leu
       370                 375                 380
   Ser Ser Pro Glu Asp Leu Ile Phe Glu Gly Ala Lys Val Thr Leu His
   385                 390                 395                 400
   Cys Glu Ala Gln Arg Gly Ser Leu Pro Ile Leu Tyr Gln Phe His His
   405 410 415
   Glu Asp Ala Ala Leu Glu Arg Arg Ser Ala Asn Ser Ala Gly Gly Val
   420 425 430
   Ala Ile Ser Phe Ser Leu Thr Ala Glu His Ser Gly Asn Tyr Tyr Cys
   435 440 445
   Thr Ala Asp Asn Gly Phe Gly Pro Gln Arg Ser Lys Ala Val Ser Leu
   450 455 460
   Ser Ile Thr Val Pro Val Ser His Pro Val Leu Thr Leu Ser Ser Ala
   465 470 475 480
   Glu Ala Leu Thr Phe Glu Gly Ala Thr Val Thr Leu His Cys Glu Val
   485 490 495
   Gln Arg Gly Ser Pro Gln Ile Leu Tyr Gln Phe Tyr His Glu Asp Met
   500 505 510
   Pro Leu Trp Ser Ser Ser Thr Pro Ser Val Gly Arg Val Ser Phe Ser
   515 520 525
   Phe Ser Leu Thr Glu Gly His Ser Gly Asn Tyr Tyr Cys Thr Ala Asp
   530 535 540
   Asn Gly Phe Gly Pro Gln Arg Ser Glu Val Val Ser Leu Phe Val Thr
   545 550 555 560
   Val Pro Val Ser Arg Pro Ile Leu Thr Leu Arg Val Pro Arg Ala Gln
   565 570 575
   Ala Val Val Gly Asp Leu Leu Glu Leu His Cys Glu Ala Pro Arg Gly
   580 585 590
   Ser Pro Pro Ile Leu Tyr Trp Phe Tyr His Glu Asp Val Thr Leu Gly
   595 600 605
   Ser Ser Ser Ala Pro Ser Gly Gly Glu Ala Ser Phe Asn Leu Ser Leu
   610 615 620
   Thr Ala Glu His Ser Gly Asn Tyr Ser Cys Glu Ala Asn Asn Gly Leu
   625 630 635 640
   Val Ala Gln His Ser Asp Thr Ile Ser Leu Ser Val Ile Val Pro Val
   645 650 655
   Ser Arg Pro Ile Leu Thr Phe Arg Ala Pro Arg Ala Gln Ala Val Val
   660 665 670
   Gly Asp Leu Leu Glu Leu His Cys Glu Ala Leu Arg Gly Ser Ser Pro
   675 680 685
   Ile Leu Tyr Trp Phe Tyr His Glu Asp Val Thr Leu Gly Lys Ile Ser
   690 695 700
   Ala Pro Ser Gly Gly Gly Ala Ser Phe Asn Leu Ser Leu Thr Thr Glu
   705 710 715 720
   His Ser Gly Ile Tyr Ser Cys Glu Ala Asp Asn Gly Leu Glu Ala Gln
   725 730 735
   Arg Ser Glu Met Val Thr Leu Lys Val Ala Val Pro Val Ser Arg Pro
   740 745 750
   Val Leu Thr Leu Arg Ala Pro Gly Thr His Ala Ala Val Gly Asp Leu
   755 760 765
   Leu Glu Leu His Cys Glu Ala Leu Arg Gly Ser Pro Leu Ile Leu Tyr
   770 775 780
   Arg Phe Phe His Glu Asp Val Thr Leu Gly Asn Arg Ser Ser Pro Ser
   785 790 795 800
   Gly Gly Ala Ser Leu Asn Leu Ser Leu Thr Ala Glu His Ser Gly Asn
   805 810 815
   Tyr Ser Cys Glu Ala Asp Asn Gly Leu Gly Ala Gln Arg Ser Glu Thr
   820 825 830
   Val Thr Leu Tyr Ile Thr Gly Leu Thr Ala Asn Arg Ser Gly Pro Phe
   835 840 845
   Ala Thr Gly Val Ala Gly Gly Leu Leu Ser Ile Ala Gly Leu Ala Ala
   850 855 860
   Gly Ala Leu Leu Leu Tyr Cys Trp Leu Ser Arg Lys Ala Gly Arg Lys
   865 870 875 880
   Pro Ala Ser Asp Pro Ala Arg Ser Pro Ser Asp Ser Asp Ser Gln Glu
                   885                 890                 895
   Pro Thr Tyr His Asn Val Pro Ala Trp Glu Glu Leu Gln Pro Val Tyr
               900                 905                 910
   Thr Asn Ala Asn Pro Arg Gly Glu Asn Val Val Tyr Ser Glu Val Arg
           915                 920                 925
   Ile Ile Gln Glu Lys Lys Lys His Ala Val Ala Ser Asp Pro Arg His
       930                 935                 940
   Leu Arg Asn Lys Gly Ser Pro Ile Ile Tyr Ser Glu Val Lys Val Ala
   945                 950                 955                 960
   Ser Thr Pro Val Ser Gly Ser Leu Phe Leu Ala Ser Ser Ala Pro His
                   965                 970                 975
   Arg
<210> 42
   <211> 16
   <212> DNA.
   <213> HOMO SAPIENS
<400> 42
   ggcacctccc cttaac 16
<210> 43
   <211> 2797
   <212> DNA
   <213> HOMO SAPIENS
<400> 43
   gtgcagtgtc ctgactgtaa gatcaagtcc aaacctgttt tggaattgag gaaacttctc      60
   ttttgatctc agcccttggt ggtccaggtc ttcatgctgc tgtgggtgat attactggtc     120
   ctggctcctg tcagtggaca gtttgcaagg acacccaggc ccattatttt cctccagcct    180
   ccatggacca cagtcttcca aggagagaga gtgaccctca cttgcaaggg atttcgcttc    240
   tactcaccac agaaaacaaa atggtaccat cggtaccttg ggaaagaaat actaagagaa    300
   accccagaca atatccttga ggttcaggaa tctggagagt acagatgcca ggcccagggc    360
   tcccctctca gtagccctgt gcacttggat ttttcttcag cttcgctgat cctgcaagct     420
   ccactttctg tgtttgaagg agactctgtg gttctgaggt gccgggcaaa ggcggaagta    480
   acactgaata atactattta caagaatgat aatgtcctgg cattccttaa taaaagaact    540
   gacttccata ttcctcatgc atgtctcaag gacaatggtg catatcgctg tactggatat    600
   aaggaaagtt gttgccctgt ttcttccaat acagtcaaaa tccaagtcca agagccattt    660
   acacgtccag tgctgagagc cagctccttc cagcccatca gcgggaaccc agtgaccctg    720
   acctgtgaga cccagctctc tctagagagg tcagatgtcc cgctccggtt ccgcttcttc    780
   agagatgacc agaccctggg attaggctgg agtctctccc cgaatttcca gattactgcc    840
   atgtggagta aagattcagg gttctactgg tgtaaggcag caacaatgcc tcacagcgtc    900
   atatctgaca gcccgagatc ctggatacag gtgcagatcc ctgcatctca tcctgtcctc    960
   actctcagcc ctgaaaaggc tctgaatttt gagggaacca aggtgacact tcactgtgaa   1020
   acccaggaag attctctgcg cactttgtac aggttttatc atgagggtgt ccccctgagg   1080
   cacaagtcag tccgctgtga aaggggagca tccatcagct tctcactgac tacagagaat   1140
   tcagggaact actactgcac agctgacaat ggccttggcg ccaagcccag taaggctgtg   1200
   agcctctcag tcactgttcc cgtgtctcat cctgtcctca acctcagctc tcctgaggac   1260
   ctgatttttg agggagccaa ggtgacactt cactgtgaag cccagagagg ttcactcccc   1320
   atcctgtacc agtttcatca tgaggatgct gccctggagc gtaggtcggc caactctgca   1380
   ggaggagtgg ccatcagctt ctctctgact gcagagcatt cagggaacta ctactgcaca   1440
   gctgacaatg gctttggccc ccagcgcagt aaggcggtga gcctctccat cactgtccct   1500
   gtgtctcatc ctgtcctcac cctcagctct gctgaggccc tgacttttga aggagccact   1560
   gtgacacttc actgtgaagt ccagagaggt tccccacaaa tcctatacca gttttatcat   1620
   gaggacatgc ccctgtggag cagctcaaca ccctctgtgg gaagagtgtc cttcagcttc   1680
   tctctgactg aaggacattc agggaattac tactgcacag ctgacaatgg ctttggtccc   1740
   cagcgcagtg aagtggtgag cctttttgtc actgttccag tgtctcgccc catcctcacc   1800
   ctcagggttc ccagggccca ggctgtggtg ggggacctgc tggagcttca ctgtgaggcc   1860
   ccgagaggct ctcccccaat cctgtactgg ttttatcatg aggatgtcac cctggggagc   1920
   agctcagccc cctctggagg agaagcttct ttcaacctct ctctgactgc agaacattct   1980
   ggaaactact catgtgaggc caacaatggc ctagtggccc agcacagtga cacaatatca   2040
   ctcagtgtta tagttccagt atctcgtccc atcctcacct tcagggctcc cagggcccag   2100
   gctgtggtgg gggacctgct ggagcttcac tgtgaggccc tgagaggctc ctccccaatc   2160
   ctgtactggt tttatcatga agatgtcacc ctgggtaaga tctcagcccc ctctggagga   2220
   ggggcctcct tcaacctctc tctgactaca gaacattctg gaatctactc ctgtgaggca   2280
   gacaatggtc tggaggccca gcgcagtgag atggtgacac tgaaagttgc aggtgagtgg   2340
   gccctgccca ccagcagcac atctgagaac tgactgtgcc tgttctccct gcagctgaaa   2400
   atggagccac agagctcctc agggctgttt gcttgtgtgg catcccagca cacttcctgc   2460
   ctgcagaacc tccctgtgaa agtctcggat cctttgtggt atggttccag gaatctgatg   2520
   tttcccagca gtcttcttga agatgatcaa agcacctcac taaaaatgca aataagactt   2580
   ttttagaaca taaactatat tctgaactga aattattaca tgaaaatgaa accaaagaat   2640
   tctgagcata tgtttctctg ccgtagaaag gattaagctg tttcttgtcc ggattcttct   2700
   ctcattgact tctaagaagc ctctactctt gagtctcttt cattactggg gatgtaaatg   2760
   ttccttacat ttccacatta aaaatcctat gttaacg                            2797
<210> 44
   <211> 759
   <212> PRT
   <213> HOMO SAPIENS
<400> 44
   Met Leu Leu Trp Val Ile Leu Leu Val Leu Ala Pro Val Ser Gly Gln
   1               5                   10                  15
   Phe Ala Arg Thr Pro Arg Pro Ile Ile Phe Leu Gln Pro Pro Trp Thr
               20                  25                  30
   Thr Val Phe Gln Gly Glu Arg Val Thr Leu Thr Cys Lys Gly Phe Arg
           35                  40                  45
   Phe Tyr Ser Pro Gln Lys Thr Lys Trp Tyr His Arg Tyr Leu Gly Lys
       50                  55                  60
   Glu Ile Leu Arg Glu Thr Pro Asp Asn Ile Leu Glu Val Gln Glu Ser
   65                  70                  75                  80
   Gly Glu Tyr Arg Cys Gln Ala Gln Gly Ser Pro Leu Ser Ser Pro Val
                   85                  90                  95
   His Leu Asp Phe Ser Ser Ala Ser Leu Ile Leu Gln Ala Pro Leu Ser
               100                 105                 110
   Val Phe Glu Gly Asp Ser Val Val Leu Arg Cys Arg Ala Lys Ala Glu
           115                 120                 125
   Val Thr Leu Asn Asn Thr Ile Tyr Lys Asn Asp Asn Val Leu Ala Phe
       130                 135                 140
   Leu Asn Lys Arg Thr Asp Phe His Ile Pro His Ala Cys Leu Lys Asp
   145                 150                 155                 160
   Asn Gly Ala Tyr Arg Cys Thr Gly Tyr Lys Glu Ser Cys Cys Pro Val
                   165                 170                 175
   Ser Ser Asn Thr Val Lys Ile Gln Val Gln Glu Pro Phe Thr Arg Pro
               180                 185                 190
   Val Leu Arg Ala Ser Ser Phe Gln Pro Ile Ser Gly Asn Pro Val Thr
           195                 200                 205
   Leu Thr Cys Glu Thr Gln Leu Ser Leu Glu Arg Ser Asp Val Pro Leu
       210                 215                 220
   Arg Phe Arg Phe Phe Arg Asp Asp Gln Thr Leu Gly Leu Gly Trp Ser
   225                 230                 235                 240
   Leu Ser Pro Asn Phe Gln Ile Thr Ala Met Trp Ser Lys Asp Ser Gly
                   245                 250                 255
   Phe Tyr Trp Cys Lys Ala Ala Thr Met Pro His Ser Val Ile Ser Asp
               260                 265                 270
   Ser Pro Arg Ser Trp Ile Gln Val Gln Ile Pro Ala Ser His Pro Val
           275                 280                 285
   Leu Thr Leu Ser Pro Glu Lys Ala Leu Asn Phe Glu Gly Thr Lys Val
       290                 295                 300
   Thr Leu His Cys Glu Thr Gln Glu Asp Ser Leu Arg Thr Leu Tyr Arg
   305                 310                 315                 320
   Phe Tyr His Glu Gly Val Pro Leu Arg His Lys Ser Val Arg Cys Glu
   325 330 335
   Arg Gly Ala Ser Ile Ser Phe Ser Leu Thr Thr Glu Asn Ser Gly Asn
   340 345 350
   Tyr Tyr Cys Thr Ala Asp Asn Gly Leu Gly Ala Lys Pro Ser Lys Ala
   355 360 365
   Val Ser Leu Ser Val Thr Val Pro Val Ser His Pro Val Leu Asn Leu
   370 375 380
   Ser Ser Pro Glu Asp Leu Ile Phe Glu Gly Ala Lys Val Thr Leu His
   385 390 395 400
   Cys Glu Ala Gln Arg Gly Ser Leu Pro Ile Leu Tyr Gln Phe His His
   405 410 415
   Glu Asp Ala Ala Leu Glu Arg Arg Ser Ala Asn Ser Ala Gly Gly Val
   420 425 430
   Ala Ile Ser Phe Ser Leu Thr Ala Glu His Ser Gly Asn Tyr Tyr Cys
   435 440 445
   Thr Ala Asp Asn Gly Phe Gly Pro Gln Arg Ser Lys Ala Val Ser Leu
   450 455 460
   Ser Ile Thr Val Pro Val Ser His Pro Val Leu Thr Leu Ser Ser Ala
   465 470 475 480
   Glu Ala Leu Thr Phe Glu Gly Ala Thr Val Thr Leu His Cys Glu Val
   485 490 495
   Gln Arg Gly Ser Pro Gln Ile Leu Tyr Gln Phe Tyr His Glu Asp Met
   500 505 510
   Pro Leu Trp Ser Ser Ser Thr Pro Ser Val Gly Arg Val Ser Phe Ser
   515 520 525
   Phe Ser Leu Thr Glu Gly His Ser Gly Asn Tyr Tyr Cys Thr Ala Asp
   530 535 540
   Asn Gly Phe Gly Pro Gln Arg Ser Glu Val Val Ser Leu Phe Val Thr
   545 550 555 560
   Val Pro Val Ser Arg Pro Ile Leu Thr Leu Arg Val Pro Arg Ala Gln
                   565                 570                 575
   Ala Val Val Gly Asp Leu Leu Glu Leu His Cys Glu Ala Pro Arg Gly
               580                 585                 590
   Ser Pro Pro Ile Leu Tyr Trp Phe Tyr His Glu Asp Val Thr Leu Gly
           595                 600                 605
   Ser Ser Ser Ala Pro Ser Gly Gly Glu Ala Ser Phe Asn Leu Ser Leu
       610                 615                 620
   Thr Ala Glu His Ser Gly Asn Tyr Ser Cys Glu Ala Asn Asn Gly Leu
   625                 630                 635                 640
   Val Ala Gln His Ser Asp Thr Ile Ser Leu Ser Val Ile Val Pro Val
                   645                 650                 655
   Ser Arg Pro Ile Leu Thr Phe Arg Ala Pro Arg Ala Gln Ala Val Val
               660                 665                 670
   Gly Asp Leu Leu Glu Leu His Cys Glu Ala Leu Arg Gly Ser Ser Pro
           675                 680                 685
   Ile Leu Tyr Trp Phe Tyr His Glu Asp Val Thr Leu Gly Lys Ile Ser
       690                 695                 700
   Ala Pro Ser Gly Gly Gly Ala Ser Phe Asn Leu Ser Leu Thr Thr Glu
   705                 710                 715                 720
   His Ser Gly Ile Tyr Ser Cys Glu Ala Asp Asn Gly Leu Glu Ala Gln
                   725                 730                 735
   Arg Ser Glu Met Val Thr Leu Lys Val Ala Gly Glu Trp Ala Leu Pro
               740                 745                 750
   Thr Ser Ser Thr Ser Glu Asn
           755

## Description

The invention disclosed was herein made in the course of work under NCI Grant No. CA 44029 from the National Cancer Institute. Accordingly, the U.S. Government has certain rights in this invention.

Throughout this application, various references are referred to in parentheses.

Full bibliographic citation for these references may be found at the end of this application, preceding the claims.

### BACKGROUND OF THE INVENTION

Abnormalities of chromosome 1q21 are common in B cell malignancies, including B cell lymphoma and myeloma, but the genes targeted by these aberrations are largely unknown. By cloning the breakpoints of a t (1;14) (q21;q32) chromosomal translocation in a myeloma cell line, we have identified two novel genes, *IRTA1* and *IRTA2,* encoding cell surface receptors with homologies to the Fc and Inhibitory Receptor families. Both genes are normally expressed in mature B cells, but with different distributions in peripheral lymphoid organs: IRTA1 is expressed in marginal zone B cells, while IRTA2 is also expressed in germinal center centrocytes and in immunoblasts. As the result of the t (1;14) translocation, the IRTA1 signal peptide is fused to the Immunoglobulin Cα domain to produce a chimaeric IRTA1/Ca fusion protein. In Multiple Myeloma and Burkitt lymphoma cell lines with lq21 abnormalities, *IRTA2* expression is deregulated. Thus, *IRTA1* and *IRTA2* are novel immunoreceptor with a potentially important role in B cell development and lymphomagenesis.

B-cell Non-Hodgkin's Lymphoma (B-NHL) and Multiple Myeloma (MM) represent a heterogeneous group of malignancies derived from mature B cells with phenotypes corresponding to pre-Germinal Center (GC) (mantle cell), GC (follicular, diffuse large cell, Burkitt's), or post-GC B cells (MM) (for review, Gaidano and Dalla-Favera, 1997; Kuppers et al., 1999). Insights into the pathogenesis of these malignancies have been gained by the identification of recurrent clonal chromosomal abnormalities characteristic for specific disease subtypes. The common consequence of these translocations is the transcriptional deregulation of protooncogenes by their juxtaposition to heterologous transcriptional regulatory elements located in the partner chromosome (Gaidano and Dalla-Favera, 1997). These heterologous transcriptional regulatory elements can be derived from the Immunoglobulin (IG) locus or from other partner chromosomal loci. Examples include MYC in t (8;14) (q24;q32) in Burkitt's lymphoma (BL) (Dalla-Favera et al., 1982; Taub et al., 1982), the *CCND1* gene deregulated by the t(11;14) (q13;q32) in mantle cell lymphoma (MCL) (Rosenberg et al., 1991) and multiple myeloma (MM) (Ronchetti et al., 1999), *BCL2* involved in the t(14,18) (q32;q21) in follicular lymphoma (FL) (Bakhshi et al., 1985), *BCL6* in t(3;14) (q27;q32) in diffuse large B cell lymphoma (DLCL) (Ye et al., 1993), as well as *FGFR3* in t (4;14) (p16,q32) (Chesi et al. , 1997), *MAF* in t(14;16) (q32;q23) (Chesi et al., 1998) and *MUM1*/*IRF4* in t(6;14)(p25;q32) (Iida et al., 1997) in multiple myeloma (MM). The identification of these oncogenes has offered valuable insights into the pathogenesis and diagnosis of their corresponding malignancies.

Chromosomal abnormalities involving band lq21-q23 are among the most frequent genetic lesions in both B-NHL and MM. Among NHL subtypes, translocation breakpoints at lq21-q23, including translocations and duplications, have been reported, often as the single chromosomal abnormality, in 17-20% of follicular and diffuse large B-cell lymphoma (DLCL), in 39% of marginal-zone B cell lymphoma (Offit et al., 1991; Whang-Peng et all., 1995; Cigudosa et al., 1999) and in 27-38% of Burkitt lymphoma, where they represent the second most common cytogenetic abnormality after translocations involving the *MYC* protooncogene (Berger and Bernheim, 1985; Kornblau et al., 1991). Comparative genome hybridization (CGH) has also identified lq21-q23 as a recurring site for high-level amplification in 10% of DLCL cases (Rao et al., 1998). In MM, trisomy of the lq21-q32 region has been reported in 20-31% of cases (Sawyer et al., 1995), amplification of the lq12-qter region in 80% of cell lines and 40% of primary tumors (Avet-Loiseau et al., 1997), and nonrandom unbalanced whole-arm translocations of lq, associated with the multiduplication of the adjacent lq21-22 region, were found in 23% of patients with abnormal karyotypes (Sawyer et al., 1998).

The high frequency of involvement of lq21 structural rearrangements in B-cell malignancies suggests that this locus may harbor genes critical to the pathogenesis of these diseases. Cloning of a t(1;14) (q21;q32) in a pre-B cell acute lymphoblastic leukemia cell line previously identified a novel gene, *BCL9* deregulated in this single case (Willis et al., 1998), but not involved in other cases. A recent report characterized the t(1;22) (q22;q11) in a follicular lymphoma (FL) cell line and found that the *FCGR2B* locus, encoding the low affinity IgG Fc receptor FCGRIIB, was targeted in this cell line and in two additional FL cases (Callanan et al., 2000). Finally, the *MUC1* locus has been identified in proximity of the breakpoint of a t(1;14) (q21;q32) in NHL (Dyomin et al., 2000; Gilles et al., 2000; and *MUC1* locus rearrangements have been found in 6% of NHL with 1q21 abnormalities (Dyomin et al., 2000). These results highlight the heterogeneity of the lq21 breakpoints and the need to identify additional candidate oncogenes situated in this locus, since the large majority of these alterations remain unexplained.

Miller et al., Blood, vol. 96, no. 11, 2000, discloses the location of a 300kb DNA region of IRTA genes. WO-A1 00/17222, cited under Article 54(3) EPC, discusses human secreted proteins and isolated nucleic acids containing the regions of the genes encoding such proteins. The nucleotide sequence of "gene 11" shows a 2230 nucleotide overlap (with 99.3% identity) with the sequence encoding the IRTA4 protein of the present invention. Furthermore, EP-A2 0 330 191 discusses a method for cloning cDNAs from mammalian expression libraries based on transient expression in mammalian host cells.

The aim of this study was to further explore the architecture of lq21 chromosomal rearrangements in B cell malignancy. To that end, we have employed a molecular cloning approach of the t(1;14) (q21;q32) present in the myeloma cell line FR4. We have identified two novel genes that are differentially targeted by lq21 abnormalities. These genes code for five novel members of the immunoglobulin receptor family, *IRTA1*, *IRTA2, IRTA3, IRTA4 and IRTA5* (Immunoglobulin superfamily Receptor Translocation Associated genes 1, 2, 3, 4, and 5), which may be important for normal lymphocyte function and B cell malignancy.

### SUMMARY OF THE INVENTION

This invention provides an isolated nucleic acid molecule which encodes immunoglobulin receptor, Immunoglobulin superfamily Receptor Translocation Associated, IRTA4, protein.

Disclosed is a method of producing an IRTA polypeptide (protein) which comprises: (a) introducing a vector comprising an isolated nucleic acid which encodes an immunoglobulin receptor, Immunoglobulin superfamily Receptor Translocation Associated, IRTA, protein into a suitable host cell; and (b) culturing the resulting cell so as to.produce the polypeptide.

This invention provides an isolated nucleic acid molecule comprising at least 15 contiguous nucleotides capable of specifically hybridizing with a unique sequence included within the sequence of the isolated nucleic acid molecule encoding IRTA4 protein, or fragment(s) thereof, having the amino acid sequence set forth in Figures 18D-1-18D-2.

Disclosed is a method for detecting a B cell malignancy or a type of B cell malignancy in a sample from a subject wherein the B cell malignancy comprises a lq21 chromosomal rearrangement which comprises:
a) contacting the RNA sample obtained from the subject with a nucleic acid molecule of at least contiguous nucleotides capable of specifically hybridizing with a unique sequence included within the sequence of an isolated RNA encoding human IRTA4 protein under conditions permitting hybridization of the RNA of step (a) with the nucleic acid molecule capable of specifically hybridizing with a unique sequence included within the sequence of an isolated RNA encoding human IRTA protein, wherein the nucleic acid molecule is labeled with a detectable marker; and b) detecting any hybridization in step a), wherein detecion of hybridization indicates presence of B cell malignancy or a type of B cell malignancy in the sample.

Disclosed is an antisense oligonucleotide having a sequence capable of specifically hybridizing to an mRNA molecule encoding the human IRTA protein so as to prevent overexpression of the mRNA molecule.

This invention provides a purified IRTA4 protein comprising the amino acid sequence set forth in Figures 18D-1-18D-2 (SEQ ID NO: 7).

This invention provides an antibody/antibodies directed to an epitope of purified IRTA4 , or fragment(s) thereof, having the amino acid sequence set forth in Figures 18D-1-18D-2.

This invention provides an antibody directed to purified IRTA4 protein.

This invention provides a pharmaceutical composition comprising an amount of the antibody directed to IRTA4 protein effective to bind to cancer cells expressing human IRTA4 so as to prevent growth of the cancer cells and a pharmaceutically acceptable carrier.

Disclosed is a pharmaceutical composition comprising an amount of any of the oligonucleotides of nucleic acid molecules encoding the IRTA protein described herein effective to prevent overexpression of a human IRTA protein and a pharmaceutically acceptable carrier capable. This invention provides a method of diagnosing B cell malignancy which comprises a lq21 chromosomal rearrangement in a sample from a subject which comprises:
a) contacting the sample obtained from the subject with an antibody directed to purifiedIRTA4 protein capable of specifically binding with human IRTA4 protein on a cell surface of a cancer cell under conditions permitting binding of the antibody with human IRTA protein on the cell surface of the cancer cell, wherein the antibody is labeled with a detectable marker; and b) detecting any binding in step a), wherein detecion of binding indicates a diagnosis of B cell malignancy in the sample.

Disclosed is a method of detecting human IRTA protein in a sample which comprises: a) contacting the sample with any of any of the above-described anti-IRTA antibodies under conditions permitting the formation of a complex between the antibody and the IRTA in the sample; and b) detecting the complex formed in step (a), thereby detecting the presence of human IRTA in the sample.

This invention provides a use for treating a subject having a B cell cancer which comprises administering to the subject an amount of anti-IRTA antibody effective to bind to cancer cells expressing an IRTA protein so as to prevent growth of the cancer cells and a pharmaceutically acceptable carrier.

Disclosed is a use for treating a subject having a B cell cancer which comprises administering to the subject an amount of an antisense oligonucleotide having a sequence capable of specifically hybridizing to an mRNA molecule encoding a human ITRA protein so as to prevent overexpression of the human IRTA protein, so as to arrest cell growth or induce cell death of cancer cells expressing IRTA protein(s) and a pharmaceutically acceptable carrier.

Also disclosed is a pharmaceutical composition comprising either an effective amount of any of the oligonucleotides described herein and a pharmaceutically acceptable carrier.

The invention also provides a pharmaceutical composition comprising either an effective amount of an antibody directed against an epitope of IRTA4 protein described herein and a pharmaceutically acceptable carrier.

### BRIEF DESCRIPTION OF THE FIGURES

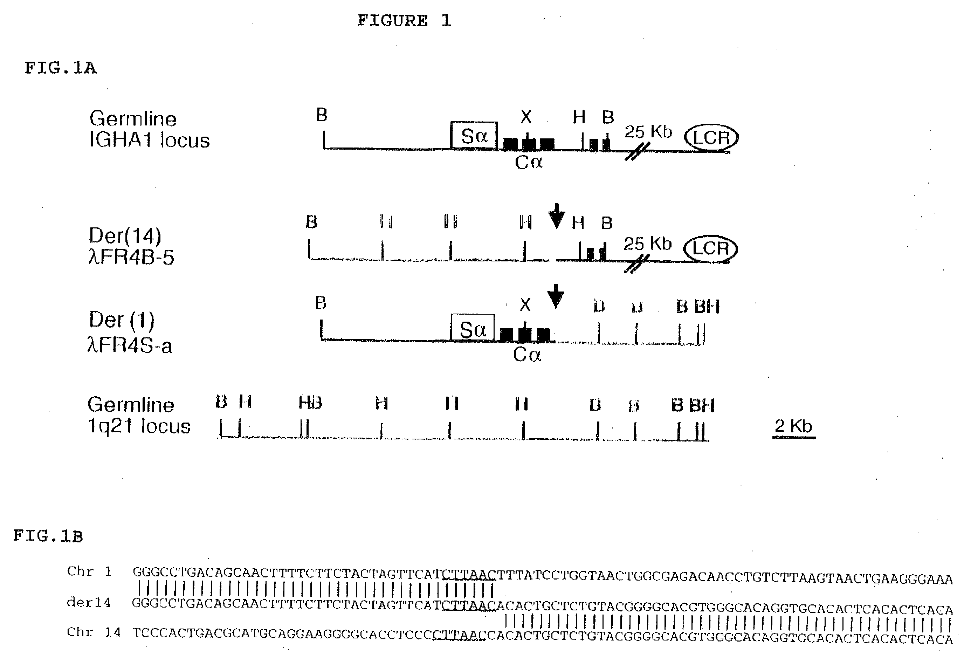

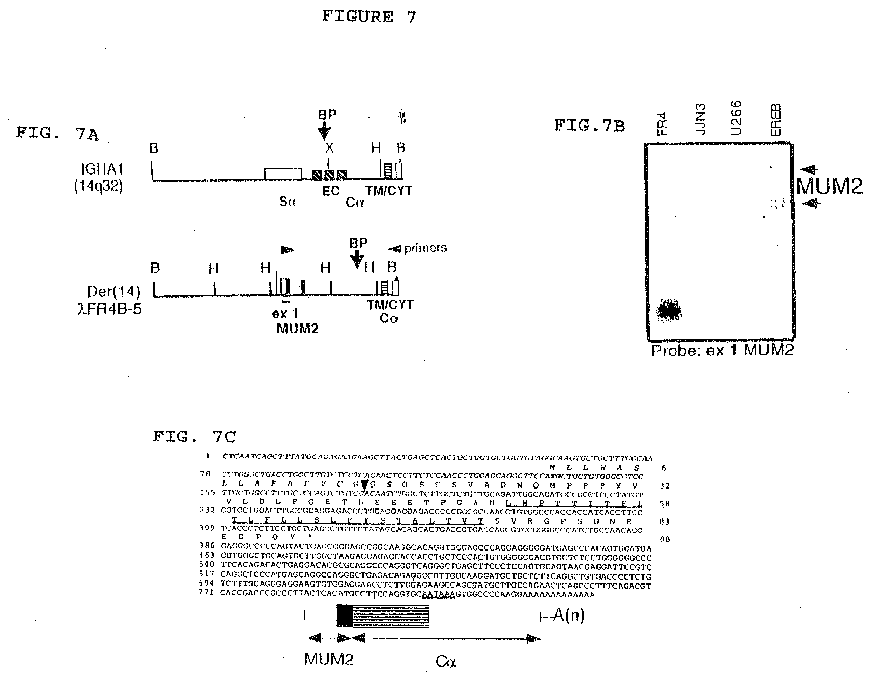

- **Figures 1A-1B.**: Molecular cloning of the translocation t(1;14) (q21;q32) in the FR4 multiple myeloma cell line. Fig. 1A) Schematic representation of the λFR4B-5 and λFR4S-a clones, representing der(14) and der(1) breakpoints, and of the germline IgH and lq21 loci. Fig. 1B) Nucleotide sequence of the breakpoint junction and its alignment to the corresponding germline regions of chromosome 14. Sα, IgA switch region; LCR: 3' IgH locus control region,; B, *BamHI;* H, *HindIII;* X, *XhoI.*

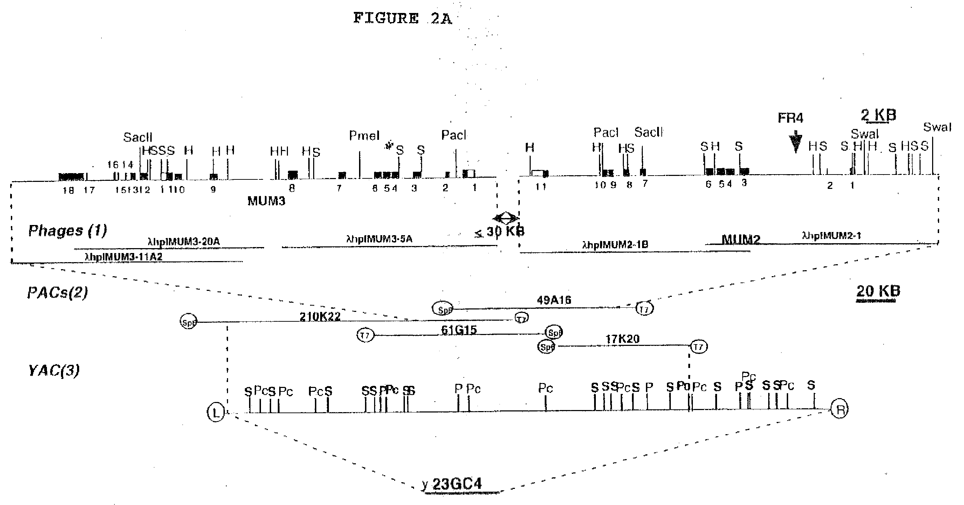

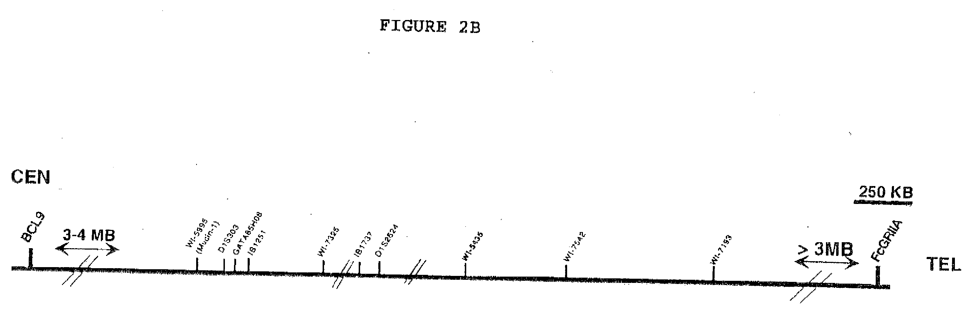

- **Figures 2A-2B.**: Genomic map of the lq21 locus in the vicinity of the FR4 breakpoint. Fig. 2A) Restriction endonuclease map and schematic representation of genomic clones, i.e. bacteriophages (1), P1 artificial chromosomes (PACs) (2), and yeast artificial chromosome (YAC) (3) spanning the germline lq21 locus at the FR4 breakpoint region (arrowhead). The name of each clone is placed directly on top of its representation. End fragments derived from the PAC and YAC inserts are depicted as circles, with either an SP6/T7 vector orientation (PAC), or left/right arm vector orientation (YAC). The top panel in Fig. 1A depicts the genomic organization of two genes surrounding the FR4 breakpoint. The two genes were identified by exon trapping of PAC 49A16. They are closely spaced in the genome, within ≤ 30 Kb of each other and are named MUM2 and MUM3 (multiple myeloma-2 and 3). In the scheme of their genomic loci, black boxes indicate coding exons, whereas white and light or medium grey boxes indicate non-coding exons. Connecting introns are lines. MUM3 (left) gives rise to three alternatively spliced mRNAs, all sharing a common 5' untranslated region (UTR) but diverse 3' UTRs (marked by different shades). Numbers underneath the boxes identify the order of exons in the cDNA. Exons less than 100 bp are depicted as thin vertical lines. The position and size of each exon was determined by sequencing of genomic PAC and phage clones and by hybridization of cDNA probes to endonuclease-digested clone DNA. PAC and YAC mapping was performed by partial digestion with rare cutting enzymes followed by Pulse-Field-Gel-Electrophoresis and hybridization to internal and end-derived probes. Dashed lines align regions of overlap. S, SacI; H, HindIII; S, Swal; PC, PacI; P, PmeI; Fig. 2B) Genethon genetic linkage map of lq21 in the region of the MUM2/MUM3 locus. Sequence-tagged sites (STS) are ordered in approximate distance previously determined by Dib, C., et al. (1996) Nature, 380:162-164. STS WI-5435 (in bold) is contained within YAC 23GC4 and PAC 49A16. Parallel vertical lines represent interrupted segments, whose approximate size is depicted above in megabases (MB). Sizing was estimated by the size of nonchimeric YAC contigs between two markers. The BCL9 gene at the centromere was cloned from a different t(1;14) (q21;q32) breakpoint by Willis T.G. et al., (1998) Blood 91, 6:1873-1881. The FcGRIIA gene is at the lq21-q22 chromosomal band border.

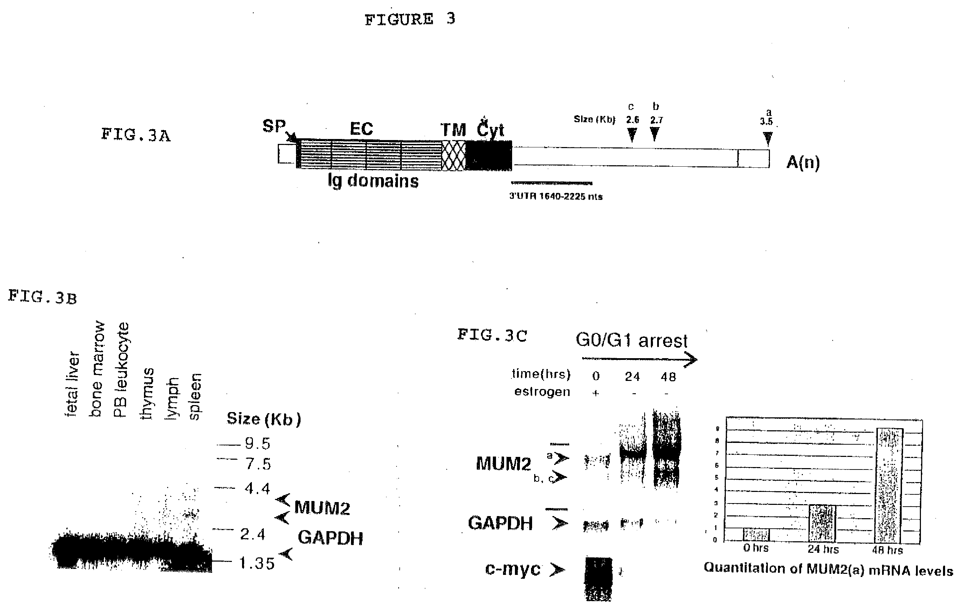

- **Figures 3A-3C.**: MUM2 mRNA structure and expression pattern. Fig. 3A) Schematic representation of MUM2 mRNA. Pattern-filled, wide boxes represent coding domains and narrow empty boxes represent untranslated regions. SP, signal peptide; EC, extracellular domain; TM, transmembrane domain; CYT, cytoplasmic domain; A(n), polyA tail. The extracellular region is composed of four immunoglobulin-like domains as depicted. Alternative polyadenylation signals (arrows) generate three MUM2 mRNA species (a, b, c) whose length (in Kb) ranges from 2.6 - 3.5. Fig. 3B) Northern blot analysis of MUM2 mRNA expression in human tissues of the immune system. The cDNA probe used for the analysis is shown as a solid bar underneath the mRNA scheme in Fig. 3A). Each lane contains 2*µ*g mRNA of the corresponding tissue. On the right side of the blot, the position of RNA molecular weight markers is depicted. The position of MUM2 and GAPDH mRNA transcripts is shown by arrows. (A GAPDH probe was included in the hybridization as an internal control - 0.15 ng labelled +50 ng unlabelled probe-). The rsults of this analysis show weak expression of MUM2 in lymph node and spleen. MUM2 expression was not detected in a variety of other human tissues (data not shown). Fig. 3C) Northern blot analysis of MUM2 expression in total RNA from EREB, a conditional EBV-transformed B lymphoblastoid cell line. EREB carries the EBV genome with an EBNA2-estrogen receptor fusion protein, active only in the presence of estrogen. For this experiment, cells were grown in the presence of estrogen (1*µ*g/ml), followed by estrogen withdrawal for the indicated times. Upon estrogen withdrawal, EREB cells undergo G0/G1 arrest, determined by the loss of c-myc expression. In Fig. 3C, a Northern blot of EREB total RNA (10*µ*g per lane) was hybridized with the MUM2 cDNA probe shown in Fig. 3A and the GAPDH. internal control probe, as in Fig. 3B. Arrows indicate the position of the corresponding mRNAs on the EREB blot. a, band c correspond to the MUM2 species in panel Fig 3A. The same blot was then stripped and reprobed with a c-myc cDNA probe (exon 2) to verify cellular G0/G1 arrest. Quantitation of MUM2 mRNA by the use of a phosphorimager densitometric analysis demonstrates a 10-fold increase in their levels within 48 hrs of estrogen withdrawal, suggesting that MUM2 expression is elevated as the cells enter a resting phase.

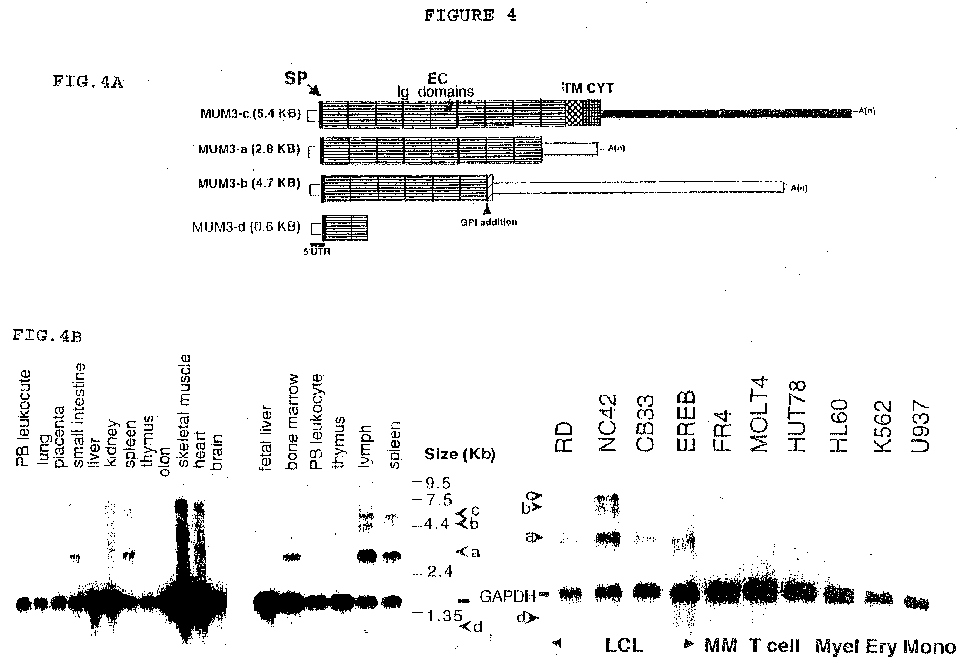

- **Figures 4A-4B.**: MUM3 mRNA structure and expression pattern. Fig. 4A) Schematic representation of MUM3 mRNA Pattern-filled, wide boxes represent coding domains and narrow empty or gray boxes represent untranslated regions. SP, signal peptide; EC, extracellular domain; TM, transmembrane domain; CYT, cytoplasmic domain; A(n), polyA tail. The extracellular region is composed of immunoglobulin-like domains, as depicted. Alternative splicing generates four mRNA species with diverse subcellular localization. MUM3-a and -d proteins are secreted, whereas MUM3-b contains a hydrophobic stretch of amino acids at its C-terminus which may serve as a signal for addition of a glycophosphatidyl-inositol anchor (GPI-anchor), as shown. MUM3-c spans the plasma membrane. Sequence identity among species is indicated by identical filling. Fig. 4B) Northern blot analysis of MUM3 mRNA expression in multiple human tissues (left) and in various lymphoid and non-lymphoid cell lines (right). The cDNA probe used is shown as a solid bar below the cDNA scheme in Fig. 4A. Each lane contains 2*µ*g mRNA of the corresponding tissue or cell line. The position of MUM3 and GAPDH mRNA transcripts is shown by arrows. (A GAPDH probe was included in the hybridization as an internal control as described in Fig. 3) a, b, c and d correspond to the MUM3 mRNA species shown in Fig. 4A. RD, NC42 and CB33, Epstein-Barr virus transformed B lymphoblastoid cell lines; EREB, conditional EBV-transformed B lymphoblastoid cell line; FR4, plasma cell line; MOLT4 and HUT78, T cell lines; HL60 and U937, myelomonocytic cell lines; K562, erythroid cell line. The results suggest that MUM3 is expressed solely in the immune system tissues of bone marrow, lymph and spleen and in particular in B cells with a lymphoblastoid phenotype.

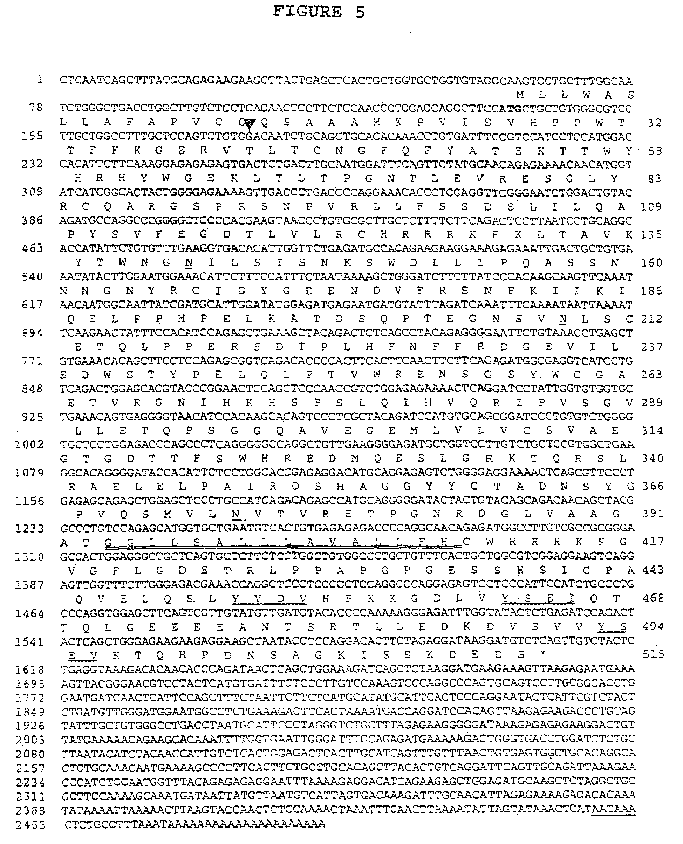

- **Figure 5.**: Nucleotide and amino acid sequence of human MUM2. The deduced amino acid sequence is shown above the nucleotide sequence in one-letter code and is numbered on the right, with position 1 set to the first codon of the signal peptide. The predicted signal peptidase site was derived by a computer algorithm described in Nielsen et al., Protein Engineering 10, 1-6 (1997) and is marked by an arrowhead. The polyadenylation signal AATAAA is underlined. Potential sites for N-glycosylation are also underlined in the amino acid sequence. A hydrophobic stretch of 16 amino acids predicted to span the plasma membrane is doubly underlined. Consensus SH2-binding sites are highlighted by a wavy underline.

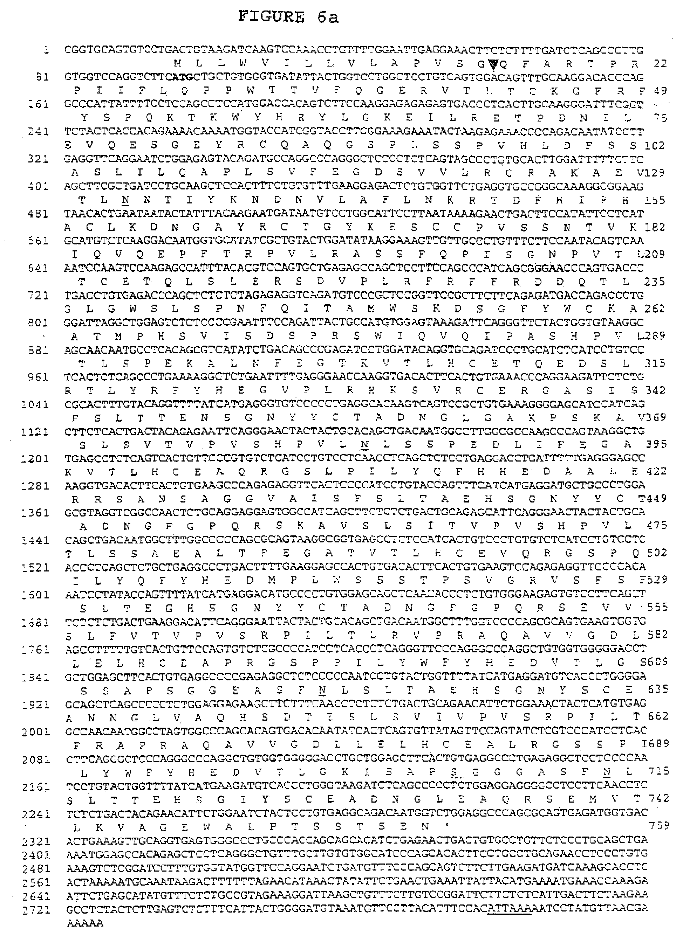

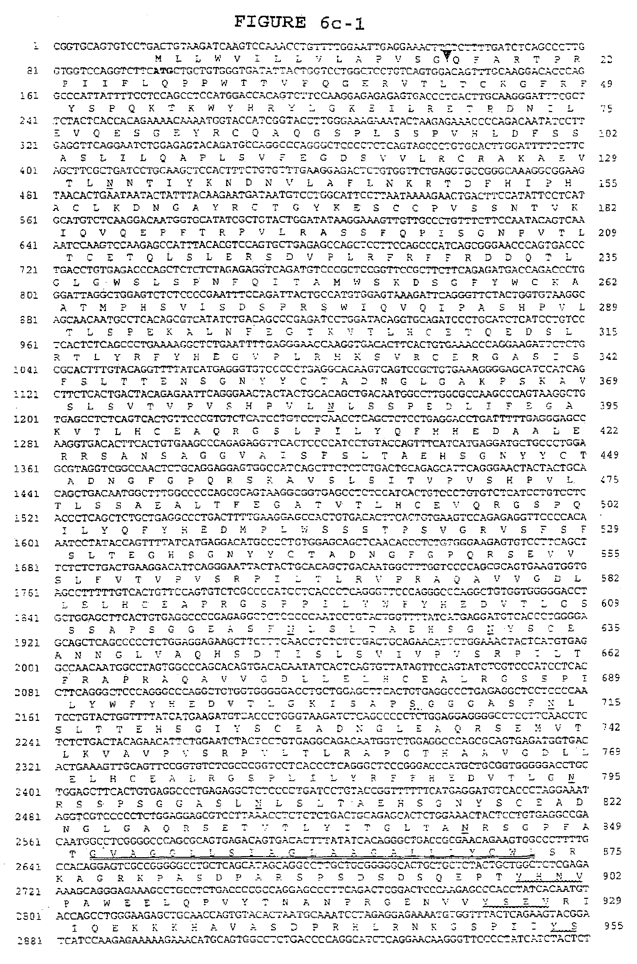

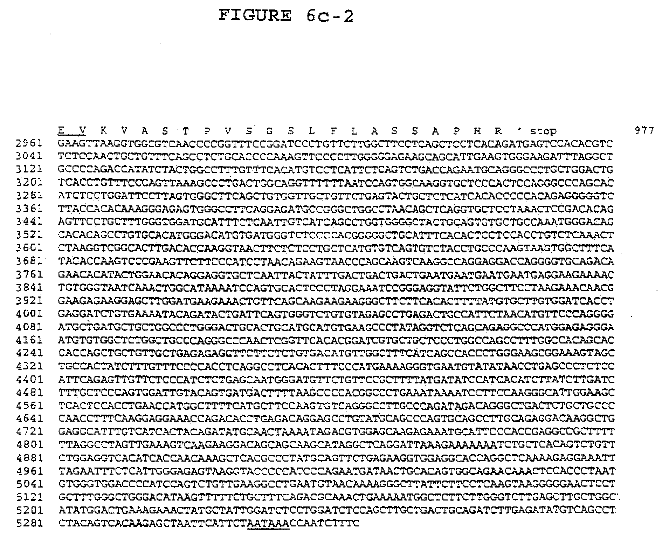

- **Figure 6A.**: Nucleotide and amino acid sequence of human MUM3-a. The deduced amino acid sequence is shown above the nucleotide sequence in one-letter code and is numbered on the right, with position 1 set to the first codon of the signal peptide. The predicted site for signal peptidase cleavage was derived as previously stated above and is marked by an arrowhead. The polyadenylation signal ATTAAA is underlined. Potential sites for N-glycosylation are also underlined in the amino acid sequence. The protein lacks a transmembrane domain and is predicted to be secreted.

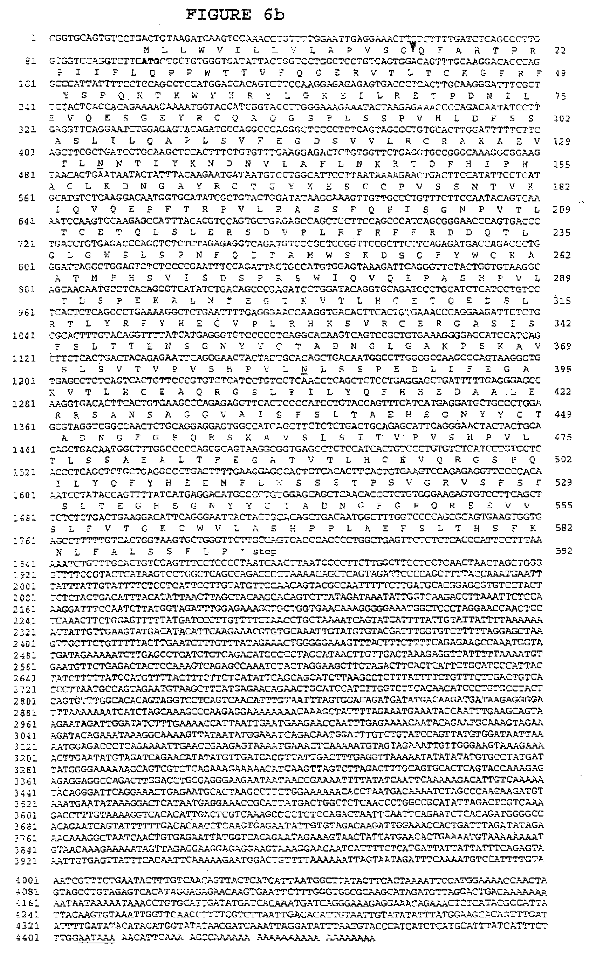

- **Figure 6B.**: Nucleotide and amino acid sequence of human MUM3-b. The deduced amino acid sequence is shown above the nucleotide sequence in one-letter code and is numbered on the right, with position 1 set to the first codon of the signal peptide. The predicted site for signal peptidase cleavage was derived as previously stated above and is marked by an arrowhead. The polyadenylation signal AATAAA is underlined. Potential sites for N-glycosylation are underlined in the amino acid sequence.
- **Figure 6C-1-6C-2.**: Nucleotide and amino acid sequence of human MUM3-c. The deduced amino acid sequence is shown above the nucleotide sequence in one-letter code and is numbered.on the right, with position 1 set to the first codon of the signal peptide. The predicted site for signal peptidase cleavage was derived as previously stated above and is marked by an arrowhead. The polyadenylation signal AATAAA is underlined. Potential sites for N-glycosylation are underlined in the amino acid sequence. A hydrophobic stretch of 23 amino acids predicted to span the plasma membrane is doubly underlined. Consensus SH2-binding sites are highlighted by a wavy underline.
- **Figures**: **7A-7C.** t(1;14) (q21;32) in FR4 generates a MUM2/Ca fusion transcript. Fig. 7A) Schematic representation of ther der(14) genomic clone λFR4B-5 and of the germline IgHA1 locus. The FR4 breakpoint is marked by an arrow. Filled and open boxes represent the MUM2 and Calpha coding and non-coding exons respectively. The position of the MUM2 exon 1 probe used for Northern blot analysis is shown by a bar. Fig. 7B) Northern blot analysis with a MUM2 exon 1 probe on FR4 and additional cell lines detects an abnormal message of 0.8 Kb, selectively in FR4. Arrowheads point to the location of normal MUM2 message in EREB mRNA. JJN3 and U266, myeloma cell lines; EREB, conditional EBV-transformed B lymphoblastoid cell line. Two *µ*g of polyA+ RNA were loaded per lane. Fig. 7C) Nucleotide ans amino acid sequence of the MUM2-Ca fusion cDNA in FR4. The cDNA was amplified by RT-PCR from FR4 total RNA using the primers shown in Fig. 7A, and was subsequently subcloned and sequenced. The deduced amino acid sequence is shown above the nucleotide sequence in one-letter code and is numbered on the right with position 1 set to the first codon of the signal peptide. The predicted site for signal peptidase cleavage was derived as previously stated above and is marked by an arrowhead. The polyadenylation signal AATAAA is underlined. The Calpha transmembrane domain is underlined. The MUM2 portion of the cDNA is shown on italic. H, HindIII; B, BamHI; X, XhoI; Sα, IgA switch region; EC, extracellular region; TM, transmembrane; CYT, cytoplasmic domain.

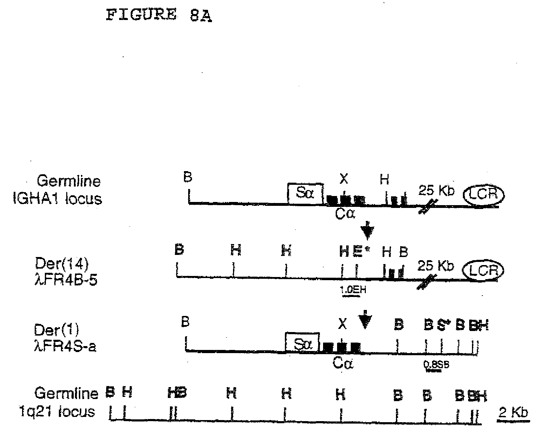

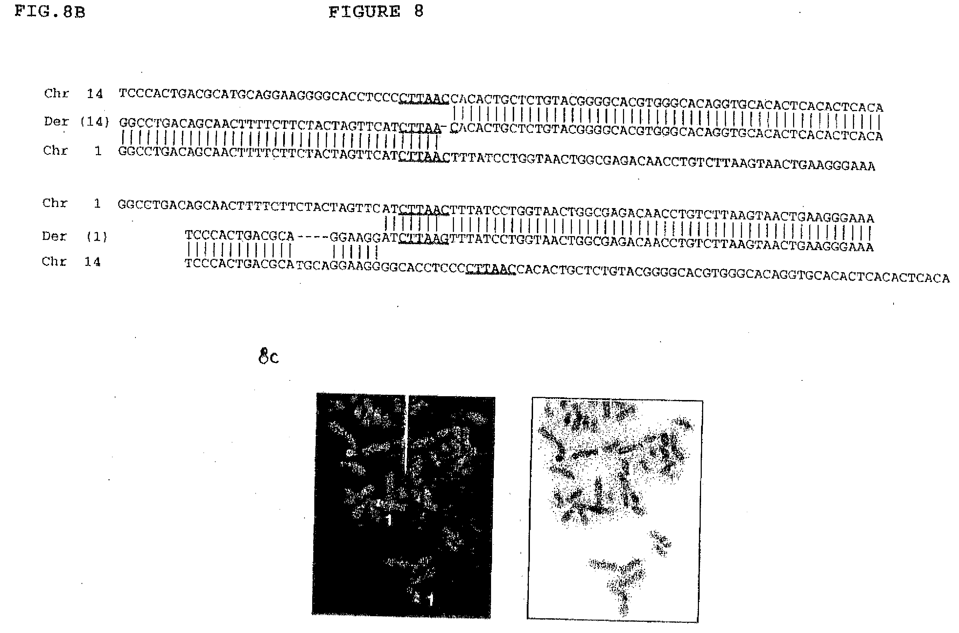

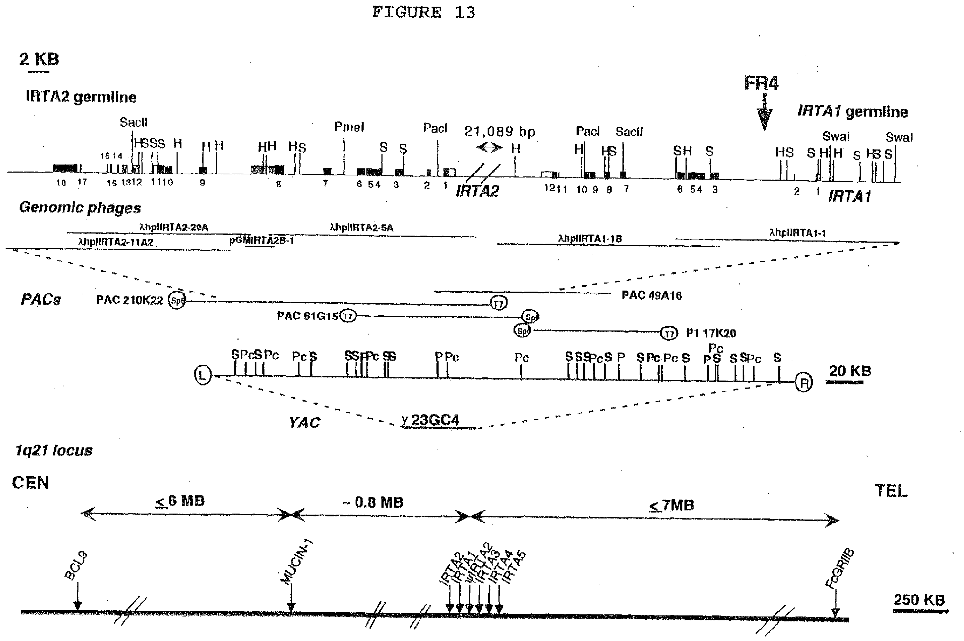

- **Figures 8A-8C.**: Molecular cloning of the translocation t(1;14)(q21;q32) in the FR4 multiple myeloma cell line. Fig. 8A) Schematic representation of the phage clones representing der(14) and der(1) breakpoints and the germline IGH and lq21 loci. Chromosome 14 sequences are indicated by a solid black line with black boxes representing Ca1 exons. Chromosome 1 sequences are shown as a grey line. The probes used for chromosomal mapping are indicated below the map. Restriction enzyme codes are: B, BamHI; H, HindIII; X, XhoI; S, SacI; E, EcoRI. For enzymes marked by (*) only sites delineating the probes are shown. Sa: IgA switch region; LCR: 3'IgH locus control region. Fig. 8B) Nucleotide sequence of the breakpoint junctions and their alignment to the corresponding germline regions of chromosomes 14 and 1. Fig. 8C) Left, fluorescence in situ hybridization (FISH) analysis on human normal metaphase spreads with the PAC clone 49A16 (Fig. 13) spanning the germlinelq21 region at the FR4 breakpoint. Right, DAPI stained image from the same metaphase spread.

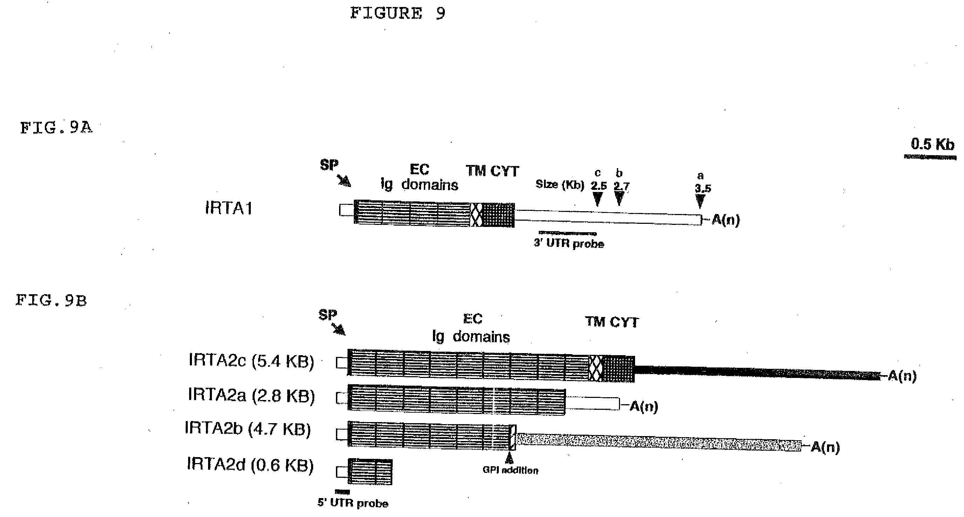

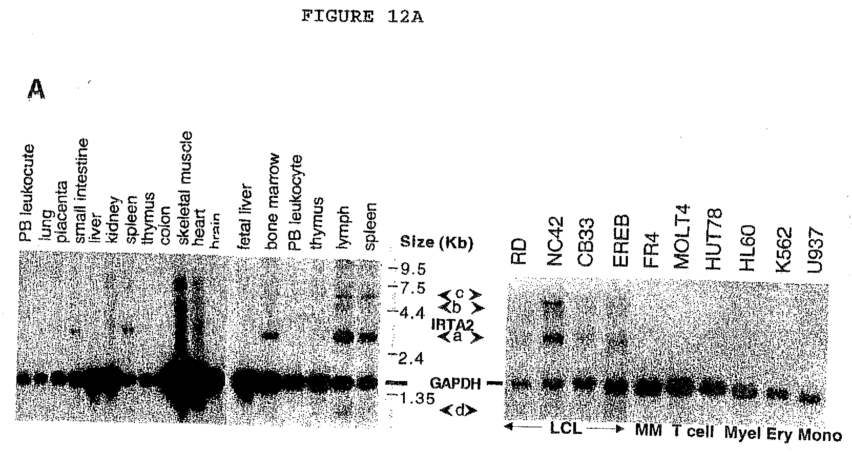

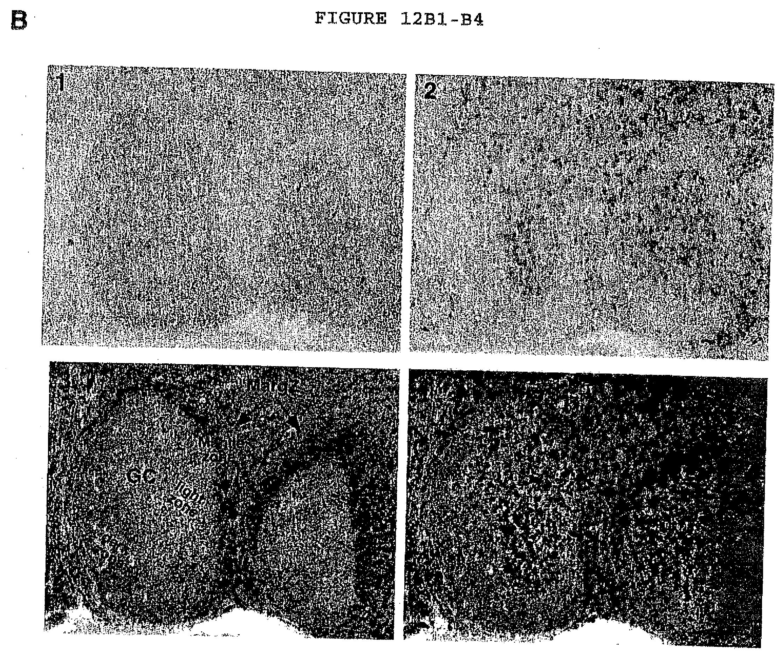

- **Figures 9A-9B.**: Structure of *IRTA1* and *IRTA2* cDNAs. Figs. 9A,9B) Schematic representation of the full-length *IRTA1* (Fig. 9A) and *IRTA2* (Fig. 9B) cDNAs. Pattern-filled, wide boxes represent coding domains and narrow boxes represent untranslated regions (UTR) The predicted site for signal peptidase cleavage is marked by an arrowhead and was derived according to the SignalIP World **Wide** Web **server** at http://www.cbs.dtu.dk/services/SignalIP. The transmembrane domain prediction algorithm is described in Tusnady et al, 1998. SP, signal peptide; EC, extracellular domain; Ig, immuno-globulin-type; TM, transmembrane domain; CYT, cytoplasmic domain; A(n), polyA tail; GPI, glycophosphatidyl inositol. In (Fig. 9A), arrows in the 3' UTR indicate different polyadenylation addition sites utilized in the *IRTA1* cDNA. In (Fig. 9B), different 3'UTR regions in *IRTA2* isoforms are differentially shaded. Bars underneath the UTR regions in (Fig. 9A) and (Fig. 9B) identify probes used for Northern blot analysis in Figure 12.

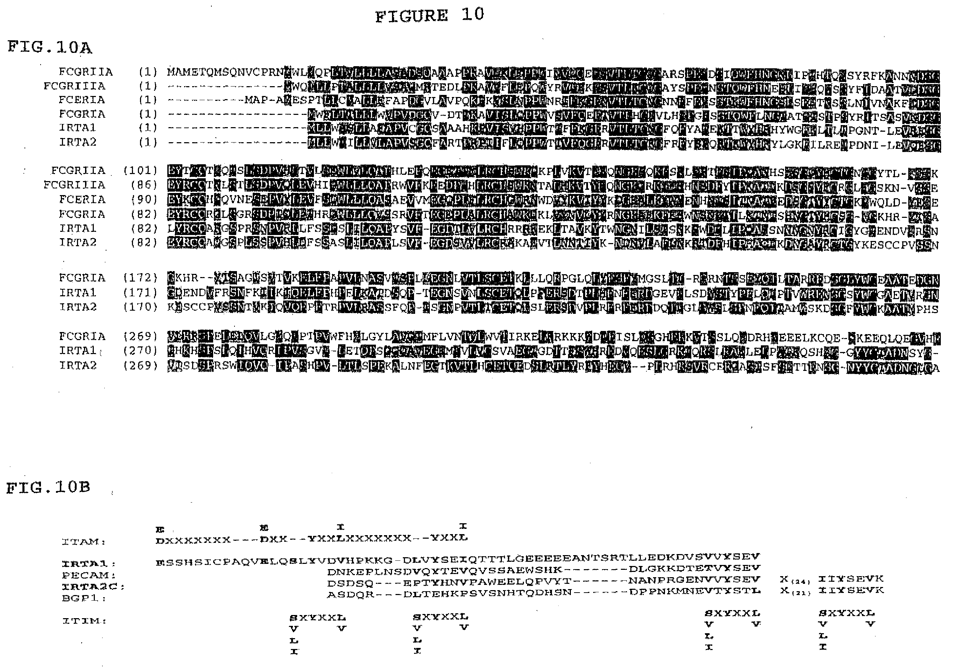

- **Figures 10A-10B.**: Comparison of the amino acid sequences of IRTA1 and IRTA2 with members of the Fc Receptor family Fig. 10A) Multiple sequence alignment of the first two (top) and the third (bottom) extracellular Ig-domains of IRTA1 and IRTA2 to Fc receptor family members. The sequences were compared using the ClustalW program (Thompson et al., 1994). Black-shaded boxes indicate conserved aminoacids among all sequences; dark-grey shaded boxes indicate conserved aminoacids among at least half of the sequences; light-shaded boxes indicate conservative substitutions. Fig. 10B) Alignment of the SH2-binding domains of IRTA1 and IRTA2 with the ITAM and ITIM consensus motifs. Conserved aminoacid positions are in bold. Symbol X represents any aminoacid.

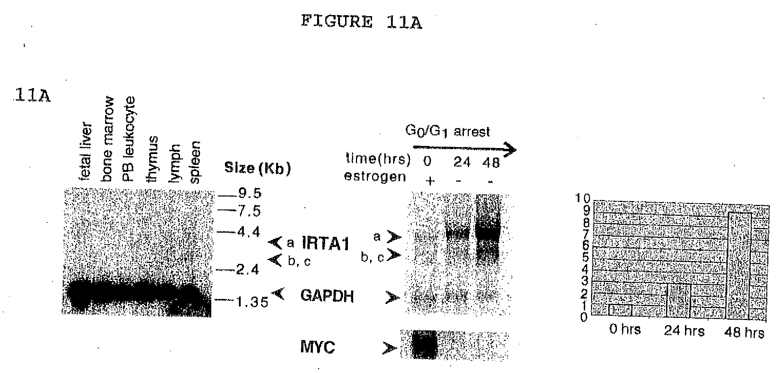

- **Figures 11A-11B-4.**: IRTA1 expression pattern. Fig. 11A) Left panel. Northern blot analysis of IRTA1 mRNA expression in tissues of the human immune system. Each lane contains 2mg mRNA. The position of RNA molecular weight markers is depicted on the right side of the blot. The positions of the *IRTA1* and *GAPDH* mRNA transcripts are shown by arrows. (A GAPDH probe was included in the hybridization as an internal control-0.15 ng labelled + 50 ng unlabelled probe-). Right Panel. Northern blot analysis of *IRTA1* expression in total RNA from the ER/EB cell line (10 mg per lane). For this experiment, cells were grown in the presence of estrogen (1mg/mz), followed by estrogen withdrawal for the indicated times. Arrows indicate the positions of the corresponding mRNAs. a, b and c correspond to the *IRTA1* differentially polyadenylated species. The same blot was stripped and reprobed with a *MYC* cDNA probe (exon 2) to verify cellular G₀/G₁ arrest. Densitometric analysis of *IRTA1* mRNA levels is plotted in the adjacent column graph. The cDNA probe used is shown as a solid bar underneath the *IRTA1* mRNA scheme in Figure 9A. Fig. 11B-1-11B-4) In *situ* hybridization analysis of *IRTA1* expression in serial sections of human tonsil. 1. Sense *IRTA1* probe 2. Antisense *IRTA1* probe 3. H&E staining 4. Antisense *IRTA1* signal superimposed over an H&E stained section. GC, germinal center, MargZ, marginal zone
- **Figure 12A-12B-4.**: *IRTA2* expression pattern. Fig. 12A) Northern blot analysis of *IRTA2* mRNA expression in multiple human tissues (left panel) and in various lymphoid and non-lymphoid cell lines (right panel). Each lane contains 2mg mRNA. The positions of the *IRTA2* and *GAPDH* transcripts are shown by arrows. a, b, c and d correspond to the alternatively spliced *IRTA2* mRNA isoforms. RD, NC42 and CB33, Epstein-Barr virus transformed B lymphoblastoid cell lines; EREB, conditional EBV-transformed B lymphoblastoid cell line; FR4, plasma cell line; MOLT4 and HUT78, T cell lines; HL60 and U937; myelomonocytic cell lines; K562, erythroid cell line. The cDNA probe used is shown as a solid bar underneath the *IRTA2* mRNA scheme in Figure 9B. Figs. 12B-1-12B-4) *In situ* hybridization analysis of *IRTA2* mRNA expression in human tonsil. Fig. 12B-1. Sense *IRTA2* cDNA probe, Fig. 12B-2. Antisense *IRTA2* cDNA probe, Fig. 12B-3. H&E staining, Fig. 12B-4. Antisense *IRTA2* cDNA probe signal superimposed over H&E stained section. GC, germinal center, MargZ, marginal zone
- **Figure 13.**: Map of the germline lq21 region spanning the FR4 breakpoint and genomic organization of *IRTA1* and *IRTA2.* Primers used to amplify *IRTA1* exons from spleen cDNA are marked by arrowheads on top panel. Black and light boxes indicate coding and non-coding exons respectively. Arrows indicate position of *BCL9, MUC1, IRTA* family and *FCGRIIB* loci. S, SacI; H, HindIII; S, SwaI; Pc, PacI; P, PmeI; Mb, Megabases

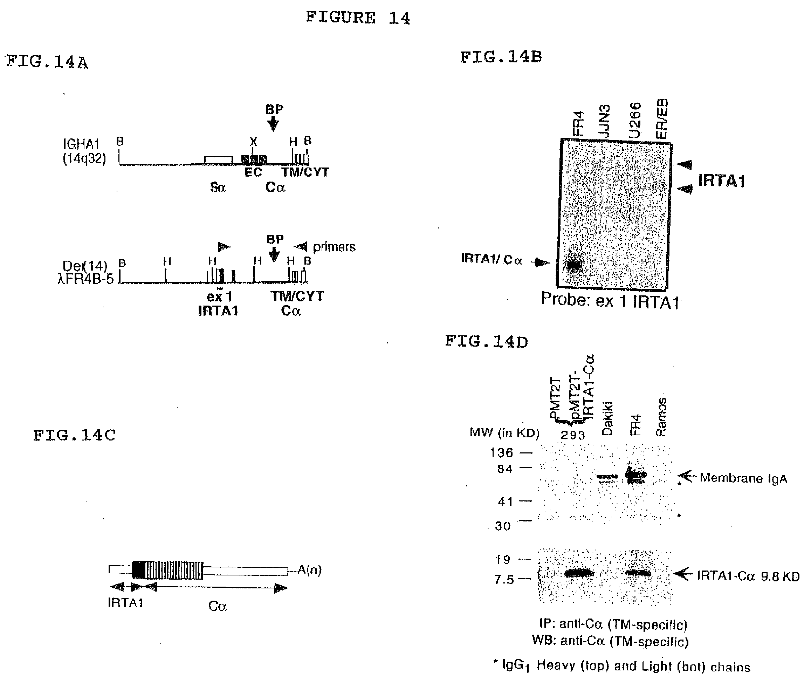

- **Figures 14A-14D.**: t(1;14) (q21;q32) in FR4 generates an *IRTA1*/*Cα* fusion transcript. Fig. 14A) Schematic representation of the der(14) genomic clone 1FR4B-5 and of the germline *IgCα₁* locus. The FR4 breakpoint is marked by an arrow. Filled and open boxes represent the *IRTA1* and *Ca*₁ coding and non-coding exons respectively. Fig. 14B) Northern blot analysis with an *IRTA1* exon 1 probe (shown by a bar in Fig. 14A) on FR4 and additional cell lines detects an abnormal message in FR4. Arrowheads point to the location of normal *IRTA1* message in ER/EB mRNA. JJN3 and U266, myeloma cell lines. Two mg of polyA+ RNA loaded per lane. Fig. 14C) Schematic representation of the *IRTA1*/*Cα* fusion cDNA in FR4. The cDNA was amplified by RT-PCR from FR4 total RNA using the primers shown in (Fig. 14A), and sequenced after subcloning. Fig. 14D) SDS/PAGE analysis of immunoprecipitates obtained from vector control transfected and *IRTA1*/*Cα* transient expression construct transfected 293-T cells (lanes 1 & 2), or the following cell lines: mIgA positive lymphoblastoid cell line-Dakiki (lane 3), FR4 (lane 4), mIgM positive NHL cell line-Ramos (lane 5). H, HindIII; B, BamHI; X, XhoI; Sa, IgA switch region; EC, extracellular region; TM, transmembrane; CYT, cytoplasmic

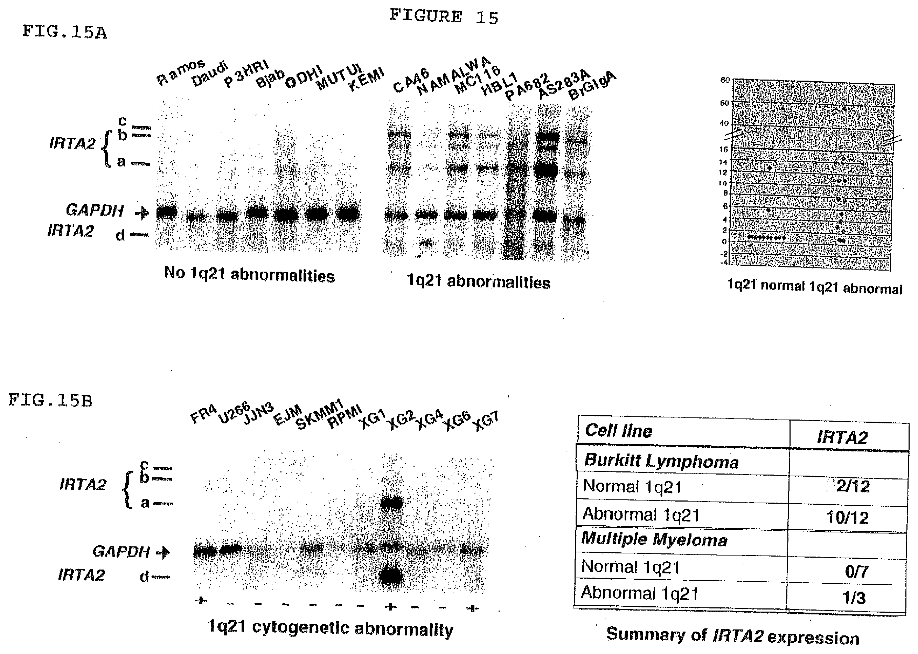

- **Figures 15A-15B.**: IRTA2 expression is deregulated in cell lines carrying lq21 abnormalities. Figs. 15A, 15B) Northern blot analysis of *IRTA2* mRNA expression in Burkitt lymphoma (Fig. 15A) and Multiple Myeloma (Fig. 15B) cell lines. The cDNA probe used is the same as in Fig. 12. Each lane contains 2mg mRNA. The positions of the *IRTA2* and *GAPDH* mRNA transcripts are shown by dashes and arrows, respectively. The relative levels of *IRTA2* mRNA expression in the left panel (Fig. 15A) were plotted on the right panel (Fig. 15A) after densitometric analysis and normalization versus the *GAPDH* levels. The right panel of (Fig. 15B) is a summary of the Northern blot analysis results.

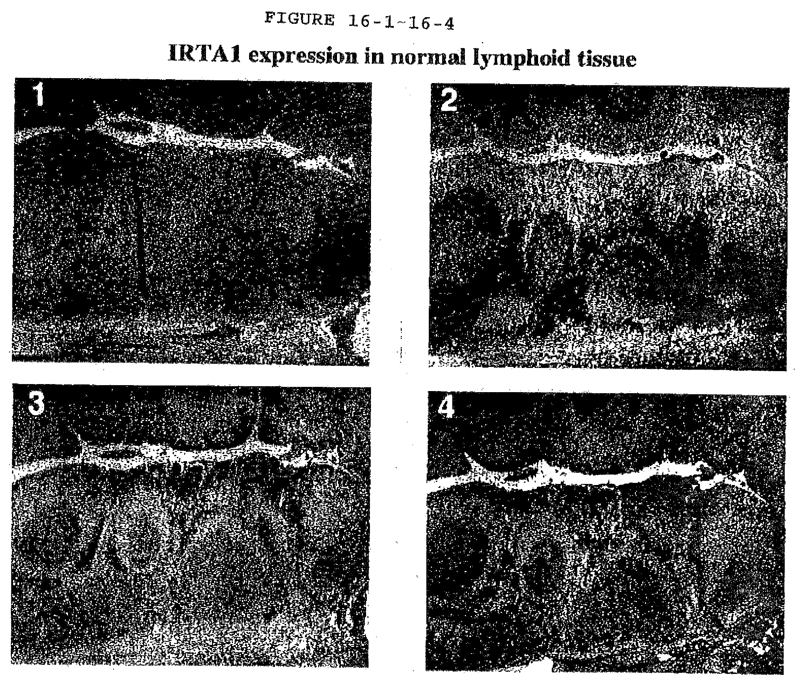

- **Figures 16-1-16-4**: IRTA1 expression in normal lymphoid tissue. Paraffin-embedded sections from normal human tonsil were stained with the following antibodies: Fig. 16-1) Negative control; Fig. 16-2) anti-CD3 mouse monoclonal to detect T cells; Fig. 16-3) anti-IRTA1 (mIRTA) mouse monoclonal; Fig. 16-4) anti-IRTA1 (J92884K) rabbit polyclonal. IRTA1 positive cells are located in the perifollicular and intraepithelial region of the tonsil, the equivalent of the marginal zone in the spleen.

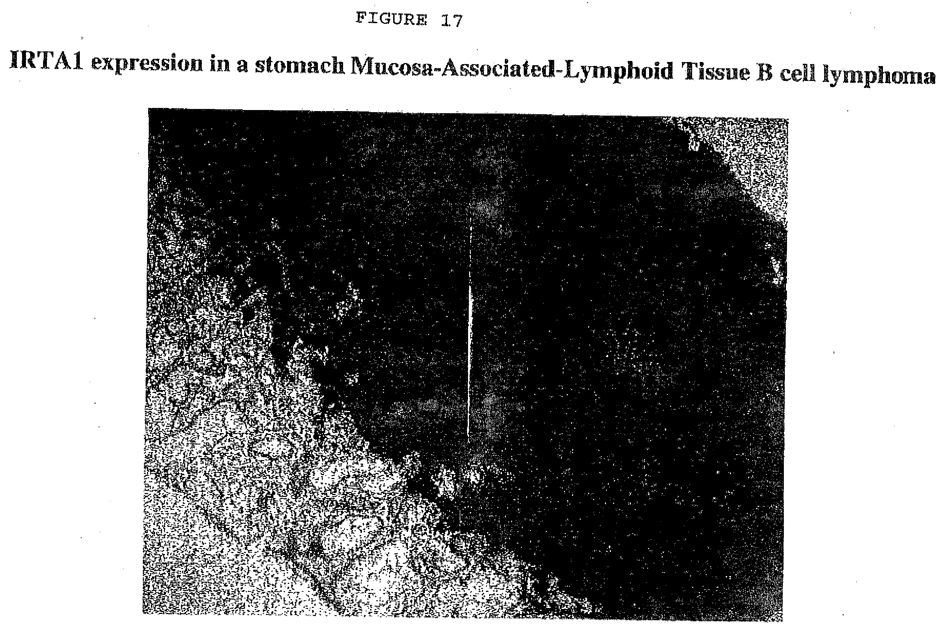

- **Figure 17**: IRTA1 expression in a stomach Mucosa-Associated-Lymphoid Tissue (MALT) B cell lymphoma. A paraffin-embedded section from a stomach MALT B cell lymphoma was stained with the anti-IRTA1 (mIRTA) mouse monoclonal antibody and counterstained with H&E. The majority of MALT lymphomas analyzed were IRTA1 positive. This antibody therefore can be an effective tool in the differential diagnosis of MALT lymphoma. The mIRTA1 antibody may also be proven useful in the therapy of this B cell tumor, similarly to the use of the anti-CD20 antibody (Rituximab) in the therapy of relapsed CD20-positive lymphomas (Foon K., Cancer J. 5 : p273).

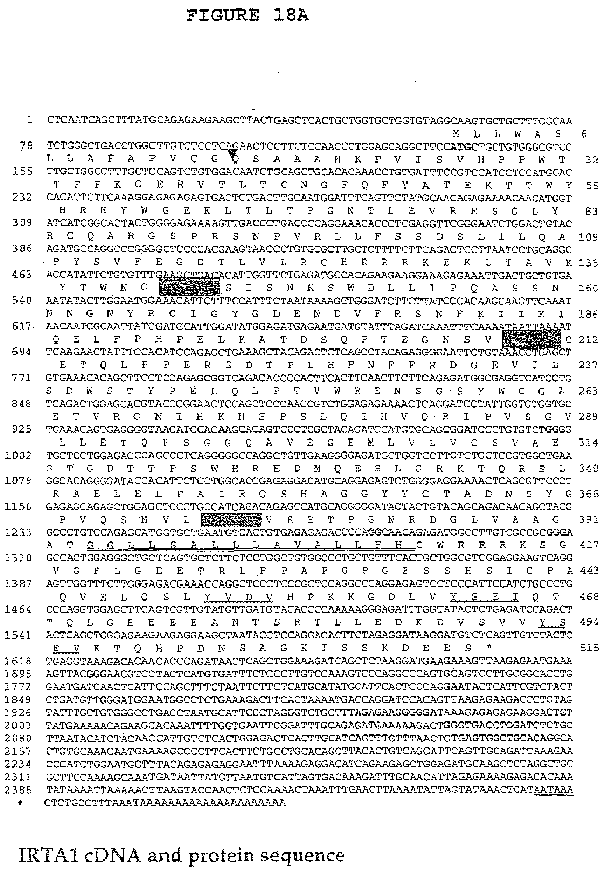

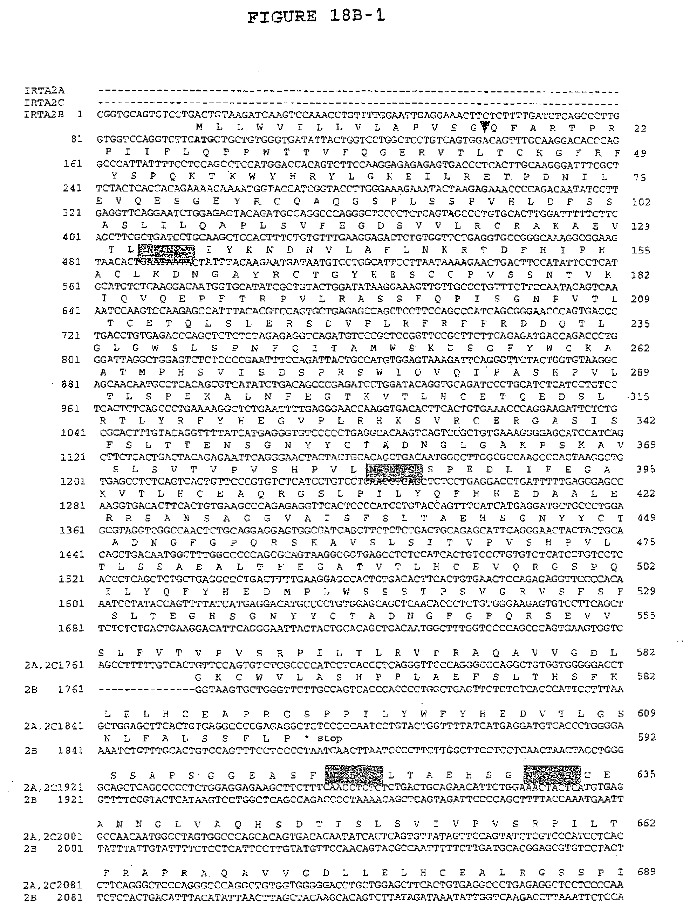

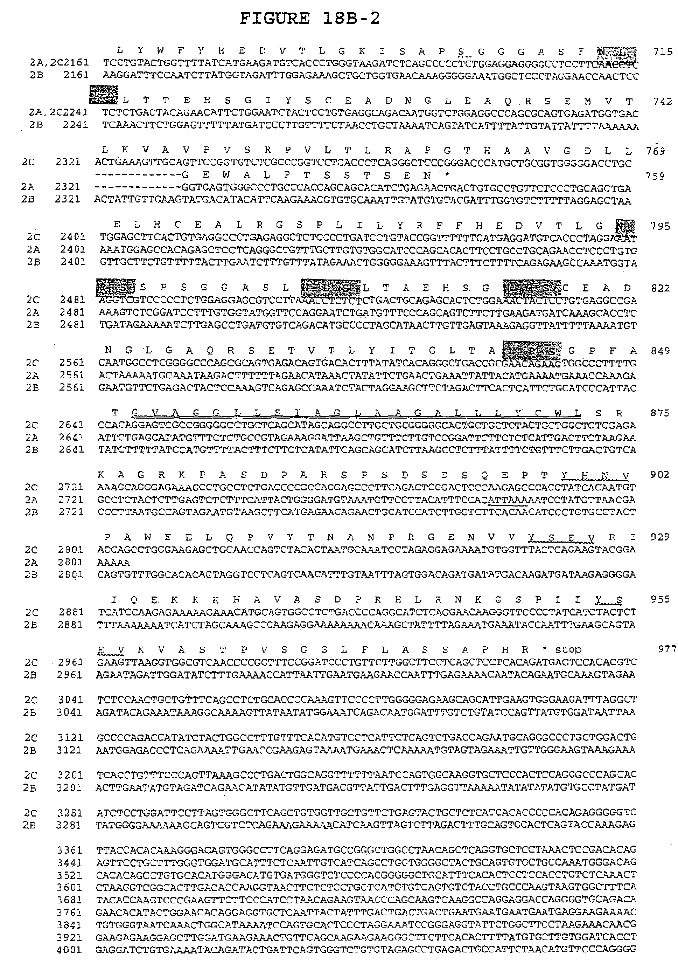

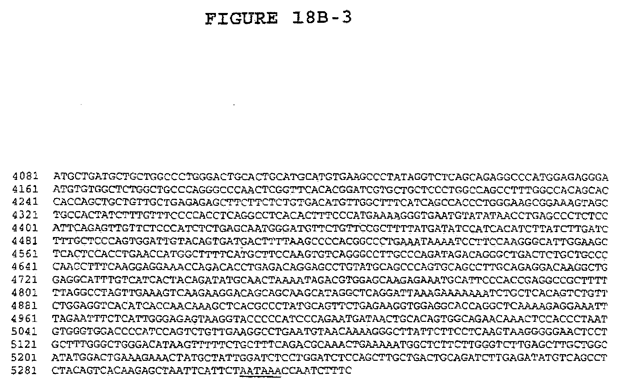

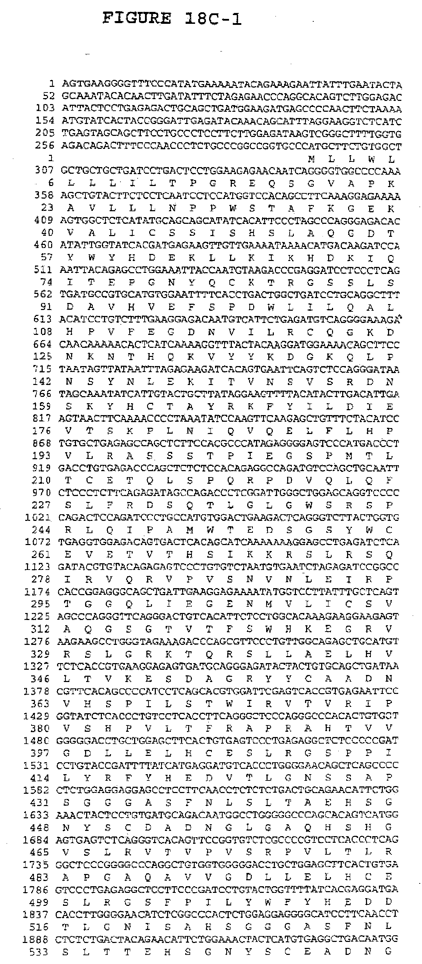

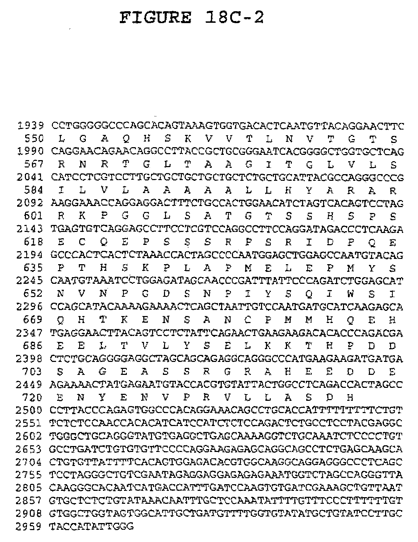

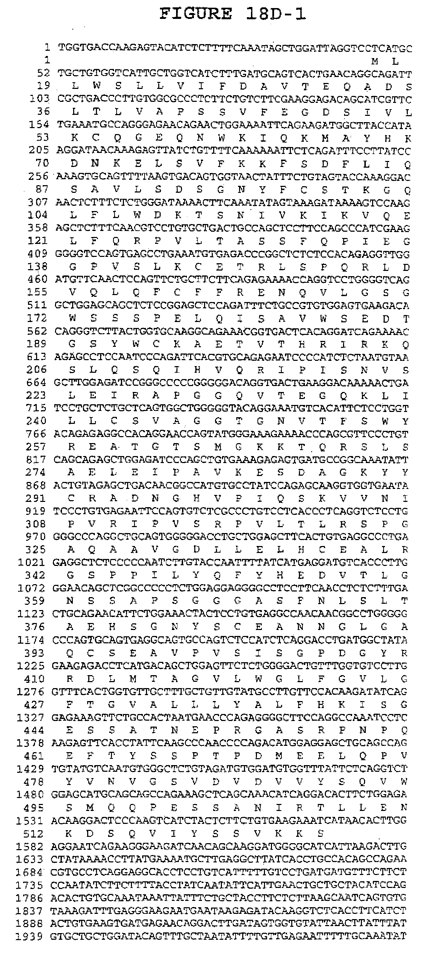

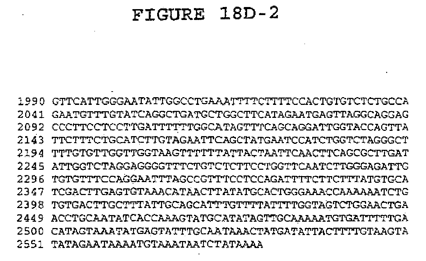

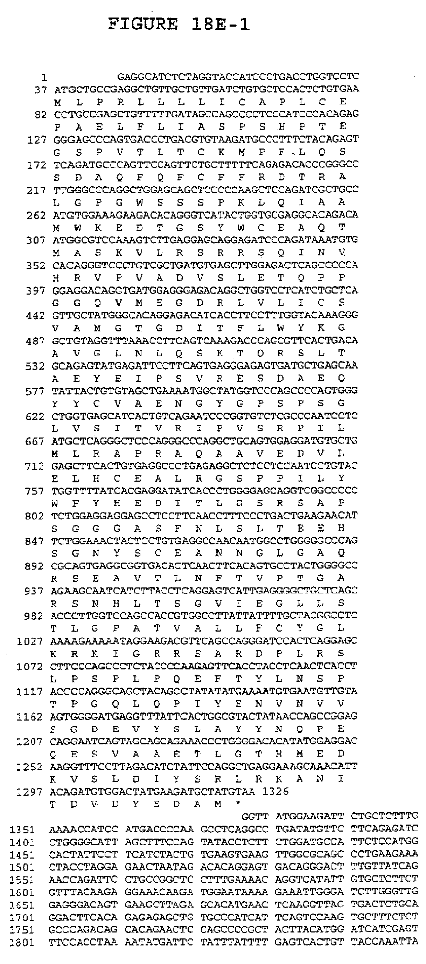

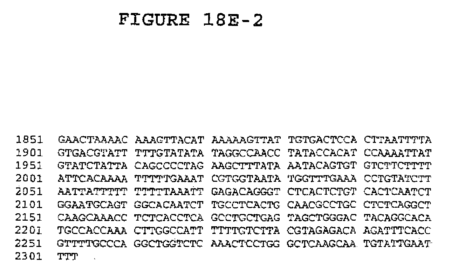

- **Figure 18A.**: IRTA1 cDNA and the amino acid sequence of the encoded IRTA1 protein.
- **Figures 18B-1-18B-3.**: IRTA2 cDNA and the amino acid sequence of the encoded IRTA2 protein.
- **Figures 18C-1-18C-2.**: IRTA3 cDNA and the amino acid sequence of the encoded IRTA3 protein.
- **Figures 18D-1-18D-2.**: IRTA4 cDNA and the amino acid sequence of the encoded IRTA4 protein.
- **Figures 18E-1-1BE-2.**: IRTA5 cDNA and the amino acid sequence of the encoded IRTA5 protein.

### DETAILED DESCRIPTION OF THE INVENTION

The following standard abbreviations are used throughout the specification to indicate specific nucleotides: C=cytosine; A=adenosine; T=thymidine and G=guanosine.

This invention provides an isolated nucleic acid molecule which encodes immunoglobulin receptor, Immunoglobulin superfamily Receptor Translocation Associated, IRTA, protein.

As used herein "Immunoglobulin Receptor Translocation Associated" genes, "IRTA" are nucleic acid molecules which encode novel immunoglobulin superfamily cell surface receptors in B cells which are important in B cell development, and whose abnormal expression, e.g. deregulated expression, perturbs cell surface B cell immunological responses and thus is involved in B cell malignancy, including lymphomagenesis.

Nucleic acid molecules encoding proteins designate "MUM-2" and "MUM-3" proteins in the First Series of Experiments are now called *"IRTA-1"* and *"IRTA-2"* genes, i.e. nucleic acid molecules which encode IRTA-1 and IRTA-2 proteins respectively. IRTA-3, -4 and -5 proteins are members of the same the immunoglobulin gene superfamily as are the IRTA-1 and IRTA-2 proteins.

In an embodiment of the above-described isolated nucleic acid molecule, the encoded IRTA protein is IRTA4 protein comprising the amino acid sequence set forth in Figures 18D-1-18D-2 (SEQ ID NO: 7).

In another embodiment of any of the above-described isolated nucleic acid molecules, the nucleic acid molecule is DNA. In further embodiments, the DNA is cDNA. In additional embodiments, the DNA is genomic DNA. In another embodiment, the nucleic acid molecule is an RNA molecule.

In an aspect , the DNA molecule is cDNA having the nucleotide sequence set forth in Figure 18A (SEQ ID NO:8).

In a preferred embodiment of the isolated nucleic acid molecule, the nucleic acid molecule encodes human IRTA4 protein.

In another aspect, the isolated nucleic acid molecules are operatively linked to a promoter of DNA transcription. In yet another aspect of the isolated nucleic acid molecule, the promoter comprises a bacterial, yeast, insect, plant or mammalian promoter.

Described is a vector comprising any of the above-described isolated nucleic acid molecule encoding IRTA4 protein , including but not limited to mammalian IRTA4 proteins, of which human and murine are preferred. In an aspect the vector is a plasmid.

Described is a host cell comprising the above-described vector comprising any of the above-described isolated nucleic acid molecule encoding IRTA4 protein

The isolated nucleic acid molecules in such vectors can be operatively linked to a promoter of DNA transcription. In another aspect of the host cell, the cell is selected from a group consisting of a bacterial cell, a plant cell, and insect cell and a mammalian cell.

Disclosed is a method of producing an IRTA polypeptide (protein) which comprises: (a) introducing a vector comprising an isolated nucleic acid which encodes an immunoglobulin receptor, Immunoglobulin superfamily Receptor Translocation Associated, IRTA, protein into a suitable host cell; and (b) culturing the resulting cell so as to produce the polypeptide. In further embodiments, the IRTA protein produced by the above-described method may be recovered and in a still further embodiment, may be purified either wholly or partially. In an embodiment the IRTA protein is IRTA4.
In a further embodiment, the IRTA protein may be a mammalian protein. In still further embodiments, the mammalian protein may be a human or mouse IRTA protein.

IRTA genes (nucleic acid molecules encoding IRTA proteins IRTA1, IRTA2, IRTA3, IRTA4 and IRTA5) are useful for the production of the IRTA proteins encoded thereby. IRTA proteins are useful for production of antibodies; such antibodies are used as reagents for differential diagnosis of lymphoma subtypes in hematopathology. Antibodies directed against IRTA proteins and which bind specifically to IRTA proteins also have therapeutic uses, i.e. to specifically target tumor cells, which may be used and administered similarly to "Rituximab" (an anti-CD20 antibody), which is an antibody approved by the FDA for therapy of relapsed CD20-positive lymphomas (Foon K., Cancer J. 6(5):273). Anti-IRTA1, anti-IRTA2, anti-IRTA3, anti-IRTA4 and anti-IRTA5 antibodies are also useful markers for isolation of specific subsets of B cells in researchstudies of normal and tumor B cell biology. Moreover, Anti-IRTA1, anti-IRTA2, anti-IRTA3, anti-IRTA4 and anti-IRTA5 antibodies are useful research reagents to experimentally study the biology of signaling in normal and tumor B cells.

Methods of introducing nucleic acid molecules into cells are well known to those of skill in the art. Such methods include, for example, the use of viral vectors and calcium phosphate co-precipitation. Accordingly, nucleic acid molecules encoding IRTA proteins IRTA1, IRTA2, IRTA3, IRTA4 and IRTA5 may be introduced into cells for the production of these IRTA proteins.

Numerous vectors for expressing the inventive protein IRTA4 may be employed. Such vectors, including plasmid vectors, cosmid vectors, bacteriophage vectors and other viruses, are well known in the art. For example, one class of vectors utilizes DNA elements which are derived from animal viruses such as bovine papilloma virus, polyoma virus, adenovirus, vaccinia virus, baculovirus, retroviruses (RSV, MMTV or MoMLV), Semliki Forest virus or SV40 virus. Additionally, cells which have stably integrated the DNA into their chromosomes may be selected by introducing one or more markers which allow for the selection of transfected host cells. The markers may provide, for example, prototrophy to an auxotrophic host, biocide resistance or resistance to heavy metals such as copper. The selectable marker gene can be either directly linked to the DNA sequences to be expressed, or introduced into the same cell by cotransformation.

Regulatory elements required for expression include promoter sequences to bind RNA polymerase and transcription initiation sequences for ribosome binding. Additional elements may also be needed for optimal synthesis of mRNA. These additional elements may include splice signals, as well as enhancers and termination signals. For example, a bacterial expression vector includes a promoter such as the lac promoter and for transcription initiation the Shine-Dalgarno sequence and the start codon AUG. Similarly, a eukaryotic expression vector includes a heterologous or homologous promoter for RNA polymerase II, a downstream polyadenylation signal, the start codon AUG, and a termination codon for detachment of the ribosome. Such vectors may be obtained commercially or assembled from the sequences described by methods well known in the art, for example the methods described above for constructing vectors in general.

These vectors may be introduced into a suitable host cell to form a host vector system for producing the inventive proteins. Methods of making host vector systems are well known to those skilled in the art.

Suitable host cells include, but are not limited to, bacterial cells (including gram positive cells), yeast cells, fungal cells, insect cells and animal cells. Suitable animal cells include, but are not limited to HeLa cells, Cos cells, CV1 cells and various primary mammalian cells. Numerous mammalian cells may be used as hosts, including, but not limited to, the mouse fibroblast cell NIH-3T3 cells, CHO cells, HeLa cells, Ltk⁻ cells and COS cells. Mammalian cells may be transfected by methods well known in the art such as calcium phosphate precipitation, electroporation and microinjection.

Disclosed is an isolated nucleic acid molecule comprising at least 15 contiguous nucleotides capable of specifically hybridizing with a unique sequence included within the sequence of the isolated nucleic acid molecule encoding IRTA4 protein, or fragment(s) thereof, having the amino acid sequence set forth in Figures 18D-1-18D-2_{•} In other aspects, the isolated nucleic acid molecules are labeled with a detectable marker. In still other embodiments of the isolated nucleic acid molecules, the detectable marker is selected from the group consisting of a radioactive isotope, enzyme, dye, biotin, a fluorescent label or a chemiluminescent label.

Disclosed is a method for detecting a B cell malignancy or a type of B cell malignancy in a sample from a subject wherein the B cell malignancy comprises a lq21 chromosomal rearrangement which comprises:
a) contacting the RNA sample obtained from the subject with a nucleic acid molecule of at least 15 contiguous nucleotides capable of specifically hybridizing with a unique sequence included within the sequence of an isolated RNA encoding human IRTA4 protein under conditions permitting hybridization of the RNA of step a) with the nucleic acid molecule capable of specifically hybridizing with a unique sequence included within the sequence of an' isolated RNA encoding human IRTA protein, wherein the nucleic acid molecule is labeled with a detectable marker; and b) detecting any hybridization in step a), wherein detecion of hybridization indicates presence of B cell malignancy or a type of B cell malignancy in the sample.

Detection of hybridization of RNA encoding IRTA proteins will indicate that a malignancy is a B cell malignancy. More specifically, detection of hybridization of RNA encoding IRTA protein indicates that the B cell malignancy is a Mucosa-Associated-Lymphoid Tissue (MALT) B cell lymphoma. Detection of hybridization of RNA encoding IRTA4 and IRTA5 proteins indicate that the B cell malignancy is a mantle cell lymphoma. In an embodiment of the above-described method, the B cell malignancy comprises a lq21 chromosomal rearrangement. One of skill will use the above-described method as a diagnostic aid in conjunction with other standard methods of detecting/diagnosing malignancies, e.g. pathology of a tumor sample, which may indicate lymphoma and the above-described method will then narrow the malignancy to a B cell lymphoma or more specifically to MALT) B cell lymphoma or a mantle cell lymphoma as discussed supra.

One of skill is familiar with known methods of detecting of hybridization nucleic acid molecules to nucleic acid oligonucleotides, i.e. nucleic acid probes encoding a protein of interest for diagnostic methods. The nucleic acid molecules encoding the IRTA proteins of the subject invention are useful for detecting B cell malignancy. One of skill will recognize that variations of the above-described method for detecting a B cell malignancy in a sample include, but are not limited to, digesting nucleic acid from' the sample with restrictio enzymes and separating the nucleic acid molecule fragments so oobtained by size fractionation before hybridization.

In an aspect of the above-described method for detecting a B cell malignancy in a sample from a subject, wherein the detectable marker is radioactive isotope, enzyme, dye, biotin, a fluorescent label or a chemiluminescent label. In a preferred embodiment as defined in the claims the B cell malignancy is selected from the group consisting of B cell lymphoma, multiple myeloma, Burkitt's lymphoma, marginal zone lymphoma, diffuse large cell lymphoma and follicular lymphoma cells. In a further embodiment, the B cell lymphoma is Mucosa-Associated-Lymphoid Tissue B cell lymphoma (MALT). In another preferred embodiment, the B cell lymphoma is non-Hodgkin's lymphoma.

Also disclosed is an antisense oligonucleotide having a sequence capable of specifically hybridizing to an mRNA molecule encoding human IRTA4 protein so as to prevent overexpression of the mRNA molecule.

In further aspects, of any of the above-described oligonucleotides of nucleic acid molecules encoding the IRTA4 protein the nucleic acid may be genomic DNA or cDNA.

One of skill is familiar with conventional techniques for nucleic acid hybridization of oligonucleotides, e.g. Ausubel, F.M. et al. Current Protocols in Molecular Biology, (John Wiley & Sons, New York, 1998), for example stringent conditions of 65°C in the presence of an elevated salt concentration. Such conditions are used for completely complementary nucleic acid hybridization, whereas conditions that are not stringent are used for hybridization of nucleic acids which are not totally complementary.

As used herein, the phrase "specifically hybridizing" means the ability of a nucleic acid molecule to recognize a nucleic acid sequence complementary to its own and to form double-helical segments through hydrogen bonding between complementary base pairs. As used herein, a "unique sequence" is a sequence specific to only the nucleic acid molecules encoding the IRTA4 protein. Nucleic acid probe technology is well known to those skilled in the art who will readily appreciate that such probes may vary greatly in length and may be labeled with a detectable label, such as a radioisotope or fluorescent dye, to facilitate detection of the probe. Detection of nucleic acid molecules encoding the IRTA4 protein is useful as a diagnostic test for any disease process in which levels of expression of the corresponding IRTA4 protein is altered. DNA probe molecules are produced by insertion of a DNA molecule which encodes mammalian IRTA4 protein or fragments thereof into suitable vectors, such as plasmids or bacteriophages, followed by insertion into suitable bacterial host cells and replication and harvesting of the DNA probes, all using methods well known in the art. For example, the DNA may be extracted from a cell lysate using phenol and ethanol, digested with restriction enzymes corresponding to the insertion sites of the DNA into the vector (discussed herein), electrophoresed, and cut out of the resulting gel. The oligonucleotide probes are useful for 'in situ' hybridization or in order to locate tissues which express this IRTA gene family, and for other hybridization assays for the presence of these genes (nucleic acid molecules encoding the IRTA4 protein) or their mRNA in various biological tissues. In addition, synthesized oligonucleotides (produced by a DNA synthesizer) complementary to the sequence of a DNA molecule which encodes IRTA4 protein are useful as probes for these genes, for their associated mRNA, or for the isolation of related genes by homology screening of genomic or cDNA libraries, or by the use of amplification techniques such as the Polymerase Chain Reaction.

This invention provides a purified IRTA4 protein comprising the amino acid sequence set forth in Figures 18D-1-18D-2 (SEQ ID NO: 7). In an embodiment the IRTA4 protein is human IRTA4.

In order to facilitate an understanding of the Experimental Details section which follows, certain frequently occurring methods and/or terms are best described in Sambrook, et al. (1989) and Harlow & Lane, Antibodies: A Laboratory Manual, Cold Spring Harbor Laboratories, Cold Spring Harbor, NY: 1988.

This invention provides an antibody/antibodies directed to an epitope of a purified IRTA4 protein, or fragment(s) thereof, having the amino acid sequence set forth in Figures 18D-1-18D-2.

As used herein, the term "antibody." includes, but is not limited to, both naturally occurring and non-naturally occurring antibodies. Specifically, the term "antibody" includes polyclonal and monoclonal antibodies, and binding fragments thereof. Furthermore, the term "antibody" includes chimeric antibodies and wholly synthetic antibodies, and fragments thereof. The polyclonal and monoclonal antibodies may be "purified" which means the polyclonal and monoclonal antibodies are free of any other antibodies. As used herein, partially purified antibody means an antibody composition which comprises antibodies which specifically bind to the IRTA protein of the subject invention, and consists of fewer protein impurities than does the serum from which the antibodies are derived. A protein impurity is a protein other than the antibodies specific for the IRTA protein of the subject invention. For example, the partially purified antibodies may be an IgG preparation.

Polyclonal antibodies (anti-IRTA antibodies) may be produced by injecting a host animal such as rabbit, rat, goat, mouse or other animal with the immunogen(s) of this invention, e.g. a purified human IRTA4 , described infra. The sera are extracted from the host animal and are screened to obtain polyclonal antibodies which are specific to the immunogen. Methods of screening for polyclonal antibodies are well known to those of ordinary skill in the art such as those disclosed in Harlow & Lane, Antibodies: A Laboratory Manual, (Cold Spring Harbor Laboratories, Cold Spring Harbor, NY: 1988) The anti-IRTA monoclonal antibodies of the subject invention may be produced by immunizing for example, mice with an immunogen (the IRTA polypeptide or fragments thereof as described herein). The mice are inoculated intraperitoneally with an immunogenic amount of the above-described immunogen and then boosted with similar amounts of the immunogen. Spleens are collected from the immunized mice a few days after the final boost and a cell suspension is prepared from the spleens for use in the fusion.

Hybridomas may be prepared from the splenocytes and a murine tumor partner using the general somatic cell hybridization technique of Kohler, B. and Milstein, C., Nature (1975) 256: 495-497. Available murine myeloma lines, such as those from the American Type Culture Collection (ATCC), 10801 University Boulevard, Manassas, VA 20110-2209, USA, may be used in the hybridization. Basically, the technique involves fusing the tumor cells and splenocytes using a fusogen such as polyethylene glycol. After the fusion the cells are separated from the fusion medium and grown in a selective growth medium, such as HAT medium, to eliminate unhybridized parent cells. The hybridomas may be expanded, if desired, and supernatants may be assayed by conventional immunoassay procedures, for example radioimmunoassay, using the immunizing agent as antigen. Positive clones may be characterized further to determine whether they meet the criteria of the invention antibodies.

Hybridomas that produce such antibodies may be grown in vitro or in vivo using known procedures. The monoclonal antibodies may be isolated from the culture media or body fluids, as the case may be, by conventional immunoglobulin purification procedures such as ammonium sulfate precipitation, gel electrophoresis, dialysis, chromatography, and ultrafiltration, if desired.

In the practice of the subject invention any of the above-described antibodies may be labeled with a detectable marker. In one embodiments, the labeled antibody is a purified labeled antibody. The term "antibody" includes, by way of example, both naturally occurring and non-naturally occurring antibodies. Specifically, the term "antibody" includes polyclonal and monoclonal antibodies, and fragments thereof. Furthermore, the term "antibody" includes chimeric antibodies and wholly synthetic antibodies, and fragments thereof. A "detectable moiety" which functions as detectable labels are well known to those of ordinary skill in the art and include, but are not limited to, a fluorescent label, a radioactive atom, a paramagnetic ion, biotin, a chemiluminescent label or a label which may be detected through a secondary enzymatic or binding step. The secondary enzymatic or binding step may comprise the use of digoxigenin, alkaline phosphatase, horseradish peroxidase, ß-galactosidase, fluorescein or steptavidin/biotin. Methods of labeling antibodies are well known in the art.

Methods of recovering serum from a subject are well known to those skilled in the art. Methods of partially purifying antibodies are also well known to those skilled in the art, and include, by way of example, filtration, ion exchange chromatography, and precipitation.

The polyclonal and monoclonal antibodies of the invention may be labeled with a detectable marker. In one embodiment, the labeled antibody is a purified labeled antibody. The detectable marker may be, for example, a radioactive or fluorescent marker. Methods of labeling antibodies are well known in the art.

Determining whether the polyclonal and monoclonal antibodies of the subject invention bind to cells, e.g. cancer cells, expressing an IRTA protein and form a complex with one or more of the IRTA protein(s) described herein, or fragments thereof, on the surface of said cells, may be accomplished according to methods well known to those skilled in the art. In the preferred embodiment, the determining is accomplished according to flow cytometry methods.

The antibodies of the subject invention may be bound to an insoluble matrix such as that used in affinity chromatography. Cells which form a complex, i.e. bind, with the immobilized polyclonal or monoclonal antibody may be isolated by standard methods well known to those skilled in the art. For example, isolation may comprise affinity chromatography using immobilized antibody.

Alternatively, the antibody may be a free antibody. In this case, isolation may comprise cell sorting using free, labeled primary or secondary antibodies. Such cell sorting methods are standard and are well known to those skilled in the art.

This invention provides an antibody directed to purified IRTA4 protein.

In a preferred embodiment of the anti-IRTA antibody the IRTA protein is human IRTA protein. The IRTA protein may be any mammalian IRTA protein, including a murine IRTA protein. In a further embodiment of any the above-described antibodies, the antibody is a monoclonal antibody. In another embodiment, the monoclonal antibody is a murine monoclonal antibody or a humanized monoclonal antibody. As used herein, "humanized" means an antibody having characteristics of a human antibody, such antibody being non-naturally occurring, but created using hybridoma techniques wherein the antibody is of human origin except for the antigen determinant portion, which is murine. In yet another embodiment, the antibody is a polyoclonal antibody.

In preferred embodiments, any of the antibodies of the subject invention may be conjugated to a therapeutic agent. In further preferred embodiments, the therapeutic agent is a radioisotope, toxin, toxoid, or chemotherapeutic agent. The conjugated antibodies of the subject invention may be administered to a subject having a B cell cancer in any of the methods provided below.

This invention provides a pharmaceutical composition comprising an amount of the antibody directed to IRTA4 protein effective to bind to cancer cells expressing IRTA4 protein so as to prevent growth of the cancer cells and a pharmaceutically acceptable carrier. The anti-IRTA antibody may be directed to an epitope of IRTA4 protein. The IRTA protein may be a human or mouse IRTA protein.

In preferred embodiments of the above-described pharmaceutical composition, the cancer cells are selected from the group consisting of B cell lymphoma, multiple myeloma, a mantle cell lymphoma, Burkitt's lymphoma, marginal zone lymphoma, diffuse large cell lymphoma and follicular lymphoma cells. In another preferred embodiment of the pharmaceutical composition, the B cell lymphoma cells are Mucosa-Associated-Lymphoid Tissue B cell lymphoma (MALT) cells. In another referred embodiment of the pharmaceutical composition, the B cell lymphoma cells are non-Hodgkin's lymphoma cells.

Disclosed is a pharmaceutical composition comprising an amount of any of the above-described oligonucleotides effective to prevent overexpression of a human IRTA protein and a pharmaceutically acceptable carrier capable. In an aspect of the pharmaceutical composition the oligonucleotide is a nucleic acid molecule which encodes IRTA4 protein,

The IRTA protein may be a human or mouse IRTA protein.

As used herein, "malignant" means capable of metastasizing. As used herein, "tumor cells" are cells which originate from a tumor, i.e., from a new growth of different or abnormal tissue. The tumor cells and cancer cells may exist as part of the tumor mass, or may exist as free-floating cells detached from the tumor mass from which they originate.

As used herein, malignant cells include, but are in no way limited to, B cell lymphoma, multiple myeloma, Burkitt's lymphoma, mantle cell lymphoma, marginal zone lymphoma, diffuse large cell lymphoma and follicular lymphoma. The B cell lymphoma is Mucosa-Associated-Lymphoid Tissue B cell lymphoma (MALT) or is non-Hodgkin's lymphoma.

As used herein, "subject" is any animal or artificially modified animal. Artificially modified animals include, but are not limited to, SCID mice with human immune systems. In a preferred embodiment, the subject is a human.

This invention provides a method of diagnosing B cell malignancy which comprises a lq21 chromosomal rearrangement in a sample from a subject which comprises:
a) contacting the sample obtained from the subject with an antibody directed to purified IRTA4 protein capable of specifically binding with human IRTA4 protein on a cell surface of a cancer cell under conditions permitting binding of the antibody with human IRTA4 protein on the cell surface of the cancer cell, wherein the antibody is labeled with a detectable marker; and b) detecting any binding in step a), wherein detecion of binding indicates a diagnosis of B cell malignancy in the sample.

In an embodiment of the above-described method of diagnosing B cell malignancy, the IRTA protein is IRTA4.

In another embodiment of the method the IRTA protein is human or mouse IRTA protein. In a further embodiment IRTA protein is purified. In a preferred embodiment of this method, the B cell malignancy is selected from the group consisting of B cell lymphoma, multiple myeloma, Burkitt's lymphoma, marginal zone lymphoma, diffuse large cell lymphoma and follicular lymphoma. In yet another embodiment of this method, the B cell lymphoma is Mucosa-Associated-Lymphoid. Tissue B cell lymphoma (MALT). In another preferred embodiment of this method, the B cell lymphoma is non-Hodgkin's lymphoma.

Disclosed is a method of detecting human IRTA protein in a sample which comprises: a) contacting the sample with any of any of the above-described anti-IRTA antibodies under conditions permitting the formation of a complex between the antibody and the IRTA in the sample; and b) detecting the complex formed in step (a), thereby detecting the presence of human IRTA in the sample. In an embodiment the IRTA protein detected may be IRTA4 having an amino acid sequence set forth in Figures 18D-1-18D-2. As described hereinabove detection of the complex formed may be achieved by using antibody labeled with a detectable marker and determining presence of labeled complex.

Detecting human IRTA protein in a sample from a subject is another method of diagnosing B cell malignancy in a subject. In an embodiment of this method of diagnosis, the B cell malignancy is selected from the group consisting of B cell lymphoma, multiple myeloma, Burkitt's lymphoma, marginal zone lymphoma, diffuse large cell lymphoma and follicular lymphoma. In yet another embodiment of this method, the B cell lymphoma is Mucosa-Associated-Lymphoid Tissue B cell lymphoma (MALT). In another preferred embodiment of this method, the B cell lymphoma is non-Hodgkin's lymphoma.

This invention provides a use for treating a subject having a B cell cancer which comprises administering to the subject an amount of anti-IRTA antibody effective to bind to cancer cells expressing an IRTA protein so as to prevent growth of the cancer cells and a pharmaceutically acceptable carrier, thereby treating the subject. Growth and proliferation of the cancer cells is thereby inhibited amd the cancer cells die. In an embodiment of the above-described use, the IRTA protein is human IRTA4.

In a preferred embodiment of the above-described use for treating a subject having a B cell cancer, the anti-IRTA antibody is a monoclonal antibody. In another embodiment of the method, the monoclonal antibody is a murine monoclonal antibody or a humanized monoclonal antibody. The antibody may be a chimeric antibody. In a further embodiment, the anti-IRTA antibody is a polyoclonal antibody. In an embodiment, the polyclonal antibody may be a murine or human polyclonal antibody. In a preferred embodiment, the B cell cancer is selected from the group consisting of B cell lymphoma, multiple myeloma, Burkitt's lymphoma, mantle cell lymphoma marginal zone lymphoma, diffuse large cell lymphoma and follicular lymphoma. In another preferred embodiment, the B cell lymphoma is Mucosa-Associated-Lymphoid Tissue B cell lymphoma (MALT). In a further preferred embodiment, the B cell lymphoma is non-Hodgkin's lymphomas. In a preferred embodiment of the above-described method of treating a subject having a B cell cancer, administration of the amount of anti-IRTA antibody effective to bind to cancer cells expressing an IRTA protein is intravenous, intraperitoneal, intrathecal, intralymphatical, intramuscular, intralesional, parenteral, epidural, subcutaneous; by infusion, liposome-mediated delivery, aerosol delivery; topical, oral, nasal, anal, ocular or otic delivery. In another preferred embodiment of the above-described methods, the anti-IRTA antibody may be conjugated to a therapeutic agent. In further preferred embodiments, the therapeutic agent is a radioisotope, toxin, toxoid, or chemotherapeutic agent.

-- Disclosed is a use for treating a subject having a B cell cancer which comprises administering to the subject an amount of an antisense oligonucleotide having a sequence capable of specifically hybridizing to an mRNA molecule encoding a human ITRA protein so as to prevent overexpression of the human IRTA protein, so as to arrest cell growth or induce cell death of cancer cells expressing IRTA protein(s) and a pharmaceutically acceptable carrier, thereby treating the subject.

In an embodiment of the above-described use for treating a subject having a B cell cancer, the IRTA protein is human IRTA4 protein. In a preferred embodiment, B cell cancer is selected from the group consisting of B cell lymphoma, multiple myeloma, Burkitt's lymphoma, marginal zone lymphoma, diffuse large cell lymphoma and follicular lymphoma. In another preferred embodiment, the B cell lymphoma is Mucosa-Associated-Lymphoid Tissue B cell lymphoma (MALT). In a yet another preferred embodiment, the B cell lymphoma is non-Hodgkin's lymphoma. In embodiments of any of the above-described oligonucleotides of nucleic acid molecules encoding the IRTA4 protein, the nucleic acid may be genomic DNA or cDNA. In a further preferred embodiment of the above-described method of treating a subject having a B cell cancer, administration of the amount of oligonucleotide of effective to prevent overexpression of a human IRTA protein is intravenous, intraperitoneal, intrathecal, intralymphatical, intramuscular, intralesional, parenteral, epidural, subcutaneous; by infusion, liposome-mediated delivery, aerosol delivery; topical, oral, nasal, anal, ocular or otic delivery. In an embodiment of the above-described methods, the oligonucleotide may be conjugated to a therapeutic agent. In further preferred embodiments, the therapeutic agent is a radioisotope, toxin, toxoid, or chemotherapeutic agent.

The invention also provides a pharmaceutical composition comprising either an effective amount of the oligonucleotides or of the antibodies described above and a pharmaceutically acceptable carrier. In the subject invention an "effective amount" is any amount of an oligonucleotide or an antibody which, when administered to a subject suffering from a disease or abnormality against which the oligonucleotide or antibody are effective, causes reduction, remission, or regression of the disease or abnormality. In the practice of this invention the "pharmaceutically acceptable carrier" is any physiological carrier known to those of ordinary skill in the art useful in formulating pharmaceutical compositions.

Pharmaceutically acceptable carriers are well known to those skilled in the art and include, but are not limited to, 0.01-0.1M and preferably 0.05M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, chelating agents, inert gases and the like.

In one preferred embodiment the pharmaceutical carrier may be a liquid and the pharmaceutical composition would be in the form of a solution. In another equally preferred embodiment, the pharmaceutically acceptable carrier is a solid and the composition is in the form of a powder or tablet. In a further embodiment, the pharmaceutical carrier is a gel and the composition is in the form of a suppository or cream. In a further embodiment the compound may be formulated as a part of a pharmaceutically acceptable transdermal patch.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellent.

Liquid pharmaceutical compositions which are sterile solutions or suspensions can be utilized by for example, intramuscular, intrathecal, epidural, intraperitoneal or subcutaneous injection. Sterile solutions can also be administered intravenously. The compounds may be prepared as a sterile solid composition which may be dissolved or suspended at the time of administration using sterile water, saline, or other appropriate sterile injectable medium. Carriers are intended to include necessary and inert binders, suspending agents, lubricants, flavorants, sweeteners, preservatives, dyes, and coatings.

The pharmaceutical composition comprising the oligonucleotide or the antibody can be administered orally in the form of a sterile solution or suspension containing other solutes or suspending agents, for example, enough saline or glucose to make the solution isotonic, bile salts, acacia, gelatin, sorbitan monoleate, polysorbate 80 (oleate esters of sorbitol and its anhydrides copolymerized with ethylene oxide) and the like.

The pharmaceutical composition comprising the oligonucleotide or the antibody can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

Optimal dosages to be administered may be determined by those skilled in the art, and will vary with the particular inhibitor in use, the strength of the preparation, the mode of administration, and the advancement of the disease condition or abnormality. Additional factors depending on the particular subject being treated will result in a need to adjust dosages, including subject age, weight, gender, diet, and time of administration.

This invention will be better understood from the Experimental Details which follow. However, one skilled in the art will readily appreciate that the specific methods and results discussed are merely illustrative of the invention as described more fully in the claims which follow thereafter.

### EXPERIMENTAL DETAILS

### First Series of Experiments

Molecular analysis of chromosomal translocations associated with multiple myeloma (MM) has indicated that the pathogenesis of this malignancy may be heterogeneous, being associated with several distinct oncogenes including BCL-1, MUM-1 and FGFR3. Structural abnormalities of chromosome 1q21, including translocation with chromosome 14q32, represent frequent cytogenetic aberrations associated with multiple myeloma. In order to identify the genes involved in these translocations, the breakpoint regions corresponding to both derivatives of a t(1;14) (q21;q32) detectable in the FR4 human plasmacytoma cell line were cloned. Analysis of the breakpoint sequences showed that they involved a reciprocal recombination between the Immunoglobulin heavy chain (IgH) locus on 14q32 and unknown sequences on 1q21. The normal locus corresponding ot hte 1q21 region involved in the translocation was cloned and athe genes adjacent to the breakpoint region were identified by an exon-trapping strategy. Two genes were found, located within a 20 Kb distance from each other, in the region spanning the breakpoint on 1q21. The first gene, called MUM-2 (multiple myeloma-2) is expressed as a 2.5 Kb mRNA transcript detectable in spleen and lymph nodes. Cloning and sequencing of the full-length MUM-2cDNA predicts a 515 amino acid cell surface glycoprotein containing four extracellular Ig-type domains, a transmembrane and a cytoplasmic domain and sharing a 37% identity (51%-homology) with Fc gamma receptor I over its first three extracellular domains. In FR4 cells, the translocation breakpoints interrupt the MUM-2 coding domain and juxtapose it to the IgH locus in the same transcriptional orientation. As a consequence, structurally abnormal FR4-specific MUM-2 transcripts (3.0, 5.2 and 6.0 Kb) in lymph nodes and spleen and encodes a protein with an extracellular domain containing six Ig-type domains homologous to members of the Fc gamma and Ig-type adhesion receptor families. The structure of the MUM-2 and MUM-3 genes and their direct involvement in a MM-associated translocation suggest that these genes code for novel cell surface receptors important for normal lymphocyte function and B cell malignancy.

### Second Series of Experiments

### Experimental Procedures

### Cell Lines

The MM cell lines used in this study (FR4, U266, JJN3, EJM, SKMM1, RPMI-8226, XG1, XG2, XG4, XG6, XG7) have been previously reported (Tagawa et al., 1990), (Jernberg et al., 1987), (Hamilton et al., 1990; Jackson et al., 1989), (Eton et al., 1989), (Zhang et al., 1994). The FR4 cell line was established in the laboratory of one of the authors (S.T). The U266, JJN3, and EJM cell lines were gifts from Dr. K. Nilsson (University of Uppsala, Uppsala, Sweden) and the SKMM-1 cell line was a gift of A.N. Houghton (Memorial Sloan Kettering Cancer Center, New York, NY). The five XG cell lines were obtained from Dr. Bernard Klein and cultured in the presence of 1 ng/ml human recombinant IL-6 as described previously (Zhang et al., 1994). The BL cell lines with lq21 abnormalities have been previously described (Polito et al., 1995), (Magrath et al., 1980) and were grown in RPMI, 10% FCS.

### Genomic and cDNA library screening and DNA sequence analysis

Two genomic libraries were constructed from FR4 genomic DNA either by BamHI complete digestion or by Sau3AI partial digestion and subsequent ligation of gel-purified fractions into the 1DASH-II phage vector (Stratagene). The BamHI library was screened with a 4.2 kb XhoI-BamHI probe derived from the Ca locus and the Sau3AI library was screened with a 5'Sa probe previously described (Bergsagel et al., 1996). A human placental DNA library (Stratagene) was screened with probe 1. OEH (Figures 8A-8C) to obtain the germline 1q21 locus. Library screening and plaque isolation were preformed according to established procedures (Sambrook et al., 1989). *IRTA1* and *IRTA2* cDNA clones were isolated from an oligo-dT/random-primed cDNA library constructed from normal human spleen RNA (Clontech). The *IRTA1* cDNA probe used for library screening was obtained from RT-PCR of human spleen cDNA using primers flanking exons 1 and 3. DNA sequencing was preformed on an ABI 373 automated sequencer (Applied Biosystems) . Sequence homology searches were carried out through the BLAST e-mail server at the National Center for Biotechnology Information, Bethesda, MD.

### PAC and YAC isolation and exon trapping

Human PAC clones were obtained by screening a human PAC library spotted onto nylon membranes (Research Genetics), with the 1.0 EH probe (Figures 8A-8C). The Zeneca (formerly ICI) human YAC library (Anand et al., 1990) obtained from the United Kingdom Human Genome Mapping Resource Center (UK-HGMP) was screened using a PCR-based pooling strategy. Exon trapping was performed using the exon trapping system (Gibco BRL), according to the manufacturer's instructions.

### Isolation of PAC/YAC end clones, pulsed-field gel electrophoresis (PFGE) and fluorescence in situ hybridization (FISH) analysis

PAC DNA extraction was performed according to standard alkaline lysis methods (Drakopoli N et al., 1996). A vectorette-PCR method was used to isolate PAC and YAC end probes (Riley et al., 1990), as previously described (Iida et al, 1996) . PFGE analysis was performed according to standard protocols (Drakopoli N et al., 1996) using the CHEF Mapper system (BioRad, Hercules, CA). Biotin labeling of PAC DNA, chromosome preparation and FISH were performed as previously described (Rao et al., 1993).

### Southern and Northern blot analyses, RACE and RT-PCR

Southern and northern blot analyses were performed as described previously (Neri et al, 1991) . For Northern blot analyses total RNA was prepared by the guanidium thiocyanate method and poly(A) RNA was selected using poly(T)-coated beads (Oligotex Kit by Quigen). For Northern blots, 2 mg of poly(A) RNA were loaded per lane. Multiple tissue Northern filters were obtained from Clontech. RACE was performed using the Marathon cDNA Amplification kit (Clontech) and Marathon-Ready spleen cDNA. First strand cDNA synthesis was performed using the Superscript RT-PCR system (Gibco BRL)

### In situ hybridization

Digoxigenin-containing antisense and sense cRNA probes were transcribed with T3 and T7 RNA polymerase, respectively, from linearized pBluescript KS+ plasmids containing coding region of cDNAs (nucleotides 62 to 1681 of IRTA1 and 18 to 2996 of IRTA2.) Hyperplastic human tonsillar tissue surgically resected from children in Babies Hospital, Columbia Presbyterian Medical Center was snap frozen in powdered dry ice. Cryostat sections were stored for several days at -80 degrees C prior to processing. Non-radioactive *in situ* hybridization was performed essentially as described (Frank et al., 1999), except that fixation time in 4% paraformaldehyde was increased to 20 minutes, and proteinase K treatment was omitted. The stringency of hybridization was 68 degrees C, in 5X SSC, 50% formamide. Alkaline phosphatase-conjugated anti-digoxigenin antibody staining was developed with BCIP/NBT substrate.

### Transfection, immunoprecipitation and Western Blotting

293 cells (ATCC), grown in DMEM, 10% FCS were transiently transfected, according to the standard calcium phosphate method, with pMT2T and pMT2T-IRTAl/Ca transient expression constructs. The latter was generated using the *IRTA1*/*Ca* RT-PCR product from FR4. Cells (2x10⁶ of transfectants and 2x10⁷ of remaining cell lines) were solubilized in Triton X-100 lysis buffer (150 mM NaCl, 10 mM Tris-HCl [pH 7.4], 1% Tx-100, 0.1% BSA) in the presence of a protease inhibitors coctail (Roche Biochemicals).

Lysates were incubated at 4°C for 2 hours with 4 mg/ml of the monoclonal antibody #117-332-1 (Yu et al., 1990) (Tanox Biosystems, Inc, Houston, Texas) that was raised against the extracellular portion of the IgA membrane peptide. Immune complexes were isolated with protein G-Sepharose (Pharmacia) prior to electrophoresis on 10-20% Tris-HCl gradient gels (Biorad) and immunobloting, using 15 mg/ml of the #117-332-1 antibody. Results were visualized by ECL (Amersham).

### RESULTS

### Molecular Cloning of the t(1;14) (q21;q32)

Chromosomal translocations involving the Ig heavy-chain (IGH) locus often occur within or near IgH switch regions as a result of "illegitimate" switch recombination events (Dalla-Favera et al., 1983 ; Chesi et al., 1996; Chesi et al., 1998) . The breakpoints can be detected by Southernblot hybridization assays as rearranged alleles in which the IGH constant (C_{H}) region sequences have lost their syntenic association with IGH joining (J_{H}) and 5' switch region (S) sequences (Dalla-Favera et al., 1983; Neri et al., 1988; Neri et al., 1991; Bergsagel et al., 1996). This assay has led to the identification of several chromosomal partners for the IgH locus in B-NHL and MM (Taub et al., 1982; Dalla-Favera et al., 1983; Neri et al., 1988; Neri et al. , 1991; Ye et al., 1993 ; Chesi et al., 1996; Richelda et al., 1997; Iida -et al., 1997; Dyomin et al., 1997; Dyomin et al., 2000) . We employed the same strategy in order to clone the 1q21 breakpoint region in FR4, a myeloma cell line carrying a t(1;14) (q21;q32), as determined by cytogenetic analysis (Tagawa et al., 1990; Taniwaki M, unpublished results). Two "illegitimately" rearranged fragments were identified within the Cα heavy-chain locus in FR4 by Southern blot hybridization analysis (data not shown), and were cloned from phage libraries constructed from FR4 genomic DNA. Restriction mapping, Southern blot hybridization and partial nucleotide sequencing of two genomic phages (clones λ FR4B-5 and λ FR4S-a, Figure 8A) demonstrated that they contained the Chromosomal breakpoints of a reciprocal balanced translocation between the *Cα*₁ locus on 14q32 and non-IGH sequences. A probe (1.0EH) representing these non-IgH sequences (Figure 8A) was then used to clone the corresponding normal genomic locus from phage, Pl artificial chromosome (PAC), and yeast artificial chromosome (YAC) human genomic libraries. Fluorescence in situ hybridization (FISH) analysis of normal human metaphase spreads using the 100-kb non-chimaeric PAC clone 49A16 which spans the breakpoint region (see below, Figure 13), identified the partner chromosomal locus as derived from band 1q21 (Figure 8C) . Mapping to a single locus within chromosome 1 was confirmed by hybridization of two non-repetitive probes to DNA from a somatic-cell hybrid panel representative of individual human chromosomes (data not shown) . These results were consistent with the cloning of sequences spanning the t(1;14) (q21;q32) in FR4.

Sequence analysis of the breakpoint regions on the derivative chromosomes and alignment with the germline 14q32 and lq21 loci revealed that the breakpoint had occurred in the intron between the CH3 and the transmembrane exon of Cα₁ on chromosome 14. Although the breakpoint region was devoid of recombination signal sequences (RSS) or switch signal sequences (Kuppers et al., 1999), the sequence CTTAAC (underlined on Figure 8B) was present in both germline chromosomes 14 and 1 at the breakpoint junction. One copy of this sequence was present in each of the derivative chromosomes, with a slight modification in the der(1) copy (point mutation in the last nucleotide: C to G). The nucleotides AT preceding CTTAAC on chromosome 1 were also present in both derivative chromosomes (Figure 8B). The translocation did not result in any loss of chromosome 1 sequences. On the other hand, in the chromosome 14 portion of der(1) we observed two deletions upstream of the breakpoint junction: a 16 nucleotide deletion (GGCACCTCCCCTTAAC) and a 4 nucleotide deletion (TGCA) 6 nucleotides upstream (Figure 8B). These observations indicate that the t(1;14) (q21;q32) in FR4 cells represents a balanced reciprocal translocation possibly facilitated by the presence of homologous sequences (CTTAAC) on both chromosomes.

### The lq21 breakpoint region contains genes coding for novel members of the Immunoglobulin Receptor Superfamily

We next investigated whether the region of chromosome lq21 spanning the translocation breakpoint in FR4 contains a transcriptional unit. DNA from partially overlapping PAC clones 49A16 and 210K22 (Figure 13) was "shotgun" cloned in plasmids, sequenced and analyzed for homology to known genes in human genome databases. In parallel, candidate genes on the 49A16 PAC were sought by an exon trapping strategy (Church et al., 1994) .

Mapping of the candidate exons on the lq21 genomic clones revealed that the FR4 breakpoint had occurred between two trapped exons (see below, Figure 13), which belonged to the same transcript since they could be linked by RT-PCR using spleen RNA. This RT-PCR product was then used as a probe to screen a spleen cDNA library in order to isolate full-length clones corresponding to this transcript. Two sets of cDNA clones were identified, belonging to two distinct transcripts and sharing a 76% mRNA sequence identity within the 443 bp probe region. Full length cDNA clones for both transcripts were obtained by rapid amplification of cDNA ends (RACE) on human spleen cDNA that generated 5' and 3' extension products.

The schematic structure of the cDNA representing the first transcript is depicted in Figure 9A. Alternate usage of three potential polyadenylation sites in its 3' untranslated region gives rise to three mRNA species of 2.6, 2.7 and 3.5 kb, encoding the same putative 515-amino acid protein (Figure 9A) . The predicted features of this protein include a signal peptide, in accordance with the [-3, -1] rule (von Heijne, 1986), four extracellular Ig-type domains carrying three potential asparagine (N)-linked glycosylation sites (Figure 9A), a 16 amino acid transmembrane and a 106 amino acid cytoplasmic domain with three putative consensus Src-homology 2 (SH2)-binding domains (Unkeless and Jin, 1997) (Figure 10B). These (SH2)-binding domains exhibit features of both ITAM (Immune-receptor Tyrosine-based Activation Motif -D/EX₇D/EX₂YXXL/IX₆₋₈YXXL/I; where X denotes non-conserved residues) (Reth, 1989) and ITIM motifs (Immune-receptor Tyrosine-based Inhibition Motif - S/V/L/IYXXL/V where X denotes non-conserved residues) (Unkeless and Jin, 1997). As shown in Figure 10B, the first two SH2-binding domains are spaced 8 aminoacids apart, consistent with the consensus ITAM motif. Diverging from the consensus, the glutamate residue (E) is positioned four rather than two aminoacids before the first tyrosine (Y). (Figure 10B), and the +3 position relative to tyrosine (Y) is occupied by valine (V) rather than leucine (L) or isoleucine (I) (Cambier, 1995) . All three domains conform to the ITIM consensus and each is encoded by a separate exon, as is the case for ITIM. Thus their arrangement may give rise to three ITIM or possibly to one ITAM and one ITIM. The overall structure of this protein suggests that it represents a novel transmembrane receptor of the Ig superfamily and it was therefore name IRTA1 (Immune Receptor Translocation Associated gene 1).

The second cDNA shares homology to *IRTA1* (68% nucleotide identity for the length of the *IRTA1* message encoding its extracellular domain) and was named *IRTA2*. The *IRTA2* locus is more complex than *IRTA1* and is transcribed into three major mRNA isoforms (*IRTA2a*, *IRTA2b, IRTA2c)* of different molecular weight (2.8, 4.7 and 5.4 kb respectively), each with its own unique 3' untranslated region (Figure 9B). In addition, a 0.6 kb transcript (Figure 12A) arises from the usage of an early polyadenylation signal at nucleotide 536 of *IRTA2.* The three predicted IRTA2 protein isoforms encoded by these transcripts share a common aminoacid sequence until residue 560, featuring a common signal peptide and six extracellular Ig-type domains (Figure 9B). IRTA2a encodes for a 759 aa secreted glycoprotein with eight Ig-type domains followed by 13 unique, predominantly polar aminoacids at its C-terminus. IRTA2b diverges from IRTA2a at amino acid residue 560, and extends for a short stretch of 32 additional residues, whose hydrophobicity is compatible with its docking to the plasma membrane via a GPI-anchor (Ferguson and Williams, 1988). IRTA2c is the longest isoform whose sequence deviates from IRTA2a at aminoacid 746. It encodes a 977 aa type I transmembrane glycoprotein with nine extracellular Ig-type domains, harboring eight potential N-linked glycosylation sites, a 23 aminoacid transmembrane and a 104 aminoacid cytoplasmic domain with three consensus SH2-binding motifs (Figure 10B). Each of the SH2-binding sites in IRTA2c agrees with the ITIM consensus (Figure 10B) and is encoded by a separate exon. These features suggest that IRTA2c is a novel transmembrane receptor of the Ig superfamily with secreted and GPI-linked isoforms.

### Homology between the IRTA proteins and Immunoglobulin Superfamily Receptors

Amino acid alignment of the entire extracellular domains of the IRTA1 and IRTA2 proteins to each other and to other Ig superfamily members revealed a remarkable homology between them (47% identity and 51% similarity) and a lower, but striking homology to the Fc gamma receptor family of proteins. This homology was stronger in the aminoacid positions conserved among the different classes of Fc receptors. Among Fc receptors, the high affinity IgG receptor FCGRI (CD64) shared the highest levels of homology with the first three Ig-domains of IRTA1 and IRTA2 (37% identity and 50% similarity) throughout its entire extracellular portion (Figure 10A). Lower levels of homology were observed' between the IRTA proteins and the extracellular domains of other cell surface molecules, including human platelet endothelial cell adhesion molecule (PECAM1), B-lymphocyte cell adhesion molecule (CD22) and Biliary Glycoprotein 1 (BGP1) (22-25% identity, 38-41% homology).

No homology is apparent between the IRTAs and members of the Fc receptor family in their cytoplasmic domains. In contrast, significant aminoacid homology is present between IRTA1 and PECAM1 (31% aminoacid identity and 45% homology), IRTA2c and BGP1 (30% identity, 35% homology) and IRTA2c and PECAM1 (28% identity, 50% homology) (Figure 10B) . These homologies suggest employment of similar downstream signaling pathways by these different proteins.

### IRTA1 and IRTA2 are normally expressed in specific subpopulations of B cells

The normal expression pattern of the *IRTA1* and *IRTA2* mRNAs was first analyzed by Northern blot hybridization of RNA derived from different normal human tissues and from human cell lines representing different hematopoietic lineages and stages of B-cell development.

*IRTA1* expression was detected at a very low level in human spleen and lymph node RNA (Figure 11A, left panel) and was undetectable in all other human tissues analyzed, including fetal liver, bone marrow, lung, placenta, small intestine, kidney, liver, colon, skeletal muscle, heart and brain (data not shown). Among B cell lines, *IRTA1* expression was absent in cell lines representing pre-B and germinal center B-cells, plasma cells and cells of erythroid, T-cell and myeloid origin (data not shown, see Materials and Methods). Expression was detectable at very low levels only in EBV-immortalized lymphoblastoid cell lines (LCL), which represent a subpopulation (immunoblasts) positioned downstream of germinal center B cells in B-cell differentiation. However, expression was induced in estrogen-deprived ER/EB cells which, being immortalized by a recombinant EBV genome in which the EBNA2 gene is fused to the estrogen receptor, proliferate in the presence of estrogen while they arrest in the G₀/G₁ phase upon estrogen deprivation (Kempkes et al., 1995). *IRTA1* expression was barely detectable in these cells in the presence of estrogen, but was induced (10-fold) upon their G₀/G₁ arrest following estrogen withdrawal (Figure 11A, right panel). Taken together, these results suggest that *IRTA1* is expressed in a lymphoid subpopulation present in spleen and lymph nodes and presumably represented by resting B cells.

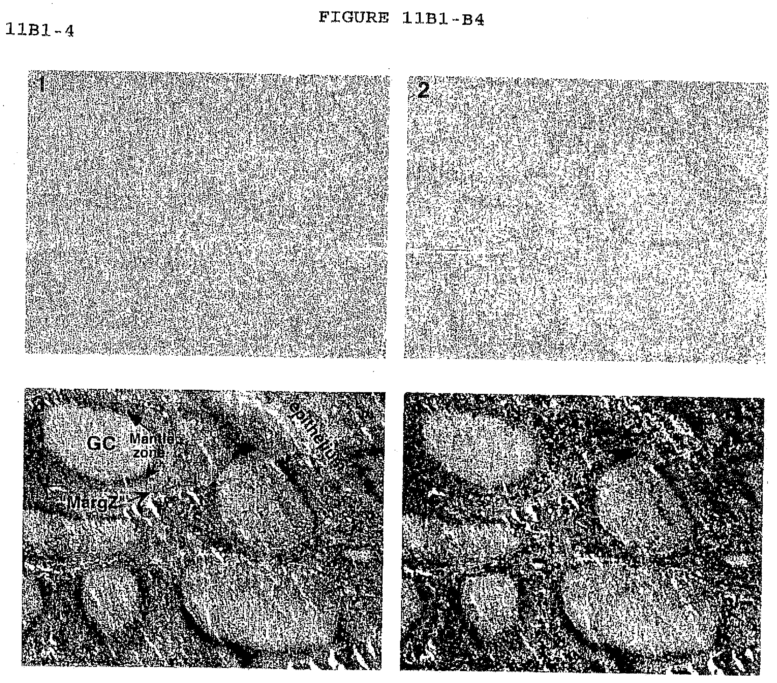

To further investigate the phenotype and tissue distribution of the cells expressing *IRTA1*, we performed in *situ* hybridization on human tonsillar tissue using a *IRTA1* antisense cDNA probe (Figure 11B). Serial sections were processed for in situ hybridization with a control sense cDNA probe (Panel # 1 in Figure 11B), an antisense cDNA probe (Panel # 2) and hematoxylin and eosin (H&E) staining (Panel # 3) to outline the architecture of the lymphoid tissue. The *IRTA1* hybridization signal was excluded from the germinal center and the mantle zone of the follicles and was characteristically concentrated in the perifollicular zone with infiltrations in the intraepithelial region (Figures 11B-2, 11B-4). In this region, only B cells were positive as documented by staining with B cell specific markers (IgD, not shown), and by immnunohistochemical analysis with anti-IRTA1 and anti-B (CD20, PAX5), anti-T (CD3), and anti-monocyte (CD68) antibodies (not shown; G. Cattoretti et al., manuscript in preparation). This perifollicular area is the "marginal zone" equivalent of the tonsil, representing a functionally distinct B-cell compartment that contains mostly memory B-cells and monocytoid B-cells (de Wolf-Peeters et al., 1997). Together with the Northern blot analysis of normal tissues and cell lines, these results indicate that *IRTA1* is expressed in a subpopulation of resting mature B-cells topographically located in the perifollicular and intraepithelial region, sites rich in memory B cells.

In the case of IRTA2, Northern blot analysis detected all alternatively spliced species in human lymph node, spleen, bone marrow and small intestine mRNA, with relative preponderance of the *IRTA2a* isoform (Figure 12A, left panel). Among the hematopoietic cell lines of lymphoid and non-lymphoid origin tested, *IRTA2* expression was restricted to B-cell lines with an immunoblastic, post-germinal center phenotype (Figure 12A, right panel). Similarly to *IRTA1,* it was absent from cell lines derived from pre-B cells, germinal center centroblasts, plasma cells, T-cells, erythroid cells and myeloid cells (Figure 12A, right panel).

*In situ* hybridization analysis of human tonsillar tissue, using the *IRTA2c* cDNA as a probe, was consistent with the results of the Northern blot analysis. The *IRTA2* mRNA was largely excluded from the mantle zone of the germinal center, with the exception of a few positive cells (Figures 12B-2, 12B4). Within the germinal center, the dark zone, represented by centroblasts, appeared negative for *IRTA2,* while the light zone, rich in centrocytes, was strongly positive (Figures 12B-2, 12B-4). Finally, *IRTA2* mRNA was detected in the "marginal zone" equivalent region outside germinal center follicles and in the intraepithelial and interfollicular regions of the tonsil. This pattern is consistent with specificity of *IRTA2* for centrocytes and post-germinal center B cells. Comparing their expression patterns, we conclude that both are specific for mature B cells, but IRTA2 has a broader pattern of expression that includes centrocytes and interfollicular B cells, while *IRTA1* is restricted to marginal zone B cells, most likely memory cells.

### Genomic organization of the IRTA1 and IRTA2 genes

To understand the consequences of lq21 abnormalities on *IRTA1* and *IRTA2* gene structure and expression, we first determined the organization of their genomic loci. The *IRTA1* gene contains 11 exons with a total genomic size of 24.5 kb (Figure 13). The *IRTA2* locus was found to span a genomic region of approximately 40 kb (Figure 13) . The three *IRTA2* alternatively spliced products share their first 8 exons, at which point *IRTA2b* does not utilize the next splicing site, and terminates by entering its 3'UTR region. *IRTA2a* and 2c isoforms splice into exon 9, with *IRTA2a* entering into its 3'UTR after exon 11 and *IRTA2c* splicing into exon 12 and extending until exon 18 (Figure 13) .

Based on sequencing data, we determined that the *IRTA1* and *IRTA2* genes are located 21 kb distant from each other, juxtaposed in the same transcriptional orientation (Figure 13) that extends from the telomere (5') towards the centromere (3'). At the lq21 locus, they are tightly linked to each other as well as to three additional genes we recently cloned through their homology to the *IRTAs* (I.M, manuscript in preparation). All five genes are contiguous, covering a ∼300 kb region at 1q21. This region is located at the interval between previously reported lq21 breakpoints. Based on the distance between genomic clones harboring the respective genes on the Whitehead Institute Radiation Hybrid map, the *IRTA1-2* locus is estimated to lie approximately 0.8 Mb away from the *MUC1* locus towards the telomere (N.P, unpublished data; Dyomin et al., 2000; Gilles et al., 2000) and less than or equal to 7 Mb away from the *FCGRIIB* locus towards the centromere (N.P, unpublished data).

### The t(1;14) (q21;q32) translocation generates an IRTA1/Ca₁ fusion protein in the FR4 myeloma cell line

Comparative restriction and nucleotide sequence analysis of germline versus rearranged sequences from the Ca₁ and *IRTA1* loci showed that the translocation had fused sequences within intron 2 of the *IRTA1* gene to the intronic sequences between the *CH3* and the transmembrane exon of Ca₁ in the same transcriptional orientation (Figure 14A) . This suggested that, if *IRTA1* sequences were expressed in the translocated locus, the intact donor site at the 3' border of the *IRTA1* exon and the intact acceptor site at the 5' of Ca₁ could be used to generate a fusion *IRTA1*/*Ca₁* mRNA, and possibly a IRTA1/Ca₁ fusion protein.

In order to test this prediction, we analyzed *IRTA1* mRNA expression in FR4 by Northern blot analysis using an *IRTA1* cDNA probe derived from exon 1 (Figure 14A). This probe detected a 0.8 kb message in FR4 that was absent from other B-cell lines, and was shorter than the normal 2.5 kb message detectable in ER/EB cells (Figure 14B). We cloned this transcript by RT-PCR of FR4 mRNA using primers derived from sequences at the 5' border of *IRTA1* exon 1 and the 3' border of the *Cα* cytoplasmic exon (Figure 14A). An RT-PCR product was obtained from FR4, but not from the DAKIKI cell line expressing wild-type surface IgA, or other cell lines lacking a t(1;14) translocation (data not shown). Direct sequencing analysis of the PCR product indicated that splicing had precisely linked *IRTA1* and *Ca₁* at canonical splicing sites and determined that the fusion transcript was 820 bp long.

Analysis of the predicted protein product indicated that the *IRTA1*/*Cα*₁ splicing had resulted in a fusion between the IRTA1 signal peptide and first two extracellular aminoacids, with the 32-amino acid long extracellular spacer, transmembrane domain and cytoplasmic tail of the membrane IgA₁ (mIgA₁) receptor (Figure 14C) . To assay for the expression of this fusion protein in FR4 protein extracts, we used an antibody directed against extracellular aminoacid residues specific for the transmembrane isoform of Cα₁ (Yu et al. , 1990) for immunoprecipitation, followed by Western blotting. Our results demonstrated that FR4 cells, but not a control cell line (DAKIKI) expressing wild-type surface IgA, express a 9.8kDa protein consistent with the predicted size of IRTA1/Cα₁ fusion protein (Figure 14D) . These results show that the translocated allele encodes a fusion protein, composed of the signal peptide and first two extracellular residues of IRTA1 (1-7 aminoacids) fused to the Cα₁ encoded transmembrane and cytoplasmic domains (71 aminoacids). In contrast to *IRTA1*/*Ca₁* overexpression on der (14), no expression was detected in FR4 for the reciprocal *Ca₁* /*IRTA1* transcript or for the intact IRTA2 gene on der (1) .

With the exception of FR4, *IRTA1* mRNA expression was not detected in any other myeloma or lymphoma cell line, regardless of the status of its chromosomal band 1q21 (data not shown). Thus, the IRTA1/Ca fusion represents a rare event in lq21 aberrations.

### Frequent deregulation of IRTA2 expression in cell lines carrying lq21 abnormalities

In order to establish the physical relationship between other lq21 breakpoints and the *IRTA1*/*2* locus, we performed FISH analysis with the PAC 49A16 on our panel of BL and MM cell lines. Among ten BL cell lines analyzed, seven with dup(1) (q21q32) and three with lq21 translocations (AS283A, BL104, BL136), we detected three signals corresponding to the *IRTA1*/*IRTA2* locus in seven of the former and two of the latter, consistent with dup(1) (q21q32) in the first case and dup(1) (q21q32) followed by a translocation breakpoint at lq21 in the second. (Table 1). FISH analysis of AS283A and BL136, using probes spanning the *IRTA* locus and with neighboring genomic clones, placed the breakpoint of the derivative chromosomes outside the *IRTA* locus in both cell lines, at a distance of >800 kb towards the centromere in AS283A and >800 kb towards the telomere in BL136 (N.P, unpublished results). Consistent with this finding, analysis of 30 cases of MM primary tumors by interphase FISH with the 300-kb YAC 23GC4 (Figure 13), showed that 15 cases (50% of total analyzed) had more than two interphase FISH signals (data not shown), while double color FISH with two PAC clones flanking the YAC centromeric and telomeric borders detected no split of these two probes in any of the cases. These results indicate that, with the exception of FR4, the breakpoints of lq21 aberrations in BL or MM are not within or in close proximity to the genomic region defined by *IRTA1* and *IRTA2.* However, the consistent outcome of either dup(1) (q21q32) (see Table 1) or dup(1) (q21q32) followed by unbalanced translocations (AS283A, BL136, XG2, XG7 in Table 1) is partial trisomy or tetrasomy of the region of lq21 containing the IRTA genes.

**Table 1. Summary of karyotypic and FISH data on IRTA1/IRTA2 locus**

| Tumour type | Cytogenetics | PAC 49A16 | Copy number of IRTA locus by FISH | IRTA2 mRNA expression |
|---|---|---|---|---|
| Burkitt Lymphoma | | | | |
| **AS283A** | der(4) t (1;4)(q21;q35) | der(4), normal 1 | 3 | ++++++ |
| **MC116** | duplq21 | duplq21 | 3 | +++ |
| **CA46** | duplq21 | duplq21 | 3 | +++ |
| **PA682** | duplq21 | duplq21 | 3 | ++ |
| **BrgIgA** | duplq21 | duplq21 | 3 | ++ |
| **BL32** | duplq21 | duplq21 | 3 | - |
| **BL92** | duplq21 | duplq21 | 3 | ++ |
| **BL103** | invduplq21 | duplq21 | 3 | + |
| **BL104** | t(1;3)(q21;p25) | der(1) | 2 | + |
| **BL136** | der(1)(qpterlq21::q21) | der(1) | 3 | ++ |
| Multiple Myeloma | | | | |
| XG2 | der(1) t(1;?)(q21;?) | der(1), normal 1 | 3 | ++++ |
| | der 19 t (1;19)(q12;?) | der(19) | | |
| XG7 | der(9) t (1;9)(q12;?) | der(9) | 4 | - |
| | der(19) t (1;19)(q12;?) | der(19) | | |
| | der(1) t(1;?)(q21;?) x2 | der(1) x2 | | |

We then investigated whether these aberrations had an effect on *IRTA2* mRNA expression. To this end, we used a cDNA probe corresponding to the *IRTA2* 5' untranslated region to screen a Northern blot with a panel of B-NHL and MM cell lines lacking or displaying lq21 chromosomal abnormalities. The results show that most (ten out of twelve) BL lines with normal lq21 chromosomes essentially lack *IRTA2* expression, consistent with the fact that BL derive from GC centroblasts which normally lack *IRTA2* expression (Figure 15A, left panel). In contrast, most BL lines carrying lq21 abnormalities (ten out of twelve) clearly display *IRTA2* mRNA upregulation (Figure 15A, right panel), ranging from 2 to 50 fold over baseline levels detected in BL with normal lq21. Among myeloma cell lines, *IRTA2* was overexpressed in one out of three lines displaying 1q21 abnormalities (XG2), while it was expressed in none out of seven with normal lq21 (Figure 15B) .

These results show a strong correlation between the presence of lq21 chromosomal aberrations and deregulation of *IRTA2* mRNA expression in BL and suggest that trisomies of the *IRTA2* locus may deregulate its expression in this lymphoma subtype (see Discussion).

### Discussion

Efforts described herein to identify genes involved in chromosomal aberrations affecting band lq21 in Multiple Myeloma and B cell lymphoma, led to the discovery of *IRTA1* and *IRTA2*, two founding members of a novel subfamily of related receptors within the immunoreceptor family; full length nucleic acid sequences encoding IRTA1 and IRTA2 proteins are provided herein, as are the amino acid sequences of the encoded IRTA1 and IRTA2 proteins. Subsequently three additional genes of members of this subfamily of related receptors were isolated, *IRTA3*, *IRTA4*, and *IRTA5,* the full length nucleic acid sequences of which are provided herein, as are the amino acid sequences of the encoded IRTA3, IRTA4, and *IRTA5* proteins. These results have implications for the normal biology of B cells as well as for the role of 1q21 aberrations in lymphomagenesis.

### IRTA1 and IRTA2 are founding members of a new subfamily within the Ig superfamily

Several features shared between the two IRTA genes and their encoded proteins suggest that they form a new subfamily within the immunoreceptor superfamily. First, they share a higher degree of homology with each other in their extracellular domains than with other superfamily members both in their mRNA (68% identity) and protein (47% identity) sequence. Second, they share homology in their cytoplasmic domains, marked by the presence of ITAM-like and ITIM signaling motifs in the context of homologous aminoacid sequences. Third, IRTA1 and IRTA2 belong to a larger subfamily of five genes displaying higher intrafamily homology and tight clustering within a ∼300 kb. region at lq21 (I.M. et al., manuscript in preparation). Their genomic organization suggests that a common ancestral gene may have given rise to this subfamily, by a process of duplication and sequence divergence, similar to the mechanism proposed for the Fc receptor family (Qiu et al., 1990).

In their extracellular domain, the *IRTA* proteins are closely related to the Fc receptor subfamily based on the high degree of aminoacid homology shared especially with the high affinity FCGRI receptor (37-45% aminoacid identity). A common evolutionary origin with Fc receptors is also suggested by the position of the *IRTA* family locus in the interval between the *FCGRI* locus on 1q21 and the *FCERI* and *FCGRII*-*III* loci on lq21-q23. Finally, the *IRTA* and *FCR* genes share a similar exon/intron organization of the gene portion that encodes their signal peptide, in particular the two 5' leader exons with the sequences encoding the signal peptidase site located within the second 21-bp exon.

Based on their cytoplasmic ITIM-like motifs, the IRTAproteins can be considered members of the Inhibitory Receptor Superfamily (IRS), a group of receptors that block activation of many cell types in the immune system (Lanier, 1998). Such members include FCGRIIB and CD22 in the human (DeLisser et al., 1994) and PIR-B in the mouse (Kubagawa et al., 1997). Analogous to IRS members, the ITIM of IRTA1 and IRTA2 are encoded by individual exons. A feature that many IRS members share is the existence of corresponding activating receptor isoforms whose cytoplasmic domains are devoid of ITIM (reviewed in Ravetch and Lanier, 1998) . It is possible that the secreted isoform of IRTA2, which lacks ITIM-like motifs, fulfills an analogous role by counteracting the effect of the transmembrane isoform.

Significant homology in the sequence and overall organization of their extracellular portion is shared among the IRTA1 and IRTA2 proteins and the Cell Adhesion Molecule (CAM) subfamily members PECAM1 , CD22 and BGP1. In addition, the ability of IRTA2 to generate three protein isoforms with distinct subcellular localization (a transmembrane, a GPI-linked or a secreted protein) by differential splicing is shared by *NCAM,* another member of the CAM subfamily (Dickson et al., 1987; Gower et al., 1988). Thus, the IRTA family is also related to the CAM family, as has been previously suggested for a member of the Fc receptor family (murine FCGRII) because of its homology to PECAM1 (CAM, IRS family) (Daeron, 1991; Newman et al., 1990; Stockinger et al., 1990).

In conclusion, the IRTA family may represent an intersection among the Fc, IRS and CAM families, combining features from all three. Accordingly, IRTA proteins may have a role in the regulation of signal transduction during an immune response (like Fc receptors), intercellular communication (like members of the IRS and CAM families) and cell migration (like CAM family members) (DeLisser et al., 1994; Ravetch and Lanier, 2000). Initial experiments indicate that IRTA1 can weakly bind heat aggregated IgA, while IRTA2c can specifically bind heat aggregated human serum IgG (with higher affinity for IgG₁ and IgG₂), but not monomeric human IgG, IgA, IgM and IgE (data not shown). These initial data lend support to a functional relationship between the IRTA and the Fc receptor families, but do not exclude functions dependent on other ligands for the IRTA proteins.

### Differential pattern of expression of IRTA genes in mature B cells

The *IRTA* genes display a specific pattern of expression in various normal B cell compartments. *IRTA1* is topographically restricted to B cells within the perifollicular region, which was originally named marginal zone in the spleen, but is also detectable in most lymphoid organs (de Wolf-Peeters et al., 1997). The *in situ* hybridization data presented here have been confirmed by immunohistochemical analysis using anti-IRTA1 antibodies which show that the IRTA1 protein is selectively expressed in marginal zone B cells, and, among NHL, in marginal zone lymphoma, the tumors deriving from these cells (G. Cattoretti et al., manuscript in preparation). On the other hand, *IRTA2* has a broader pattern of expression that includes GC centrocytes, as well as a broad spectrum of perifollicular cells, which may include immunoblasts and memory cells. Initial data suggest that the pattern of expression of *IRTA3* is analogous to *IRTA2,* while *IRTA4* and *IRTA5* are selectively expressed in mantle zone B cells (I. Miller et al., manuscript in preparation), the pre-GC compartment of mature B cells (MacLennan, I. C., 1994). This topographic restriction of *IRTA* gene expression in lymphoid organs suggests that the IRTA molecules may play a role in the migration or activity of various B cell subpopulations in specific functional B cell compartments. In addition, IRTA expression should be useful for the differential diagnosis of NHL subtypes deriving from various B cell compartments, particularly IRTA1 in the diagnosis of marginal zone lymphoma.

### IRTA1 locus and 1q21 abnormalities in MM

In the FR4 cell line, the consequence of the t(1;14) translocation is the formation of an *IRTA1*/*Cα*₁ fusion gene. Despite the fact that this gene is driven by the *IRTA1* promoter region, which is normally silent in plasma cells, its expression is high in FR4, presumably due to the influence of the *Cα₁* 3' LCR, which is retained downstream of the *Cα*₁ locus. The fusion gene encodes a IRTA1/Cα₁ fusion protein which contains only the signal peptide and first two amino acids of IRTA1 linked to the surface IgA receptor. The latter has been almost completely deprived of its extracellular domain, but retains all its transmembrane and intracellular domains. This structure indicates that the IRTA1/Cα₁ fusion protein, though probably unable to bind any ligand, may retain the potential for dimerization and signaling. In particular, the membrane (m) IgA-derived extracellular portion contains a cysteine residue, which can be involved in disulphide bonds between two α-chains or between α-chains and associated proteins, such as the auxilliary surface receptor CD19 (Leduc et al., 1997). The fusion protein also carries the intact, 14 amino acid mIgA cytoplasmic domain, which is highly conserved in evolution (Reth, 1992) and may play an essential role in the proliferation, survival and differentiation of mature B-cells, analogous to the role of mIgG and mIgE (Kaisho et al., 1997) . Thus, the emergence of the IRTA1/Ca₁ protein in FR4 may have provided the cells with a proliferative and survival advantage during tumor development through ligand (antigen)-independent activation of the BCR pathway. This fusion event however, appears to be rare in B-cell malignancy, since so far we were able to detect it only in FR4 cells.

### IRTA2 locus and lq21 abnormalities in MM and BL

Abnormal expression of *IRTA2* is a frequent consequence of lq21 abnormalities. Although this gene is not expressed normally either in centroblasts, the presumed normal counterparts of BL (Kuppers et al., 1999), or in BL with normal lq21, its levels are upregulated on average by 10 - fold in BL cell lines with lq21 abnormalities. This deregulation appears to be specific for *IRTA2* since all the other 4 *IRTA* genes present within 300 kb on lq21 are either not expressed in BL (*IRTA1*)*,* or their pattern of expression does not correlate with the presence of lq21 abnormalities (*IRTA3,* 4, 5, not shown) . The mechanism by which this deregulation occurs is difficult to ascertain in the absence of structural lesions within or adjacent to the *IRTA2* gene. Since the heterogeneous aberrations that affect la21 all cause an excess copy number of the *IRTA* locus, it is possible that this may lead to regulatory disturbances, as is the case for low level amplification of *BCL2* in FL lacking (14;18) translocations (Monni et al., 1997), *REL* in diffuse large cell lymphoma (Houldsworth et al., 1996; Rao et al., 1998) and deregulation of Cyclin D1 in some MM cases with trisomy 11 (Pruneri et al., 2000). On the other hand, lq21 abnormalities, including translocation and duplications, change the genomic context of the *IRTA* locus and may lead to deregulation of *IRTA2* by distant cis-acting enhancer chromatin organizing elements acting on its promoter as is the case for MYC in endemic BL (Pelicci et al., 1986) and MM (Shou et al., 2000) and for *CCND1* in mantle cell lymphoma (Bosch et al., 1994; Swerdlow et al., 1995) and MM. (Pruneri et al., 2000).

The biological consequences of deregulated *IRTA2* expression are difficult to predict at this stage. The observation that IRTA2 has homology with CAM adhesion receptors, together with its specific distribution in the light zone of the GC suggest that its ectopic expression in centroblasts may cause a disruption in the GC development and architecture. On the other hand, our initial observations that IRTA2 can bind IgG immune complexes comparably to bona fide Fc receptors suggest that its inappropriate expression may perturb the dynamics of cell surface regulation of B cell immunological responses, possibly leading to clonal expansion. Deregulated expression of *FCGR2B* as a result of the t(1;14) (q21;q32) in follicular lymphoma has been proposed to contribute to lymphomagenesis in this tumor type (Callanan et al., 2000), by a mechanism involving escape by tumor cells of anti-tumor immune surveillance through their Fc binding and inactivation of tumor specific IgG. Similar evasion mechanisms have been observed in cells infected by Fc-encoding herpesvisures (Dubin et al., 1991). The role of IRTA2 deregulation needs to be tested in "gain of function" transgenic mice constitutively expressing IRTA2 in the GC.

### References for Second Series of Experiments

Anand, R., Riley, J. H., Butler, R., Smith, J. C., and Markham, A. F. (1990). A 3.5 genome equivalent multi access YAC library: construction, characterisation, screening and storage. Nucleic Acids Res 18, 1951-6.
Avet-Loiseau, H., Andree-Ashley, L. E., Moore, D., 2nd, Mellerin, M. P., Feusner, J., Bataille, R., and Pallavicini, M. G. (1997). Molecular cytogenetic abnormalities in multiple myeloma and plasma cell leukemia measured using comparative genomic hybridization. Genes Chromosomes Cancer 19, 124-33.
Bakhshi, A., Jensen, J. P., Goldman, P., Wright, J. J., McBride, O. W., Epstein, A. L., and Korsmeyer, S. J. (1985). Cloning the chromosomal breakpoint of t(14;18) human lymphomas: clustering around JH on chromosome 14 and near a transcriptional unit on 18. Cell 41, 899-906.
Berger, R., Bernheim, A. (1985). Cytogenetics of Burkitt's lymphoma-leukaemia: a review. IARC Sci Publ 60, 65-80.
Bergsagel, P. L., Chesi, M., Nardini, E., Brents, L. A., Kirby, S. L., and Kuehl, W. M. (1996). Promiscuous translocations into immunoglobulin heavy chain switch regions in multiple myeloma. Proc Natl Acad Sci U S A 93, 13931-6.
Bosch, F., Jares, P., Campo, E., Lopez-Guillermo, A., Piris, M. A., Villamor, N., Tassies, D., Jaffe, E. S., Montserrat, E., Rozman, C.et al. (1994). PRAD-1/cyclin D1 gene overexpression in chronic lymphoproliferative disorders: a highly specific marker of mantle cell lymphoma. Blood 84, 2726-32.
Callanan, M. B., Le Baccon, P., Mossuz, P., Duley, S., Bastard, C., Hamoudi, R., Dyer, M. J., Klobeck, G., Rimokh, R., Sotto, J. J., and Leroux, D. (2000). The IgG Fc receptor, FcgammaRIIB, is a target for deregulation by chromosomal translocation in malignant lymphoma. Proc Natl Acad Sci U S A 97, 309-14.
Cambier, J. C. (1995). Antigen and Fc receptor signaling. The awesome power of the immunoreceptor tyrosine-based activation motif (ITAM). J Immunol 155, 3281-5.
Chesi, M., Bergsagel, P. L., Brents, L. A., Smith, C. M., Gerhard, D. S., and Kuehl, W. M. (1996). Dysregulation of cyclin D1 by translocation into an IgH gamma switch region in two multiple myeloma cell lines [see comments]. Blood 88, 674-81.
Chesi, M., Bergsagel, P. L., Shonukan, O. O., Martelli, M. L., Brents, L. A., Chen, T., Schrock, E., Ried, T., and Kuehl, W. M. (1998). Frequent dysregulation of the c-maf proto-oncogene at 16q23 by translocation to an Ig locus in multiple myeloma. Blood 91, 4457-63.
Chesi, M., Nardini, E., Brents, L. A., Schrock, E., Ried, T., Kuehl, W. M., and Bergsagel, P. L. (1997). Frequent translocation t(4;14) (p16.3;q32.3) in multiple myeloma is associated with increased expression and activating mutations of fibroblast growth factor receptor 3. Nat Genet 16, 260-4.
Church, D. M., Stotler, C. J., Rutter, J. L., Murrell, J. R., Trofatter, J. A., and Buckler, A. J. (1994). Isolation of genes from complex sources of mammalian genomic DNA using exon amplification. Nat Genet 6, 98-105.
Cigudosa, J. C., Parsa, N. Z., Louie, D. C., Filippa, D. A., Jhanwar, S. C., Johansson, B., Mitelman, F., and Chaganti, R. S. (1999). Cytogenetic analysis of 363 consecutively ascertained diffuse large B- cell lymphomas. Genes Chromosomes Cancer 25, 123-33.
Daeron, M. (1991). Fc receptors, or the elective affinities of adhesion molecules. Immunol Lett 27, 175-81.
Dalla-Favera, R., Bregni, M., Erikson, J., Patterson, D., Gallo, R.C., and Croce, C.M.: The human c-myc onc-gene is located on the region of chromosome 8 which is translocated in Burkitt lymphoma cells. Proc. Nat. Acad. Sci. USA 79:7824-7827, 1982.
Dalla-Favera, R., Martinotti, S., Gallo, R. C., Erikson, J., and Croce, C. M. (1983). Translocation and rearrangements of the c-myc oncogene locus in human undifferentiated B-cell lymphomas. Science 219, 963-7.
de Wolf-Peeters, C. , Pittaluga, S., Dierlamm, J., Wlodarska, I., and Van Den Berghe, H. (1997). Marginal zone B-cell lymphomas including mucosa-associated lymphoid tissue type lymphoma (MALT), monocytoid B-cell lymphoma and splenic marginal zone cell lymphoma and their relation to the reactive marginal zone. Leuk Lymphoma 26, 467-78.
DeLisser, H. M., Newman, P. J., and Albelda, S. M. (1994). Molecular and functional aspects of PECAM-1/CD31. Immunol Today 15, 490-5.
Dickson, G., Gower, H. J., Barton, C. H., Prentice, H. M., Elsom, V. L., Moore, S. E., Cox, R. D., Quinn, C., Putt, W., and Walsh, F. S. (1987). Human muscle neural cell adhesion molecule (N-CAM): identification of a musclespecific sequence in the extracellular domain. Cell 50, 1119-30.
Dierlamm, J., Pittaluga, S., Wlodarska, I., Stul, M., Thomas, J., Boogaerts, M., Michaux, L., Driessen, A., Mecucci, C., Cassiman, J. J., and et al. (1996). Marginal zone B-cell lymphomas of different sites share similar cytogenetic and morphologic features [see comments]. Blood 87, 299-307.
Dracopoli, C. N., Haines, J. L., Korf, B. R., Morton, C. C., Seidman, C. E., Seidman, J.G., Smith, D. R. (1997). Current Protocols in Human Genetics (New York: Wiley & Sons)
Dubin, G., Socolof, E., Frank, I., Friedman, H. M. (1991). Herpes simplex virus type 1 Fc receptor protects infected cells from antibody-dependent cellular cytotoxicity. Journal of Virology 65, 7046-50.
Dyomin, V. G., Palanisamy, N., Lloyd, K. O., Dyomina, K., Jhanwar, S. C., Houldsworth, J., and Chaganti, R. S. (2000). MUC1 is activated in a B-cell lymphoma by the t(1;14)(q21;q32) translocation and is rearranged and amplified in B-cell lymphoma subsets. Blood 95, 2666-71.
Dyomin, V.G., Rao, P.H., Dalla-Favera, R., Chaganti, R.S.K. (1997). BCL8 a novel gene involved in translocations affecting band 15q11-13 in diffuse largecell lymphoma. Proc Natl Acad Sci USA 94, 5728-32.
Eton, O., Scheinberg, D. A., and Houghton, A. N. (1989). Establishment and characterization of two human myeloma cell lines secreting kappa light chains. Leukemia 3, 729-35.
Ferguson, M. A., and Williams, A. F. (1988). Cell-surface anchoring of proteins via glycosyl-phosphatidylinositol structures. Annu Rev Biochem 57, 285-320.
Frank, D., Mendelsohn, C. L., Ciccone, E., Svensson, K., Ohlsson, R., and Tycko, B. (1999). A novel pleckstrin homology-related gene family defined by Ipl/Tssc3, TDAG51, and Tih1: tissue-specific expression, chromosomal location, and parental imprinting. Mamm Genome 10, 1150-1159.
Gaidano, G., and Dalla-Favera, R. (1997). Molecular Biology of Lymphomas. In: Principles and Practice of Oncology, Fifth Ed, DeVita, VT, Hellman, S., Rosenberg SA (eds) JB Lippincott Co (publ.), 2131-2145.
Gilles, F., Goy, A., Remache, Y., Shue, P., and Zelenetz, A. D. (2000). MUC1 dysregulation as the consequence of a tt(1;14) (q21;q32) translocation in an extranodal lymphoma. Blood 95, 2930-2936.
Gower, H. J., Barton, C. H., Elsom, V. L., Thompson, J., Moore, S. E., Dickson, G., and Walsh, F. S. (1988). Alternative splicing generates a secreted form of N-CAM in muscle and brain. Cell 55, 955-64.
Hamilton, M. S., Ball, J., Bromidge, E., Lowe, J., and Franklin, I. M. (1990). Characterization of new IgG lambda myeloma plasma cell line (EJM): a further tool in the investigation of the biology of multiple myeloma. Br J Haematol 75, 378-84.
Houldsworth, J., Mathew, S., Rao, P. H., Dyomina, K., Louie, D. C., Parsa, N., Offit, K., Chaganti, R. S. (1996). REL proto-oncogene is frequently amplified in extranodal diffuse large cell lymphoma. Blood 87, 25-9.
Iida, S., Rao, P. H., Butler, M., Corradini, P., Boccadoro, M., Klein, B., Chaganti, R. S., and DallaFavera, R. (1997). Deregulation of MUM1/IRF4 by chromosomal translocation in multiple myeloma. Nat Genet 17, 226-30.
Jackson, N., Lowe, J., Ball, J., Bromidge, E., Ling, N. R., Larkins, S., Griffith, M. J., and Franklin, I. M. (1989). Two new. IgAl-kappa plasma cell leukaemia cell lines (JJN-1 & JJN-2) which proliferate in response to B cell stimulatory factor 2. Clin Exp Immunol 75, 93-9.
Jernberg, H., Zech, L., and Nilsson, K. (1987). Cytogenetic studies on human myeloma cell lines. Int J Cancer 40, 811-7.
Juliusson, G., Oscier, D. G., Fitchett, M., Ross, F. M., Stockdill, G., Mackie, M. J., Parker, A. C., Castoldi, G. L., Guneo, A., Knuutila, S., and et al. (1990). Prognostic subgroups in B-cell chronic lymphocytic leukemia defined by specific chromosomal abnormalities. N Engl J Med 323, 720-4.
Kaisho, T., Schwenk, F., and Rajewsky, K. (1997). The roles of gamma 1 heavy chain membrane expression and cytoplasmic tail in IgG1 responses. Science 276, 412-5.
Kempkes, B., Spitkovsky, D., Jansen-Durr, P., Ellwart, J. W., Kremmer, E., Delecluse, H. J., Rottenberger, C., Bornkamm, G. W., and Hammerschmidt, W. (1995). B-cell proliferation and induction of early Gl-regulating proteins by Epstein-Barr virus mutants conditional for EBNA2. Embo J 14, 88-96.
Kornblau, S. M., Goodacre, A., Cabanillas, F. (1991). Chromosomal abnormalities in adult non-endemic Burkitt's lymphoma and leukemia: 22 new reports and a review of 148 cases from the literature. Hematol Oncol 9, 63-78
Kubagawa, H., Burrows, P. D., and Cooper, M. D. (1997). A novel pair of immunoglobulin-like receptors expressed by B cells and myeloid cells [see comments]. Proc Natl Acad Sci U S A 94, 5261-6.
Kuppers, R., Klein, U., Hansmann, M. L., and Rajewsky, K. (1999). Cellular origin of human B-cell lymphomas. N Engl J Med 341, 1520-9.
Lanier, L. L. (1998). NK cell receptors. Annu Rev Immunol 16, 359-93.
Leduc, I., Drouet, M., Bodinier, M. C. , Helal, A., and Cogne, M. (1997). Membrane isoforms of human immunoglobulins of the A1 and A2 isotypes: structural and functional study. Immunology 90, 330-6.
MacLennan, I. C. (1994). Germinal Centers. Annu Rev Immunol 12, 117-39.
Magrath, I. T., Pizzo, P. A., Whang-Peng, J., Douglass, E. C., Alabaster, O., Gerber, P., Freeman, C. B., and Novikovs, L. (1980). Characterization of lymphoma-derived cell lines: comparison of cell lines positive and negative for Epstein-Barr virus nuclear antigen. I. Physical, Cytogenetic, and growth characteristics. J Natl Cancer Inst 64, 465-76.
Monni, O. Joensuu, H., Franssila, K., Klefstrom, J., Alitalo, K., and Knuutila, S. (1997). BCL2 overexpression associated with chromosomal amplification in diffuse large B-cell lymphoma. Blood 90, 1168-74.
Neri, A., Barriga, F., Knowles, D. M., Magrath, I. T., and Dalla-Favera, R. (1988). Different regions of the immunoglobulin heavy-chain locus are involved in chromosomal translocations in distinct pathogenetic forms of Burkitt lymphoma. Proc Natl Acad Sci U S A 85, 2748-52.
Neri, A., Chang, C. C., Lombardi, L., Salina, M., Corradini, P., Maiolo, A. T., Chaganti, R. S., and Dalla-Favera, R. (1991). B cell lymphoma-associated chromosomal translocation involves candidate oncogene lyt-10, homologous to NF-kappa B p50. Cell 67, 1075-87.
Newman, P. J., Berndt, M. C., Gorski, J., White, G. C. d., Lyman, S., Paddock, C., and Muller, W. A. (1990). PECAM-1 (CD31) cloning and relation to adhesion molecules of the immunoglobulin gene superfamily. Science 247, 1219-22.
Offit, K., Louie, D. C., Parsa, N. Z., Roy, P., Leung, D., Lo Coco, F., Zelenetz, A., Dalla-Favera, R., Chaganti, R. S. (1995). BCL6 gene rearrangement and other cytogenetic abnormalities in diffuse large cell lymphoma. Leuk Lymphoma 20, 85-9.
Pelicci, P. G., Knowles, D. M. d., Magrath, I., and DallaFavera, R. (1986). Chromosomal breakpoints and structural alterations of the c-myc locus differ in endemic and sporadic forms of Burkitt lymphoma. Proc Natl Acad Sci U S A 83, 2984-8.
   Polito, P., Cilia, A. M., Gloghini, A., Cozzi, M., Perin, T., De Paoli, P., Gaidano, G., and Carbone, A. (1995). High frequency of EBV association with non-random abnormalities of the chromosome region lq21-25 in AIDS-related Burkitt's lymphoma-derived cell lines. Int J Cancer 61, 370-4.
Pruneri, G., Fabris, S., Baldini, L., Carboni, N., Zagano, S., Colombi, M. A., Ciceri, G., Lombardi, L., Rocchi, M., Buffa, R., Maiolo, A. T., Neri, A. (2000). Immunohistochemical analysis of cyclin D1 shows deregulated expression in multiple myeloma with the t(11;14). Am J Pathol 156, 1505-13.
Qiu, W. Q., de Bruin, D., Brownstein, B. H., Pearse, R., Ravetch, J. V. (1990). Organization of the human and mouse low-affinity Fc gamma R genes: duplication and recombination. Science 248, 732-5.
Rao, P. H., Houldsworth, J., Dyomina, K., Parsa, N. Z., Cigudosa, J. C., Louie, D. C., Popplewell, L., Offit, K., Jhanwar, S. C., and Chaganti, R. S. (1998). Chromosomal and gene amplification in diffuse large B-cell lymphoma. Blood 92, 234-40.
Rao, P. H., Murty, V. V., Gaidano, G., Hauptschein, R., Dalla-Favera, R., and Chaganti, R. S. (1993). Subregional localization of 20 single-copy loci to chromosome 6 by fluorescence in situ hybridization. Genomics 16, 426-30.
Ravetch, J. V., and Lanier, L. L. (2000). Immune inhibitory receptors [In Process Citation]. Science 290, 84-9.
Reth,. M. (1989). Antigen receptor tail clue [letter] Nature 338, 383-4.
   Reth, M. (1992). Antigen receptors on B lymphocytes. Annu Rev Immunol 10, 97-121.
Richelda, R., Ronchetti, D., Baldini, L., Cro, L., Viggiano, L., Marzella, R., Rocchi, M., Otsuki, T., Lombardi, L., Maiolo, A. T., Neri, A. (1997). A novel chromosomal translocation t(4; 14)(p16.3; q32) in multiple myeloma involves the fibroblast growth-factor receptor 3 gene [see comments]. Blood 90, 4062-70.
Riley, J., Butler, R., Ogilvie, D., Finniear, R., Jenner, D., Powell, S., Anand, R., Smith, J. C., and Markham, A. F. (1990). A novel, rapid method for the isolation of terminal sequences from yeast artificial chromosome (YAC) clones. Nucleic Acids Res 18, 2887-90.
Ronchetti, D., Finelli, P., Richelda, R., Baldini, L., Rocchi, M., Viggiano, L., Cuneo, A., Bogni, S., Fabris, S., Lombardi, L., Maiolo, A. T., and Neri, A. (1999). Molecular analysis of 11q13 breakpoints in multiple myeloma. Blood 93, 1330-7.
Rosenberg, C. L., Wong, E., Petty, E. M., Bale, A. E., Tsujimoto, Y., Harris, N. L., and Arnold, A. (1991). PRAD1, a candidate BCL1 oncogene: mapping and expression in centrocytic lymphoma. Proc Natl Acad Sci U S A 88, 9638-42.
Sawyer, J. R., Tricot, G., Mattox, S., Jagannath, S., and Barlogie, B. (1998). Jumping translocations of chromosome 1q in multiple myeloma: evidence for a mechanism involving decondensation of pericentromeric heterochromatin. Blood 91, 1732-41.
Sawyer, J. R., Waldron, J. A., Jagannath, S., Barlogie, B. (1995). Cytogenetic findings in 200 patients with multiple myeloma. Cancer Genet Cytogenet 82, 41-9.
Shou, Y., Martelli, M. L., Gabrea, A., Qi, Y., Brents, L. A., Roschke, A., Dewald, G., Kirsch, I. R., Bergsagel, P. L., and Kuehl, W. M. (2000). Diverse karyotypic abnormalities of the c-myc locus associated with c- myc dysregulation and tumor progression in multiple myeloma. Proc Natl Acad Sci U S A 97, 228-33.
Stockinger, H., Gadd, S. J., Eher, R., Majdic, O., Schreiber, W., Kasinrerk, W., Strass, B., Schnabl, E., and Knapp, W. (1990). Molecular characterization and functional analysis of the leukocyte surface protein CD31. J Immunol 145, 3889-97.
Swerdlow, S. H., Yang, W. I., Zukerberg, L. R., Harris, N. L., Arnold, A., Williams, M. E. (1995). Expression of cyclin D1 protein in centrocytic/mantle cell lymphomas with and without rearrangement of the BCLZ/cyclin D1 gene. Hum Pathol 26, 999-1004.
Tagawa, S., Doi, S., Taniwaki, M., Abe, T., Kanayama, Y., Nojima, J., Matsubara, K., and Kitani, T. (1990). Amylase-producing plasmacytoma cell lines, AD3 and FR4, with der (14) t (8;14) and dic(8) t (1;8) established from ascites. Leukemia 4, 600-5.
Taub, R., Kirsch, I., Morton, C., Lenoir, G., Swan, D., Tronick, S., Aaronson, S., Leder, P. (1982). Translocation of the c-myc gene into the immunoglobulin heavy chain locus in human Burkitt lymphoma and murine plasmacytoma cells. Proc Natl Acad Sci U S A 79, 7837-41.
   Thompson, J. D., Higgins, D. G., Gibson, T. J. CLUSTAL W: improving the sensitivity of progressive multiple sequence alignment through sequence weighting, position-specific gap penalties and weight matrix choice. Nucleic Acids Res 22, 4673-80.
Tusnady, G. E., Simon, I. (1998). Principles governing amino acid composition of integral membrane proteins: application to topology prediction. J Mol Bio 283, 489-506.
Unkeless, J. C., and Jin, J. (1997). Inhibitory receptors, ITIM sequences and phosphatases. Curr Opin Immunol 9, 338-43.
von Heijne, G. (1986). A new method for predicting signal sequence cleavage sites. Nucleic Acids Res 14, 4683-90.
Whang-Peng, J., Knutsen, T., Jaffe, E. S., Steinberg, S. M., Raffeld, M., Zhao, W. P., Duffey, P., Condron, K., Yano, T., Longo, D. L. (1995). Sequential analysis of 43 patients with non-Hodgkin's lymphoma: clinical correlations with cytogenetic, histologic, immunophenotyping, and molecular studies. Blood 85, 203-16.
willis, T. G., Zalcberg, I. R., Coignet, L. J., Wlodarska, I., Stul, M., Jadayel, D. M., Bastard, C., Treleaven, J. G., Catovsky, D., Silva, M. L., and Dyer, M. J. (1998). Molecular cloning of translocation tt (1;14) (q21;q32) defines a novel gene (BCL9) at chromosome lq21. Blood 91, 1873-81.
Ye, B. H., Lista, F., Lo Coco, F., Knowles, D. M., Of fit, K., Chaganti, R. S., and Dalla-Favera, R. (1993). Alterations of a zinc finger-encoding gene, BCL-6, in diffuse large- cell lymphoma. Science 262, 747-50.
Yu, L. M., Peng, C., Starnes, S. M., Liou, R. S., and Chang, T. W. (1990). Two isoforms of human membrane-bound alpha Ig resulting from alternative mRNA splicing in the membrane segment. J Immunol 145, 3932-6.
Zhang, X. G., Gaillard, J. P., Robillard, N., Lu, Z. Y., Gu, Z. J., Jourdan, M., Boiron, J. M., Bataille, R., and Klein, B. (1994). Reproducible obtaining of human myeloma cell lines as a model for tumor stem cell study in human multiple myeloma. Blood 83, 3654-63.

### Third Series of Experiments

Chromosome 1q21 is frequently altered by translocations and duplications in several types of B cell malignancy, including multiple myeloma, Burkitt lymphoma, marginal zone lymphomas, and follicular lymphoma. To identify the genes involved in these aberrations, cloned was the chromosomal breakpoint of a t (1;14) (q21;q32) in the myeloma cell line FR4. A 300kb region spanning the breakpoint contains at least five highly related adjacent genes which encode surface receptor molecules that are members of the immunoglobulin gene superfamily, and thus called IRTA (Immunoglobulin Receptor Translocation Associated). The various IRTA molecules have from three to nine extracellular immunoglobulin superfamily domains and are related to the Fc gamma receptors. They have transmembrane and cytoplasmic domains containing ITIM-like and ITAM-like (ITRA-1, IRTA-3, IRTA-4) signaling motifs. In situ hybridization experiments show that all IRTA genes are expresed in the B cell lineage with distinct developmental stage-specific patterns: IRTA-1 is expressed in a marginal B cell pattern. IRTA-2 is expressed in centrocytes and more mature B cells. As a result of the translocation in FR4, IRTA,-1 is broken and produces a fusion transcript with the immunoglobulin locus. The IRTA-2 gene, normally silent in centroblasts, is overexpressed in multiple myeloma and in Burkitt lymphoma cell lines carrying 1q21 abnormalities. The data here suggests that IRTA genes are novel B cell regulatory molecules that may also have a role in lymphomagenesis.

## Claims

1. An antibody directed to a human IRTA4 protein, wherein the amino acid sequence of the human IRTA4 protein is set forth in Figure 18D1 - 18D2.

2. An antibody directed to a human IRTA4 protein, wherein the antibody is conjugated to a therapeutic agent, wherein the therapeutic agent is selected from the group consisting of a radioisotope, a toxin, a toxoid, or a chemotherapeutic agent, and wherein the amino acid sequence of the human IRTA4 protein is set forth in Figure 18D1 - 18D2.

3. The antibody of claim 1 or 2 for use in treating a subject having a B cell cancer.

4. An antibody directed to a human IRTA4 protein effective to bind to cancer cells expressing a human IRTA4 protein so as to prevent growth of the cancer cells for use in treating a subject having a B cell cancer wherein the amino acid sequence of the human IRTA4 protein is set forth in Figure 18D1 - 18D2.

5. The antibody of any one of claims 1-4, wherein the antibody specifically binds to the extracellular domain of a human IRTA4 protein.

6. The antibody of any one of claims 1-5, wherein the antibody is a monoclonal antibody.

7. The antibody of claim 6, wherein the monoclonal antibody is a humanized monoclonal antibody.

8. A composition comprising an amount of the antibody of any one of claims 1-7 effective to prevent the growth of cancer cells expressing human IRTA4 and a pharmaceutically acceptable carrier.

9. The composition of claim 8 for use in the treatment of a B cell cancer.

10. The composition of claim 8, wherein the cancer cells are selected from the group consisting of B cell lymphoma, a mantle cell lymphoma, multiple myeloma, Burkitt's lymphoma, marginal zone lymphoma, diffuse large cell lymphoma and follicular lymphoma cells.

11. The composition of claim 8, wherein the cancer cells are Mucosa-Associated-Lymphoid Tissue B cell lymphoma (MALT) cells or non-Hodgkin's Lymphoma cells.

12. The composition of claim 8, wherein the antibody is a monoclonal antibody.

13. The composition of claim 12, wherein the monoclonal antibody is a humanized monoclonal antibody.

14. A human IRTA4 protein comprising amino acids, the sequence of which is set forth in Figure 18D1 - 18D2.

15. A method of diagnosing a B cell malignancy which comprises a 1q21 chromosomal rearrangement in a sample containing cancer cells from a subject which comprises:
a) contacting a sample obtained from the subject with the antibody of claim 1, wherein the antibody is labeled with a detectable marker; and
b) detecting any binding in step (a), wherein detecting of binding indicates a diagnosis of B cell malignancy in the sample.

## Patentansprüche

1. Antikörper, der auf ein humanes IRTA4-Protein gerichtet ist, wobei die Aminosäuresequenz des humanen IRTA4-Proteins in Abbildung 18D1 bis 18D2 dargestellt ist.

2. Antikörper, der auf ein humanes IRTA4-Protein gerichtet ist, wobei der Antikörper an ein therapeutisches Agens konjugiert ist, wobei das therapeutische Agens aus der Gruppe bestehend aus einem Radioisotop, einem Toxin, einem Toxoid oder einem chemotherapeutischen Agens ausgewählt ist und wobei die Aminosäuresequenz des humanen IRTA4-Proteins in Abbildung 18D1 bis 18D2 dargestellt ist.

3. Antikörper nach Anspruch 1 oder 2 zur Verwendung bei der Behandlung eines Individuums, das eine B-Zell-Krebserkrankung hat.

4. Antikörper, der auf ein humanes IRTA4-Protein gerichtet ist, das wirksam ist, an Krebszellen zu binden, die ein humanes IRTA4-Protein exprimieren, sodass das Wachstum der Krebszellen verhindert wird, zur Verwendung bei der Behandlung eines Individuums, das eine B-Zell-Krebserkrankung hat, wobei die Aminosäuresequenz des humanen IRTA4-Proteins in Abbildung 18D1 bis 18D2 dargestellt ist.

5. Antikörper nach einem der Ansprüche 1 bis 4, wobei der Antikörper an die extrazelluläre Domäne eines humanen IRTA4-Proteins spezifisch bindet.

6. Antikörper nach einem der Ansprüche 1 bis 5, wobei der Antikörper ein monoclonaler Antikörper ist.

7. Antikörper nach Anspruch 6, wobei der monoclonale Antikörper ein humanisierter monoclonaler Antikörper ist.

8. Zusammensetzung, die eine Menge des Antikörpers nach einem der Ansprüche 1 bis 7, die wirksam ist, um das Wachstum von Krebszellen zu verhindern, die humanes IRTA4-Protein exprimieren, und einen pharmazeutisch verträglichen Träger umfasst.

9. Zusammensetzung nach Anspruch 8 zur Verwendung bei der Behandlung einer B-Zell-Krebserkrankung.

10. Zusammensetzung nach Anspruch 8, wobei die Krebszellen ausgewählt sind aus der Gruppe bestehend aus B-Zell-Lymphom, einem Mantelzell-Lymphom, Multiplem Myelom, Burkitt-Lymphom, Marginalzonen-Lymphom, diffusem großzelligem Zell-Lymphom und follikulären Lymphomzellen.

11. Zusammensetzung nach Anspruch 8, wobei die Krebszellen Zellen des B-Zell-Lymphoms des Mukosa-assoziierten lymphatischen Gewebes (MALT) oder des Non-Hodgkin-Lymphoms sind.

12. Zusammensetzung nach Anspruch 8, wobei der Antikörper ein monoclonaler Antikörper ist.

13. Zusammensetzung nach Anspruch 12, wobei der monoclonale Antikörper ein humanisierter monoclonaler Antikörper ist.

14. Humanes IRTA4-Protein, das Aminosäuren umfasst, deren Sequenz in Abbildung 18D1 bis 18D2 dargestellt ist.

15. Verfahren zum Diagnostizieren eines B-Zell-Malignoms, das ein Rearrangement des Chromosoms 1q21 umfasst, in einer Probe, die Krebszellen von einem Individuum enthält, das umfasst:
a) Inkontaktbringen der vom Individuum erhaltenen Probe mit dem Antikörper nach Anspruch 1, wobei der Antikörper mit einem nachweisbaren Marker markiert ist; und
b) Nachweisen einer Bindung in Schritt (a), wobei der Nachweis einer Bindung auf eine Diagnose eines B-Zell-Malignoms in der Probe hinweist.

## Revendications

1. Anticorps dirigé contre une protéine IRTA4 humaine, dans lequel la séquence d'acides aminés de la protéine IRTA4 humaine est présentée dans la figure 18D1 - 18D2.

2. Anticorps dirigé contre une protéine IRTA4 humaine, dans lequel l'anticorps est conjugué à un agent thérapeutique, dans lequel l'agent thérapeutique est sélectionné dans le groupe constitué par un radio-isotope, une toxine, une anatoxine ou un agent chimio-thérapeutique, et dans lequel la séquence d'acides aminés de la protéine IRTA4 humaine est présentée dans la figure 18D1 - 18D2.

3. Anticorps selon la revendication 1 ou 2 pour une utilisation dans le traitement d'un sujet ayant un cancer des cellules B.

4. Anticorps dirigé contre une protéine IRTA4 humaine efficace pour se lier à des cellules cancéreuses exprimant une protéine IRTA4 humaine de manière à empêcher la croissance des cellules cancéreuses pour une utilisation dans le traitement d'un sujet ayant un cancer des cellules B dans lequel la séquence d'acides aminés de la protéine IRTA4 humaine est présentée dans la figure 18D1 - 18D2.

5. Anticorps selon l'une quelconque des revendications 1-4, dans lequel l'anticorps se lie spécifiquement au domaine extracellulaire d'une protéine IRTA4 humaine.

6. Anticorps selon l'une quelconque des revendications 1-5, dans lequel l'anticorps est un anticorps monoclonal.

7. Anticorps selon la revendication 6, dans lequel l'anticorps monoclonal est un anticorps monoclonal humanisé.

8. Composition comprenant une certaine quantité d'anticorps selon l'une quelconque des revendications 1-7 efficace pour empêcher la croissance des cellules cancéreuses exprimant IRTA4 humaine et un support acceptable du point de vue pharmaceutique.

9. Composition selon la revendication 8 pour une utilisation dans le traitement d'un cancer des cellules B.

10. Composition selon la revendication 8, dans laquelle les cellules cancéreuses sont sélectionnées dans le groupe constitué par le lymphome à cellules B, un lymphome à cellules du manteau, le myélome multiple, le lymphome de Burkitt, le lymphome de la zone marginale, le lymphome diffus à grandes cellules et des cellules de lymphome folliculaire.

11. Composition selon la revendication 8, dans laquelle les cellules cancéreuses sont des cellules à lymphome des cellules B du tissu lymphoïde associé aux muqueuses (MALT) ou des cellules à lymphome non-hodgkinien.

12. Composition selon la revendication 8, dans laquelle l'anticorps est un anticorps monoclonal.

13. Composition selon la revendication 12, dans laquelle l'anticorps monoclonal est un anticorps monoclonal humanisé.

14. Protéine IRTA4 humaine comprenant des acides aminés dont la séquence est présentée dans la figure 18D1 - 18D2.

15. Procédé de diagnostic d'une tumeur maligne des cellules B qui comprend un réarrangement chromosomique 1q21 dans un échantillon contenant des cellules cancéreuses provenant d'un sujet qui comprend:
a) la mise en contact d'un échantillon obtenu à partir du sujet avec l'anticorps tel que défini par la revendication 1, dans lequel l'anticorps est marqué avec un marqueur détectable; et
b) la détection de toute liaison dans l'étape (a), dans lequel la détection de liaison indique un diagnostic d'une tumeur maligne des cellules B dans l'échantillon.
